(19) Europäisches Patentamt
European Patent Office
Office européen des brevets

(11) **EP 4 495 265 A1**

(12) **EUROPEAN PATENT APPLICATION**

(43) Date of publication:
**22.01.2025 Bulletin 2025/04**

(21) Application number: **23186098.2**

(22) Date of filing: **18.07.2023**

(51) International Patent Classification (IPC):
***C12Q 1/6886*** (2018.01)

(52) Cooperative Patent Classification (CPC):
**C12Q 1/6886;** C12Q 2600/118; C12Q 2600/158

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventor: **HOFFMANN, Ralf Dieter**
**Eindhoven (NL)**

(74) Representative: **Philips Intellectual Property & Standards**
**High Tech Campus 52**
**5656 AG Eindhoven (NL)**

Remarks:
The complete document including Reference Table(s) and the Sequence Listing(s) can be downloaded from the EPO website

(54) **PREDICTION OF AN OUTCOME OF A SUBJECT SUFFERING FROM A GLIOMA**

(57) The invention relates to a method of predicting an outcome of a subject having a glioma, comprising determining or receiving the result of a determination of a first gene expression profile for each of one or more immune defense response genes, and/or of a second gene expression profile for each of one or more T-Cell receptor signaling genes, and/or of a third gene expression profile for each of one or more PDE4D7 correlated genes, said first, second, and third expression profile(s) being determined in a biological sample obtained from the subject, determining the prediction of outcome based on the first gene expression profile(s), or on the second gene expression profile(s), or on the third gene expression profile(s), or on the first, second, and third gene expression profile(s), and, optionally, providing the prediction to a medical caregiver or the subject.

Fig. 1

EP 4 495 265 A1

**Description**

FIELD OF THE INVENTION

The invention relates to a method of predicting an outcome of a subject suffering from a glioma, and to an apparatus for predicting an outcome of a subject suffering from a glioma. Moreover, the invention relates to a diagnostic kit, to a use of the kit, to a use of the kit in a method of predicting an outcome of a subject suffering from a glioma, to a use of first, second, and/or third gene expression profile(s) in a method of predicting an outcome of a subject suffering from a glioma, and to a corresponding computer program product.

BACKGROUND OF THE INVENTION

Cancer is a class of diseases in which a group of cells displays uncontrolled growth, invasion and sometimes metastasis. These three malignant properties of cancers differentiate them from benign tumors, which are self-limited and do not invade or metastasize. Brain tumors are relatively rare, but even when benign can be life-threatening. They are grouped under the brain and other nervous system cancers. For 2020, it is estimated that almost 24,000 new cases will be diagnosed (representing 1.3% of all new cancer cases), and more than 18,000 will die from the disease, representing 3% of all cancer deaths. The median age at diagnosis is 59 years, with quite some spread around the age groups; the 5-year relative survival is 32.6%. In general, the earlier cancers are diagnosed, the higher the chance of surviving more than 5 years after diagnosis. For brain and other nervous system cancer, 77% is diagnosed at the local stage, for which the 5-year relative survival is 35.3%, just barely higher than for distant (32.7%) and regional (20.3%) localization at diagnosis. Men are affected slightly more than women, and it is more common in those with certain genetic syndromes. Although the cause for most adult brain and spinal cord tumors is not known, there are some risk factors for certain types of brain tumors. For example, being exposed to vinyl chloride may increase the risk of glioma, and infection with the Epstein-Barr virus, having AIDS, or receiving an organ transplant may increase the risk of primary CNS lymphoma. Also, having certain genetic syndromes may increase the risk for brain tumors, such as neurofibromatosis type 1 or 2, von Hippel-Lindau disease, tuberous sclerosis, Li-Fraumeni syndrome, Turcot syndrome type 1 or 2, and nevoid basal cell carcinoma syndrome.

Brain tumors can be divided in glial and non-glial tumors. Most adult brain tumors start in the glial cells. These tumors are called gliomas. Non-glial tumors generally start in areas outside the brain tissue, for example in the nerves, the covering of the brain (the meninges) or the nearby glands, such as the pituitary or pineal gland.

Glial brain tumors (gliomas) rarely spread to other parts of the body, but they can spread through the brain tissue. Also benign forms can be harmful, as they tend to press and destroy normal brain tissue, sometimes leading to life-threatening damage. As they are located in the brain, the main concerns are around how fast they grow, if they spread through the rest of the brain, where they are located, and if they can be removed. Brain and spinal cord tumors are different in adults and children, with respect to where they are formed, which cell types are involved, and differing in outlook and treatment options.

In adults, secondary brain tumors (tumors that start in another part of the body and have spread to the brain; non-glial tumors) are more common than primary brain tumors. Conversely, primary brain tumors rarely spread to other organs, although they can still cause a lot of damage in the brain. The brain consists of different types of tissue with different functions. AS such, depending on where the tumor originates the tumor can have different properties and consequential different treatments are considered.

Gliomas start in glial cells, and can be astrocytomas (including glioblastomas), oligodendrogliomas and ependymomas. Low grade (I+II) astrocytomas tend to grow slowly, whereas high grade (II+IV) tend to grow quickly and spread into the surrounding brain tissue. Glioblastomas are grade IV astrocytomas and are the fastest growing, making up more than 50% of all gliomas, and which are the most common malignant brain tumors in adults. Oligodendromas start in brain glial cells and tend to grow slowly (grade II), but most of them can grow into surrounding tissue, making them hard to remove surgically. Ependymomas start in ependymal cells, and typically grow in the ventricles or spinal cord in adults. These tumors are more likely to spread along the cerebrospinal fluid than other gliomas but do not spread outside the brain or spinal cord.

Meningiomas originate in the meninges, the layers that surround the outer part of the brain, and account for about 30% of brain tumors, which makes them the most common primary brain tumors in adults. Medulloblastomas originate from neuroectodermal cells in the cerebellum. They are fast growing, and more common in children than adults. These tumors can be treated by surgery, radiation therapy and chemotherapy. A very uncommon type of brain tumors are the gangliogliomas, slow growing tumors which contain neurons and glial cells. 8% of all CNS tumors are Schwannomas which are almost always grade I tumors. Finally, craniopharyngiomas are slow-growing tumors that often cause hormonal and vision problems due to their location.

There is no standard staging system for this type of tumors. Treatment is based on the type of cell in which the tumor began, the location of the tumor, the amount of cancer left after surgery -if possible-, the grade of the tumor.

Repeated imaging is used to help plan more treatment. Currently, there are five types of standard treatment used:

- Active surveillance: can be used for slow-growing tumors, and to avoid or delay the need for radiation therapy or surgery
- Surgery: to diagnose and to relieve pressure of the tumor on the brain. Surgery can be combined with adjuvant chemotherapy or RT
- RT: to kill or slow growth of cancer cells. Different forms of RT are in use to prevent radiation damage to the surrounding brain tissue.
- Chemotherapy: can be applied systemically, although many drugs are not able to cross the blood-brain barrier. Therefore, using intrathecal chemotherapy, it can be directly placed into the cerebrospinal fluid.
- Targeted therapy: specifically attacks tumor cells with a certain characteristic and may cause less side effects than chemotherapy and RT. Examples are monoclonal antibodies, tyrosine receptor kinase inhibitors and VEGF inhibitors.
- New therapies that are being tested in clinical trials are different forms of immunotherapy and proton beam RT.

Attempts at characterizing gliomas are aimed at providing a better insight in the tumor biology/physiology in order to get to a better treatment decision. A few examples are summarized below. Diagnosis and treatment of gliomas is significantly limited by their molecular and cellular complexity, as well as by inter and intra-tumoral heterogeneity.

Metabolic profiles produced by (1)H MRS can be used to distinguish between two distinct glioblastoma phenotypes: low-generation (LG) and high-generation (HG) tumors. In the LG tumors, a more pronounced anaerobic metabolism was present, suggesting a more malignant phenotype [Thorsen et al. NMR Biomed. 2008 Oct;21(8):830-8.].

Molecular and cellular complexity, and heterogeneity factors complicate the ability to develop effective and reliable biomarkers. The most commonly glioma associated molecular aberrations include IDH mutations, EGFR amplification, P53 and RB mutations, and abnormalities in the pathways involving RTK, Akt, PI3K and Ras [Kan et al. BMJ Neurol Open. 2020 Aug 24;2(2):e000069.].

In addition to molecular aberrations, RNA expression profiling studies found correlations with patient survival. In one study, using multiple datasets, unsupervised hierarchical clustering for subclassification based on RNA expression profiles identified molecular subgroups that are distinct from histologic subgroups, correlating better with patient survival [Gravendeel et al. Cancer Res. 2009 Dec 1;69(23):9065-72.]. These data also provided evidence for treatment response. Additionally, it was found that specific genetic changes (EGFR amplification, IDH1 mutation, and 1p19q LOH) segregate in distinct molecular subgroups. However, the limitations of using unsupervised hierarchical clustering are insufficient sample size in case of rare tumors, and not including all tumor types, which may lead to insufficient/incorrect classifications. Nevertheless, the authors concluded that the identified subtypes improve on histological classification of gliomas and are an accurate predictor of prognosis. Therefore, molecular classification may form a rational for clinical decision making and novel targeted therapies.

Standard care for glioblastoma is still similar to that of other cancers, but are not always very effective. Immunotherapy has provided a more specific and efficient approach to prolong patients' lives, but is faced with several challenges. GBM is considered a 'cold' tumor and therefore often immunotherapy fails. Understanding the mechanisms that underlie this, may improve treatment decisions. Recent research has vested hope on proteomics, which can reveal the actual state of the tumor cells by quantifying thousands of functional proteins. This may supplement the 'traditional' molecular subtyping which reveals information on the tumor origin.

In conclusion, an improved understanding of the (molecular) mechanisms of disease, and the emerging availability of treatments underlines the strong need for better prediction of response to treatment remains. As many articles have concluded, there is a strong need for biomarkers that aid in diagnosis and treatment, that identify recurrent disease, and that can indicate treatment response.

SUMMARY OF THE INVENTION

In a first aspect, the invention relates to a method of predicting an outcome of a subject having a glioma, the method comprising determining or receiving the result of a determination of six or more gene expression levels selected from a first gene expression profile, a second gene expression profile and/or a third gene expression profile, wherein the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1; the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70; the third gene expression profile consists of the PDE4D7 correlated genes selected from the group consisting of: ABCC5, CUX2, KIAA1549, PDE4D, RAP1GAP2, SLC39A11, TDRD1, and VWA2; said gene expression levels being determined in a biological sample obtained from the subject; and determining the prediction of the outcome based on six or more gene expression levels.

In a second aspect the invention relates to an apparatus for predicting an outcome of a subject having a glioma, comprising an input adapted to receive data indicative of six or more gene expression levels selected from a first gene expression profile, a second gene expression profile and/or a third gene expression profile, wherein the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1; the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70; the third gene expression profile consists of the PDE4D7 correlated genes selected from the group consisting of: ABCC5, CUX2, KIAA1549, PDE4D, RAP1GAP2, SLC39A11, TDRD1, and VWA2; said gene expression levels being determined in a biological sample obtained from the subject; and a processor adapted to determine the prediction of outcome based on the six or more gene expression levels, and optionally, a providing unit adapted to provide the prediction to a medical caregiver or the subject.

In a third aspect the invention relates to a computer program product comprising instructions which, when the program is executed by a computer, cause the computer to carry out a method for predicting an outcome of a subject having a glioma comprising receiving data indicative of six or more gene expression levels selected from a first gene expression profile, a second gene expression profile and/or a third gene expression profile, wherein the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1; the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70; the third gene expression profile consists of the PDE4D7 correlated genes selected from the group consisting of: ABCC5, CUX2, KIAA1549, PDE4D, RAP1GAP2, SLC39A11, TDRD1, and VWA2; said gene expression levels being determined in a biological sample obtained from the subject; and determining a prediction of an outcome of the subject based on the six or more gene expression levels, and optionally, providing the prediction to a medical caregiver or the subject.

In a fourth aspect the invention relates to the use of a kit, the use comprising:

- determining six or more gene expression levels in a sample obtained from a subject having a glioma; and providing an outcome for the subject based on the six or more gene expression levels; wherein the kit comprises means for determining six or more gene expression levels selected from a first gene expression profile, a second gene expression profile and/or a third gene expression profile, wherein the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1; the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70; the third gene expression profile consists of the PDE4D7 correlated genes selected from the group consisting of: ABCC5, CUX2, KIAA1549, PDE4D, RAP1GAP2, SLC39A11, TDRD1, and VWA2.

In a fifth aspect the invention relates to a product for use in the treatment or amelioration of a subject having a glioma, the product comprising a chemotherapeutic compound, a targeted cancer therapeutic compound or an immunotherapeutic compound, wherein the use comprises determining or receiving the result of a determination of six or more gene expression levels selected from a first gene expression profile, a second gene expression profile and/or a third gene expression profile, wherein the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1; the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70; the third gene expression profile consists of the PDE4D7 correlated genes selected from the group consisting of: ABCC5, CUX2, KIAA1549, PDE4D, RAP1GAP2, SLC39A11, TDRD1, and VWA2; said gene expression levels being determined in a biological sample obtained from the subject; and determining the prediction of the outcome based on six or more gene expression levels; and administering to the subject the product.

BRIEF DESCRIPTION OF THE DRAWINGS

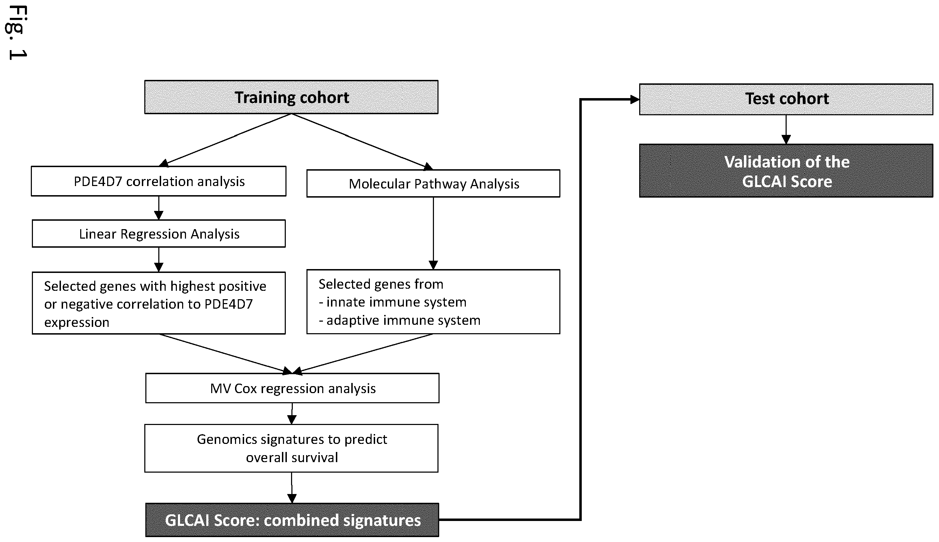

Fig. 1 Provides a schematic overview of how the different gene signature based models are trained on a training cohort and validated on a test cohort. Briefly, a cohort of patients gene expression data from patients having different types of gliomas is divided randomly between a test and training cohort. The figure depicts the development of the full "GLCAI Score" signature, but the same principle applies to the individual signatures (TCR, IFR, PDE4D7 correlated genes) or individually selected genes from the different genes signatures described herein. Using Cox regression analysis the signatures are trained in predicting overall survival. Validation of the different models was performed on the test cohort.

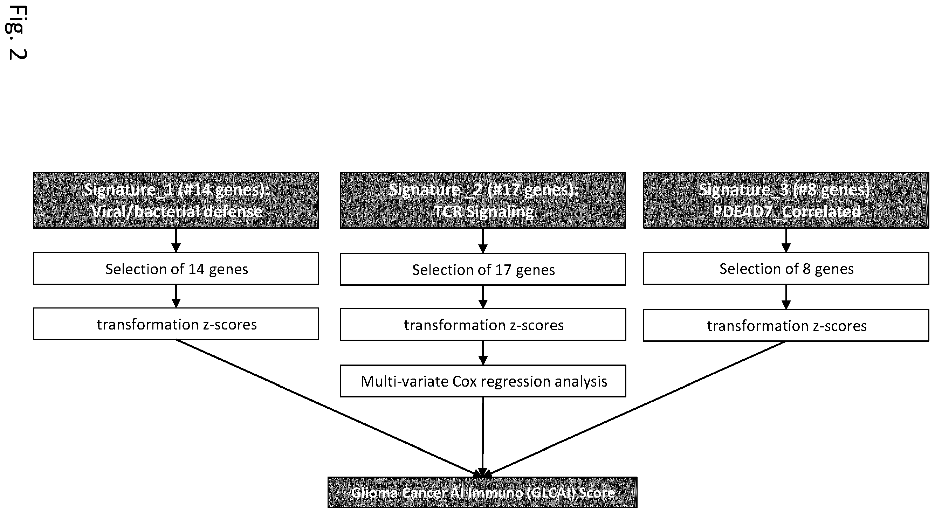

Fig. 2 describes schematically how the different individual gene signatures are developed and how they are combined to the GLCAI Score.

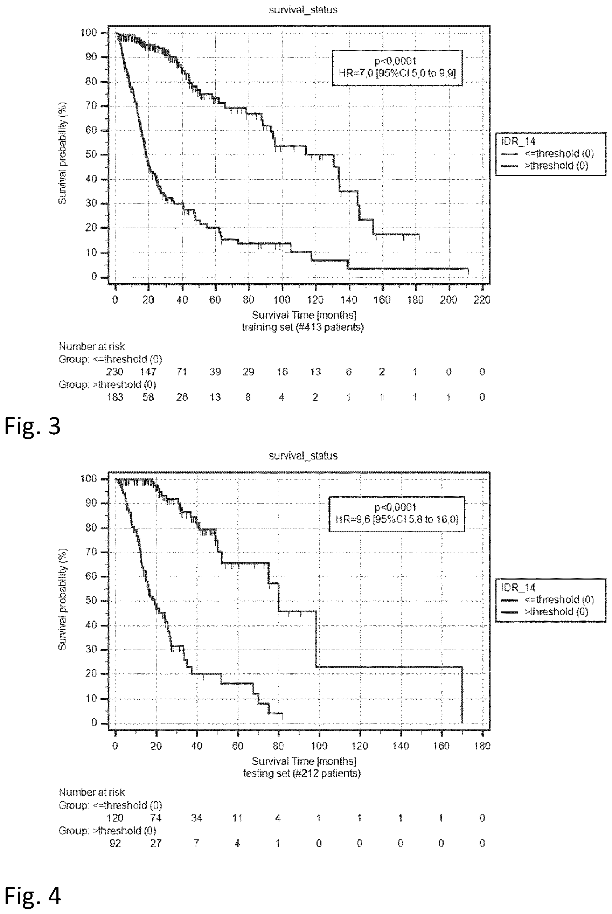

Figs. 3 and 4 show a Kaplan-Meier curve of the IDR_14 model in a 413 patient cohort (training set used to develop the IDR_14 model; top graph) or in a 212 patient cohort (test set to validate the IDR_14 model; bottom graph) with all patients having a glioma. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1+2 of the total cohort), and were fixed for validation in the test cohort (group 3 of the total cohort). Logrank, HR and confidence interval are included in the figure. The included supplementary lists indicate the number of patients at risk (threshold = 0, low risk (<=0), high risk(>0)) for the IDR_14 model classes analysed.

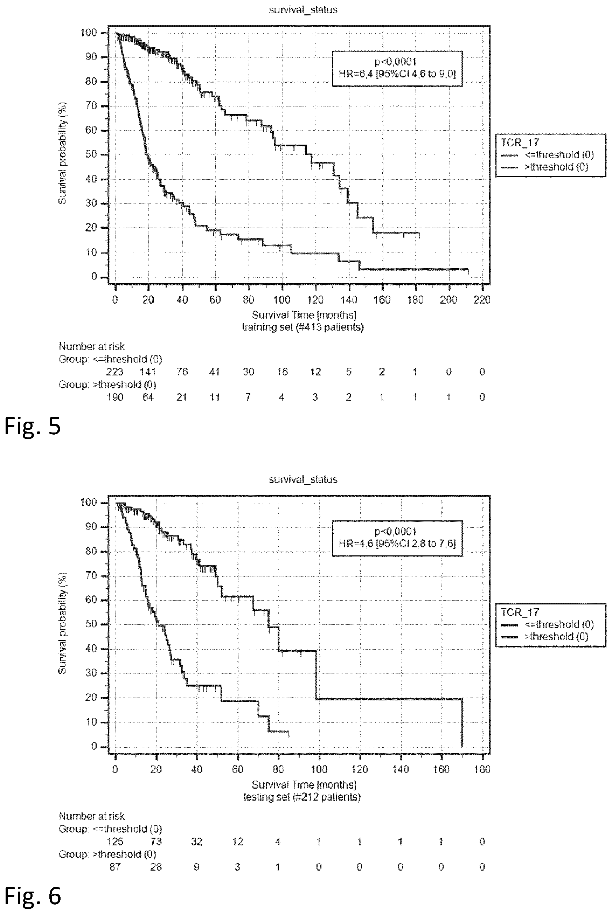

Figs. 5 and 6 show a Kaplan-Meier curve of the TCR_17 model in a 413 patient cohort (training set used to develop the TCR_17 model; top graph) or in a 212 patient cohort (test set to validate the TCR_17 model; bottom graph) with all patients having a glioma. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1+2 of the total cohort), and were fixed for validation in the test cohort (group 3 of the total cohort). Logrank, HR and confidence interval are included in the figure. The included supplementary lists indicate the number of patients at risk (threshold = 0, low risk (<=0), high risk(>0)) for the TCR_17 model classes analysed.

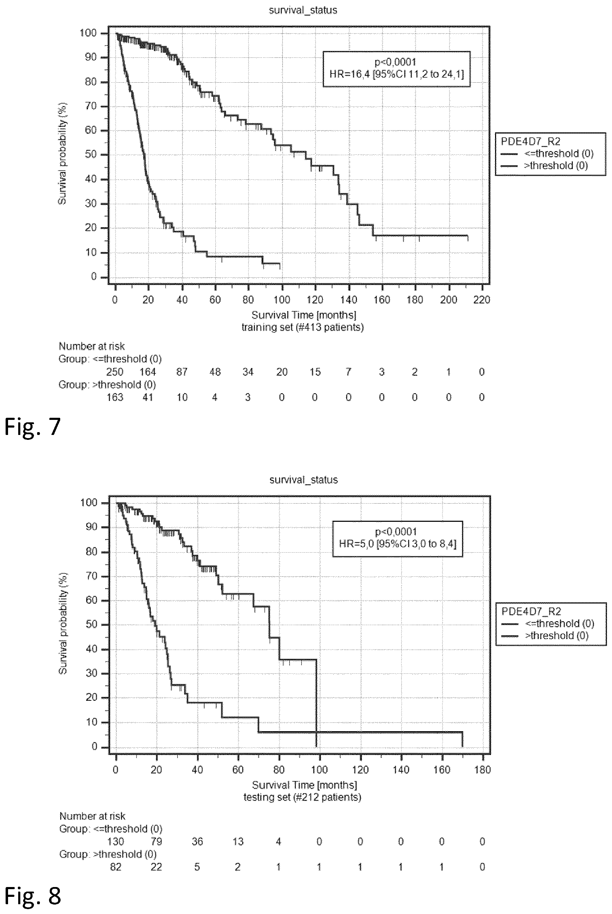

Figs. 7 and 8 show a Kaplan-Meier curve of the PDE4D7_CORR model in a 413 patient cohort (training set used to develop the PDE4D7_CORR model; top graph) or in a 212 patient cohort (test set to validate the PDE4D7_CORR model; bottom graph) with all patients having a glioma. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1+2 of the total cohort), and were fixed for validation in the test cohort (group 3 of the total cohort). Logrank, HR and confidence interval are included in the figure. The included supplementary lists indicate the number of patients at risk (threshold = 0, low risk (<=0), high risk(>0)) for the PDE4D7_CORR model classes analysed.

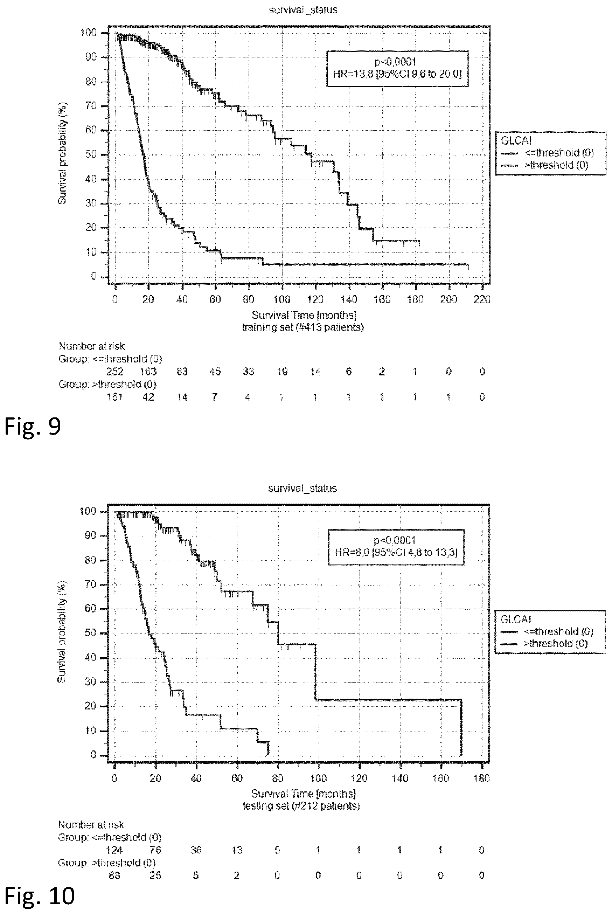

Figs. 9 and 10 show a Kaplan-Meier curve of the GLCAI model in a 413 patient cohort (training set used to develop the GLCAI model; top graph) or in a 212 patient cohort (test set to validate the GLCAI model; bottom graph) with all patients having a glioma. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1+2 of the total cohort), and were fixed for validation in the test cohort (group 3 of the total cohort). Logrank, HR and confidence interval are included in the figure. The included supplementary lists indicate the number of patients at risk (threshold = 0, low risk (<=0), high risk(>0)) for the GLCAI model classes analysed.

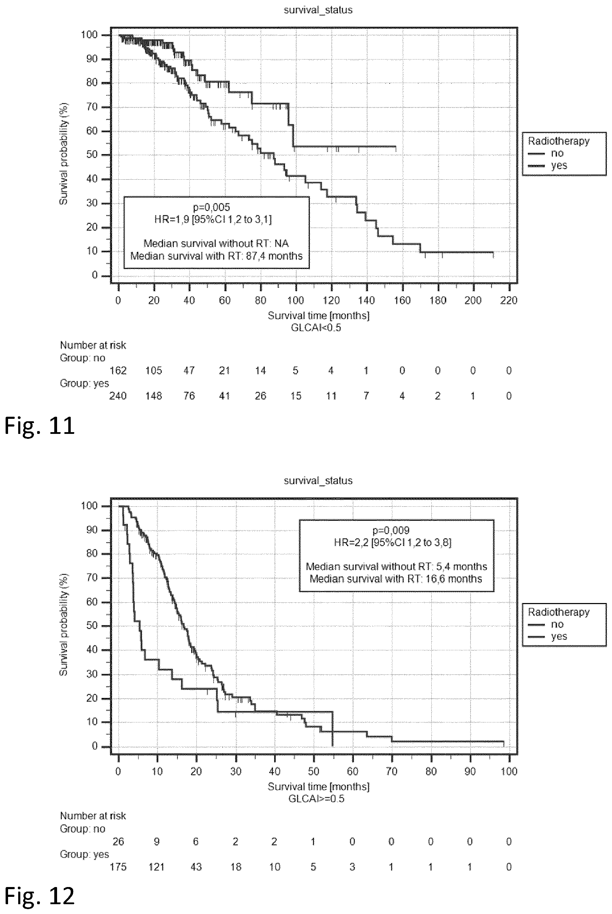

Figs. 11 and 12 show a Kaplan-Meier curve of the GLCAI Radiotherapy model in a 402 patient cohort (GLCAI low risk group; top graph) and in a 201 patient cohort (GLCAI high risk group; bottom graph) with all patients having a glioma. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. In the low risk group (GLCAI<0) patients who received no RT had a 40% less chance to die, a trend that became more pronounced over time. In the high risk group (GLCAI>0) patients who received no RT had a 2.3 fold increased chance to die. Logrank, HR and confidence interval are included in the figure. The included supplementary lists indicate the number of patients at risk (threshold = 0, low risk (<=0), high risk(>0)) for the GLCAI model classes analysed.

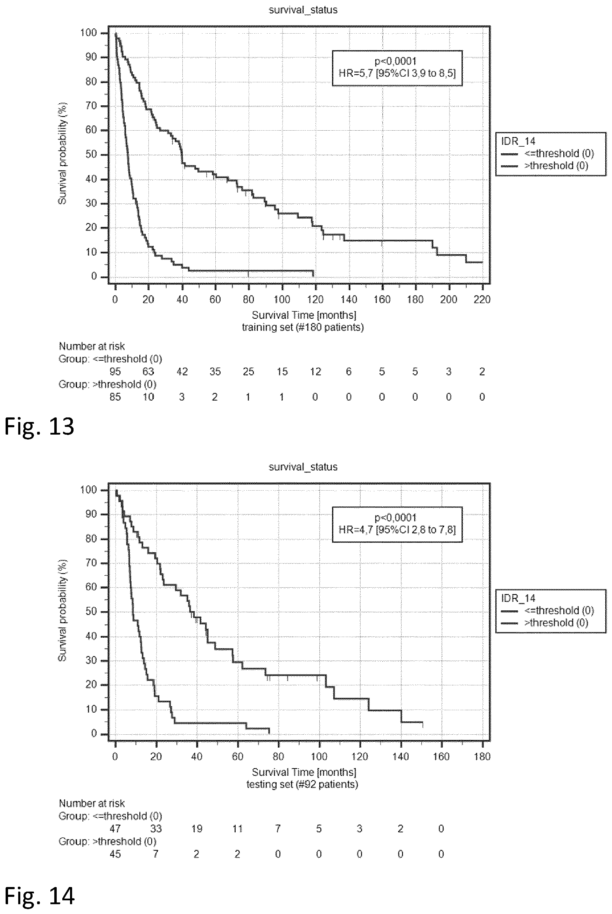

Figs. 13 and 14 show a Kaplan-Meier curve of the IDR_14 model in a 180 patient cohort (training set used to develop the IDR_14 model; top graph) or in a 92 patient cohort (test set to validate the IDR_14 model; bottom graph) with all patients having a glioma. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1+2 of the total cohort), and were fixed for validation in the test cohort (group 3 of the total cohort). Logrank, HR and confidence interval are included in the figure. The included supplementary lists indicate the number of patients at risk (threshold = 0, low risk (<=0), high risk(>0)) for the IDR_14 model classes analysed.

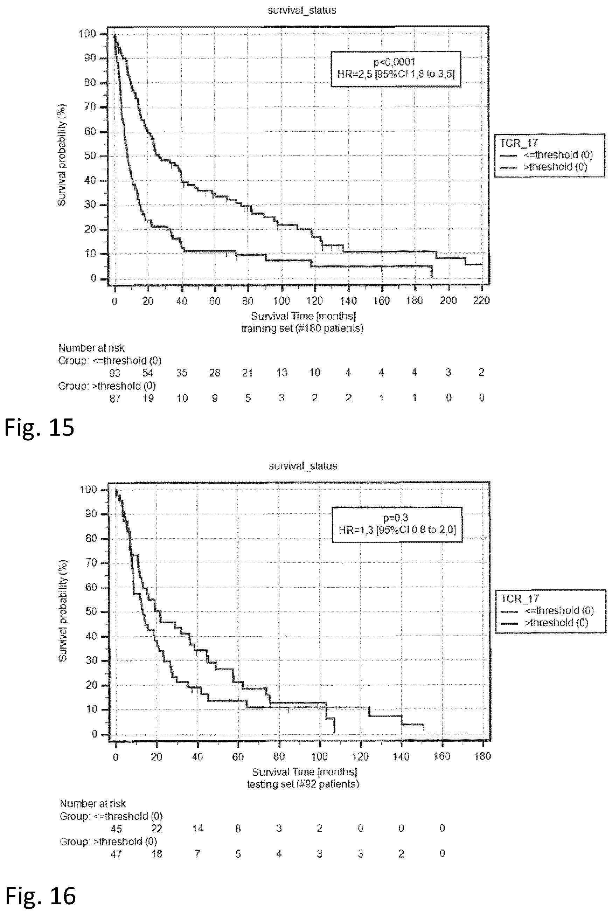

Figs. 15 and 16 show a Kaplan-Meier curve of the TCR_17 model in a 180 patient cohort (training set used to develop the TCR_17 model; top graph) or in a 92 patient cohort (test set to validate the TCR_17 model; bottom graph) with all patients having a glioma. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1+2 of the total cohort), and were fixed for validation in the test cohort (group 3 of the total cohort). Logrank, HR and confidence interval are included in the figure. The included supplementary lists indicate the number of patients at risk (threshold = 0, low risk (<=0), high risk(>0)) for the TCR_17 model classes analysed.

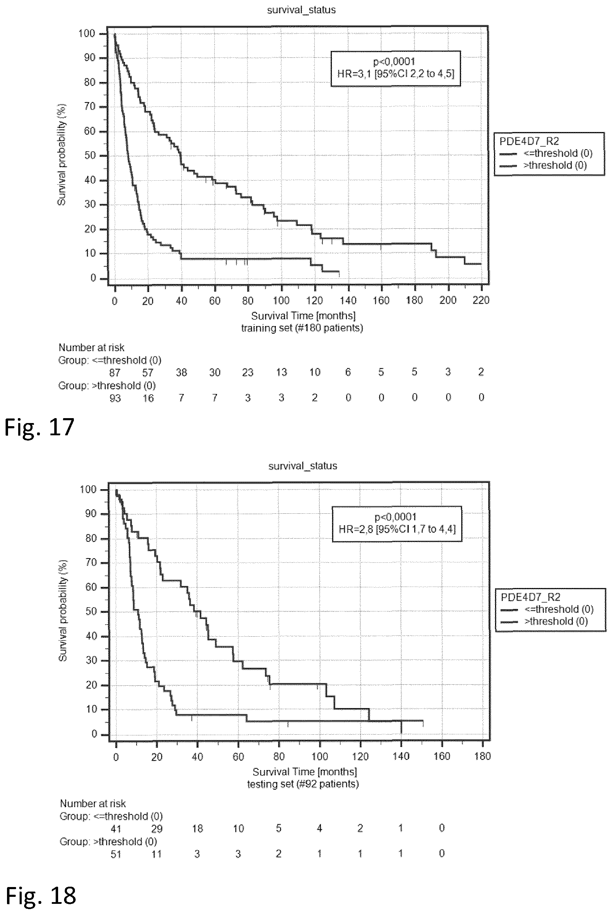

Figs. 17 and 18 show a Kaplan-Meier curve of the PDE4D7_CORR model in a 180 patient cohort (training set used to develop the PDE4D7_CORR model; top graph) or in a 92 patient cohort (test set to validate the PDE4D7_CORR model; bottom graph) with all patients having a glioma. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1+2 of the total cohort), and were fixed for validation in the test cohort (group 3 of the total cohort). Logrank, HR and confidence interval are included in the figure. The included supplementary lists indicate the number of patients at risk (threshold = 0, low risk (<=0), high risk(>0)) for the PDE4D7_CORR model classes analysed.

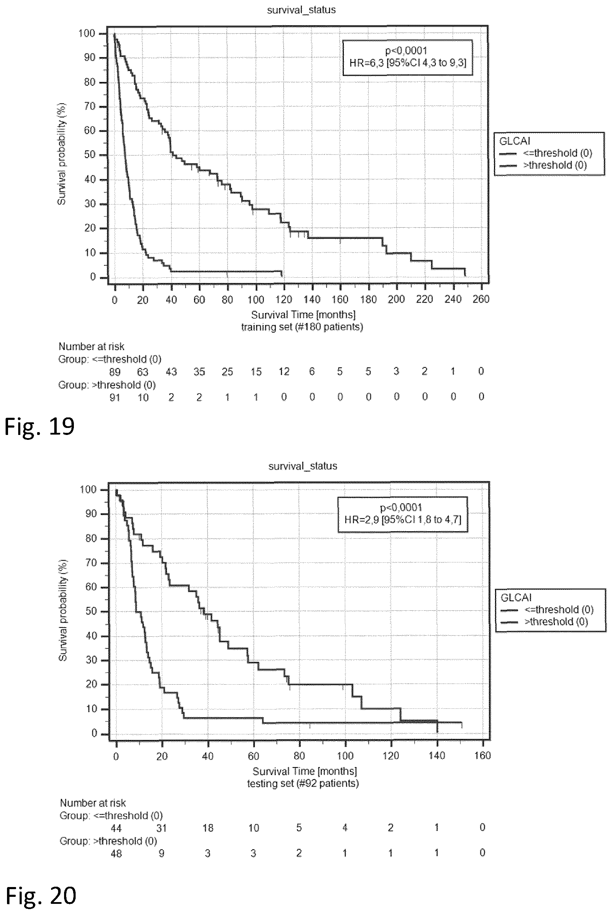

Figs. 19 and 20 show a Kaplan-Meier curve of the GLCAI model in a 180 patient cohort (training set used to develop the GLCAI model; top graph) or in a 92 patient cohort (test set to validate the GLCAI model; bottom graph) with all patients having a glioma. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1+2 of the total cohort), and were fixed for validation in the test cohort (group 3 of the total cohort). Logrank, HR and confidence interval are included in the figure. The included supplementary lists indicate the number of patients at risk (threshold = 0, low risk (<=0), high risk(>0)) for the GLCAI model classes analysed.

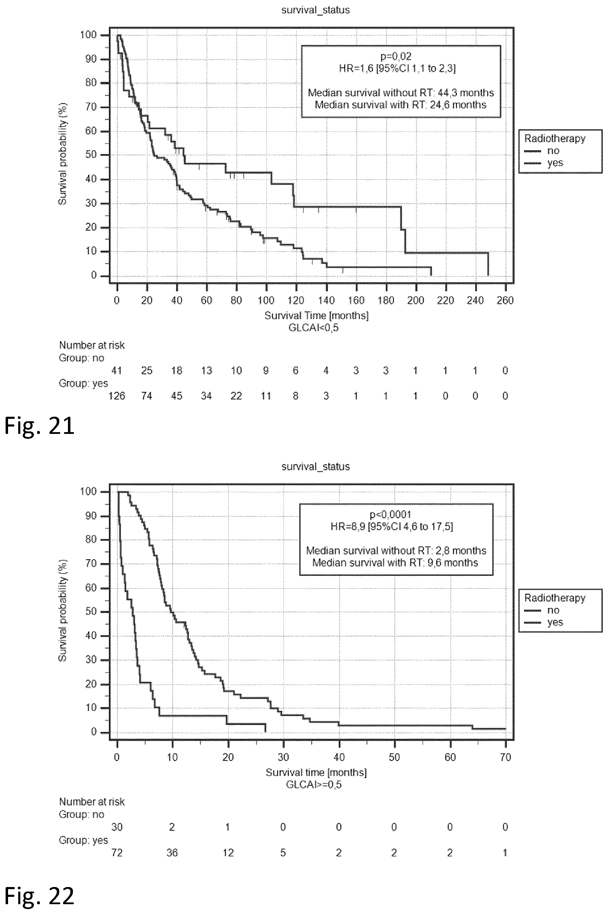

Figs. 21 and 22 show a Kaplan-Meier curve of the GLCAI Radiotherapy model in a 167 patient cohort (GLCAI low risk group; top graph) and in a 102 patient cohort (GLCAI high risk group; bottom graph) with all patients having a glioma. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. In the low risk group (GLCAI<0) patients who received no RT had a 40% less chance to die, a trend that became more pronounced over time. In the high risk group (GLCAI>0) patients who received no RT had a 2.3 fold increased chance to die. Logrank, HR and confidence interval are included in the figure. The included supplementary lists indicate the number of patients at risk (threshold = 0, low risk (<=0), high risk(>0)) for the GLCAI model classes analysed.

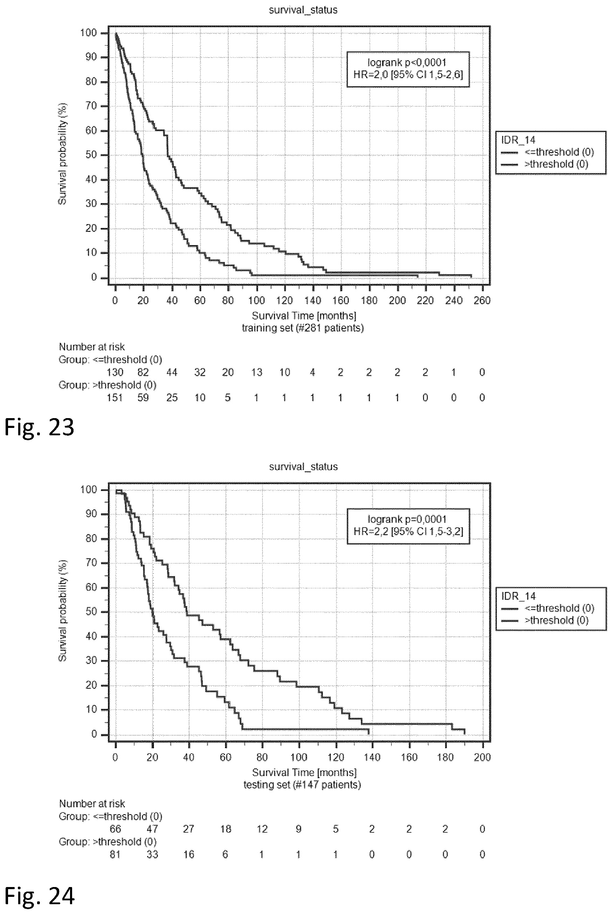

Figs. 23 and 24 show a Kaplan-Meier curve of the IDR_14 model in a 281 patient cohort (training set used to develop the IDR_14 model; top graph) or in a 147 patient cohort (test set to validate the IDR_14 model; bottom graph) with all patients having a glioma. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1+2 of the total cohort), and were fixed for validation in the test cohort (group 3 of the total cohort). Logrank, HR and confidence interval are included in the figure. The included supplementary lists indicate the number of patients at risk (threshold = 0, low risk (<=0), high risk(>0)) for the IDR_14 model classes analysed.

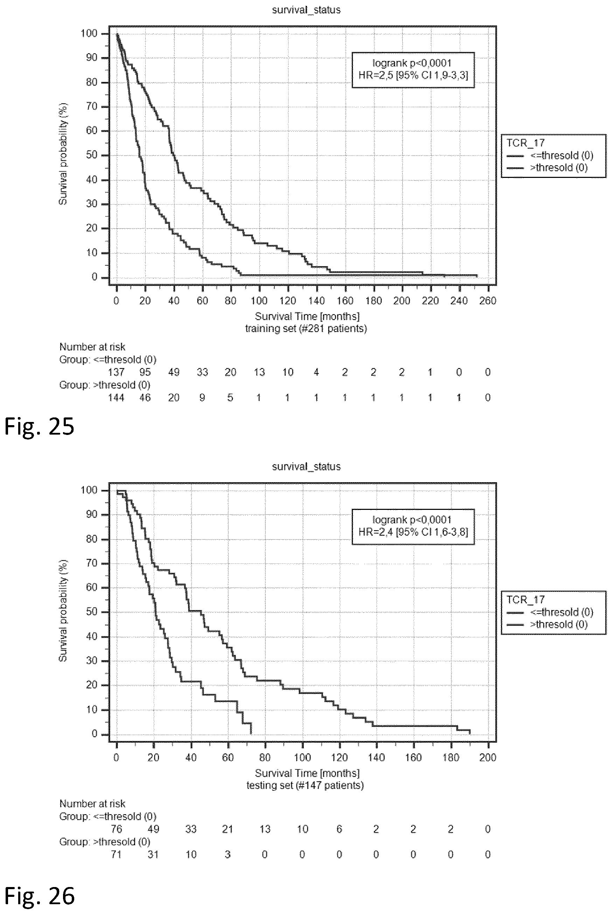

Figs. 25 and 26 show a Kaplan-Meier curve of the TCR_17 model in a 281 patient cohort (training set used to develop the TCR_17 model; top graph) or in a 147 patient cohort (test set to validate the TCR_17 model; bottom graph) with all patients having a glioma. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1+2 of the total cohort), and were fixed for validation in the test cohort (group 3 of the total cohort). Logrank, HR and confidence interval are included in the figure. The included supplementary lists indicate the number of patients at risk (threshold = 0, low risk (<=0), high risk(>0)) for the TCR_17 model classes analysed.

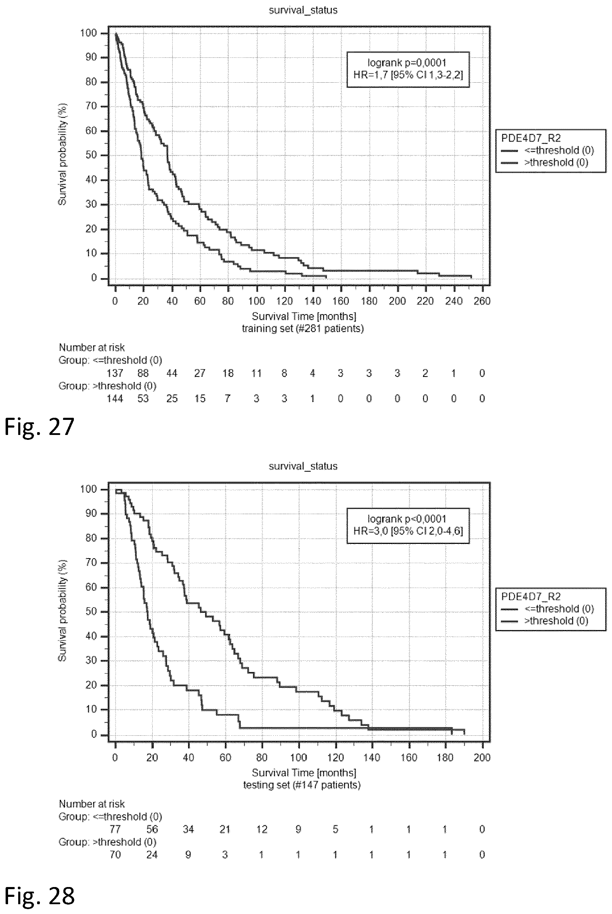

Figs. 27 and 28 show a Kaplan-Meier curve of the PDE4D7_CORR model in a 281 patient cohort (training set used to develop the PDE4D7_CORR model; top graph) or in a 147 patient cohort (test set to validate the PDE4D7_CORR model; bottom graph) with all patients having a glioma. The clinical endpoint tested was time to death in months.

Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1+2 of the total cohort), and were fixed for validation in the test cohort (group 3 of the total cohort). Logrank, HR and confidence interval are included in the figure. The included supplementary lists indicate the number of patients at risk (threshold = 0, low risk (<=0), high risk(>0)) for the PDE4D7_CORR model classes analysed.

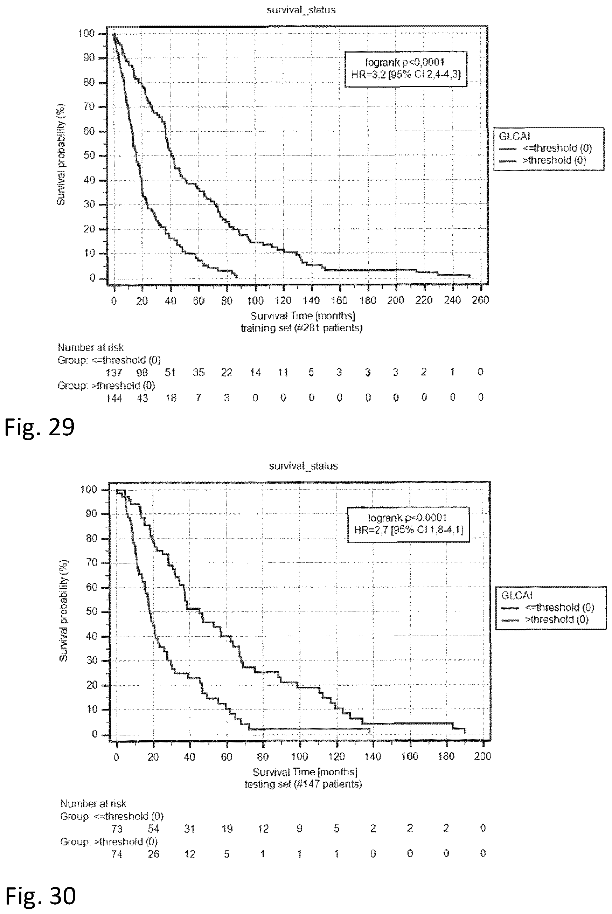

Figs. 29 and 30 show a Kaplan-Meier curve of the GLCAI model in a 281 patient cohort (training set used to develop the GLCAI model; top graph) or in a 147 patient cohort (test set to validate the GLCAI model; bottom graph) with all patients having a glioma. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1+2 of the total cohort), and were fixed for validation in the test cohort (group 3 of the total cohort). Logrank, HR and confidence interval are included in the figure. The included supplementary lists indicate the number of patients at risk (threshold = 0, low risk (<=0), high risk(>0)) for the GLCAI model classes analysed.

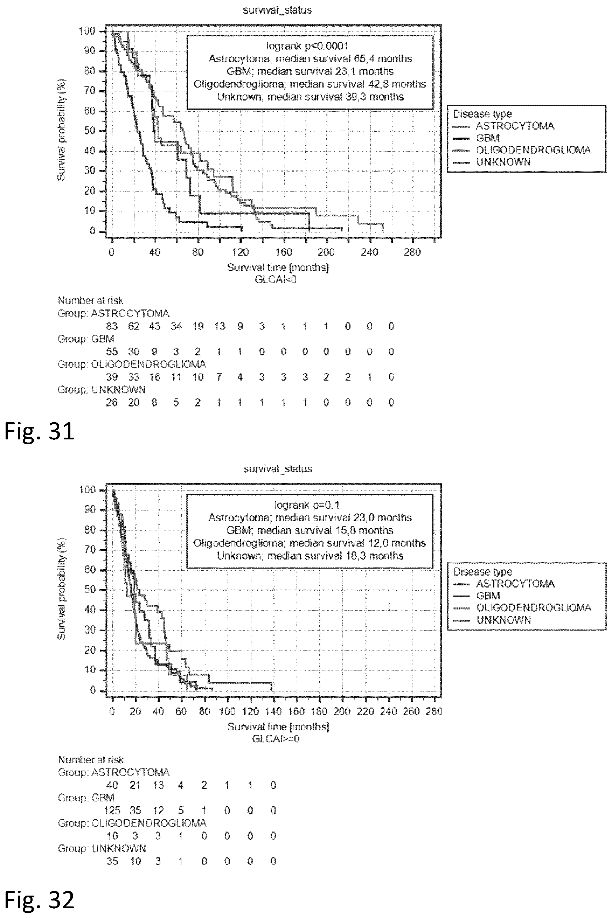

Figs. 31 and 32 show a Kaplan-Meier curve of the GLCAI model in a 203 patient cohort per disease type (GLCAI low risk group; top graph) and in a 216 patient cohort (GLCAI high risk group; bottom graph) with all patients having a glioma. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Logrank, HR and confidence interval are included in the figure. The included supplementary lists indicate the number of patients at risk (threshold = 0, low risk (<=0), high risk(>0)) for the GLCAI model classes analysed.

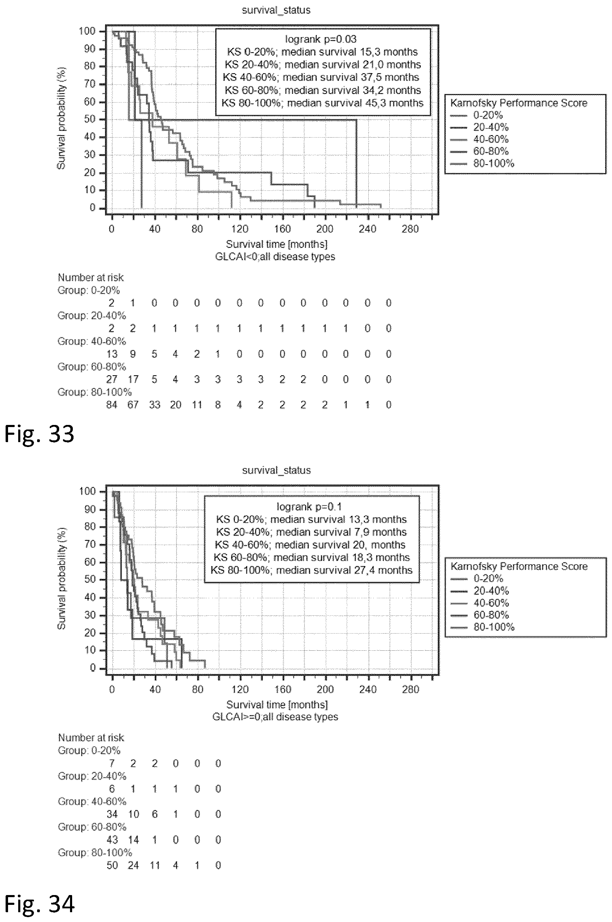

Figs. 33 and 34 show a Kaplan-Meier curve of the GLCAI model in a 203 patient cohort per Karnofsky Performance Score category (GLCAI low risk group; top graph) and in a 216 patient cohort (GLCAI high risk group; bottom graph) with all patients having a glioma. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Logrank, HR and confidence interval are included in the figure. The included supplementary lists indicate the number of patients at risk (threshold = 0, low risk (<=0), high risk(>0)) for the GLCAI model classes analysed.

DEFINITIONS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of $\pm 20\%$, preferably $\pm 15\%$, more preferably $\pm 10\%$, and even more preferably $\pm 5\%$.

**"About" and "approximately"**: these terms, when referring to a measurable value such as an amount, a temporal duration, and the like, is meant to encompass variations of $\pm 20\%$ or $\pm 10\%$, more preferably $\pm 5\%$, even more preferably $\pm 1\%$, and still more preferably $\pm 0.1\%$ from the specified value, as such variations are appropriate to perform the disclosed methods.

**"Antagonist"** and **"inhibitor"**: These terms are used interchangeably, and they refer to a compound or agent having the ability to reduce or inhibit a biological function of a target protein or polypeptide, such as by reducing or inhibiting the activity or expression of the target protein or polypeptide. Accordingly, the terms "antagonist" and "inhibitor" are defined in the context of the biological role of the target protein or polypeptide. An inhibitor need not completely abrogate the biological function of a target protein or polypeptide, and in some embodiments reduces the activity by at least 20%, 30%, 40%, 50%, 60%, 70%, 80%, 90%, 95%, or 99%. While some antagonists herein specifically interact with (e.g., bind to) the target, compounds that inhibit a biological activity of the target protein or polypeptide by interacting with other members of the signal transduction pathway of which the target protein or polypeptide are also specifically included within this definition. Non-limiting examples of biological activity inhibited by an antagonist include those associated with the development, growth, or spread of a tumor, or an undesired immune response as manifested in autoimmune disease.

**"Anti-cancer effect"**: This refers to the effect a therapeutic agent has on cancer, e.g., a decrease in growth, viability, or both of a cancer cell. The IC50 of cancer cells can be used as a measure the anti-cancer effect. IC50 refers to a measure of the effectiveness of a therapeutic agent in inhibiting cancer cells by 50%.

**"Alleviating cancer"**: The term, in the context of specific cancers and/or their pathologies, refers to degrading a tumor, for example, breaking down the structural integrity or connective tissue of a tumor, such that the tumor size is reduced when compared to the tumor size before treatment. "Alleviating" metastasis of cancer includes reducing the rate at which the cancer spreads to other organs.

**"Compositions", "products" or "combinations"**: These encompass those compositions suitable for various routes of administration, including, but not limited to, intravenous, subcutaneous, intradermal, subdermal, intranodal, intratumoral, intramuscular, intraperitoneal, oral, nasal, topical (including buccal and sublingual), rectal, vaginal, aerosol and/or parenteral or mucosal application. The compositions, formulations, and products according to the disclosure invention normally comprise the drugs/compound/inhibitor (alone or in combination) and one or more suitable pharmaceutically acceptable excipients or carriers.

**"Combination therapy", or "in combination with":** These term refers to the use of more than one compound or agent to treat a particular disorder or condition. For example, Compound 1 may be administered in combination with at least one additional therapeutic agent. By "in combination with," it is not intended to imply that the other therapy and Compound 1 must be administered at the same time and/or formulated for delivery together, although these methods of delivery are within the scope of this disclosure. Compound 1 can be administered concurrently with, prior to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks before), or subsequent to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks, or 16 weeks after), one or more other additional agents. In general, each therapeutic agent will be administered at a dose and/or on a time schedule determined for that particular agent. The other therapeutic agent can be administered with Compound 1 herein in a single composition or separately in a different composition. Higher combinations, e.g., triple therapy, are also contemplated herein.

It is to be understood that the term "comprising" is not limiting. For the purposes of the present invention the term "consisting of' is considered to be a preferred embodiment of the term "comprising of". If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

As used herein, the term "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc. As used herein, the term "at most" a particular value means that particular value or less. For example, "at most 5" is understood to be the same as "5 or less" i.e., 5, 4, 3, ....-10, -11, etc.

As used herein, the term "and/or" indicates that one or more of the stated cases may occur, alone or in combination with at least one of the stated cases, up to with all of the stated cases.

As used herein, the word "comprise" or variations thereof such as "comprises" or "comprising" will be understood to include a stated element, integer or step, or group of elements, integers or steps, but not to exclude any other element, integer or steps, or groups of elements, integers or steps. The verb "comprising" includes the verbs "essentially consisting of" and "consisting of".

As used herein, the term "conventional techniques" refers to a situation wherein the methods of carrying out the conventional techniques used in methods of the invention will be evident to the skilled worker. The practice of conventional techniques in molecular biology, biochemistry, computational chemistry, cell culture, recombinant DNA, bioinformatics, genomics, sequencing and related fields are well-known to those of skill in the art and are discussed, for example, in the following literature references: Sambrook et al., Molecular Cloning. A Laboratory Manual, 2nd Edition, Cold Spring Harbor Laboratory Press, Cold Spring Harbor, N. Y., 1989; Ausubel et al., Current Protocols in Molecular Biology, John Wiley & Sons, New York, 1987 and periodic updates; and the series Methods in Enzymology, Academic Press, San Diego.

As used herein, the term "identity" refers to a measure of the identity of nucleotide sequences or amino acid sequences. In general, the sequences are aligned so that the highest order match is obtained. "Identity" per se has an art-recognized meaning and can be calculated using published techniques. See, e.g.: (Computational Molecular Biology, Lesk, A. M., ED., Oxford University Press, New York, 1988; Biocomputing: Informatics And Genome Projects, Smith, D. W., ED., Academic Press, New York, 1993; Computer Analysis Of Sequence Data, Part I, Griffin, A. M., And Griffin, H. G., EDS., Humana Press, New Jersey, 1994; Sequence Analysis In Molecular Biology, Von Heinje, G., Academic Press, 1987; and Sequence Analysis Primer; Gribskov, M. and Devereux, J., eds., M Stockton Press, New York, 1991). While there exist a number of methods to measure identity between two nucleotide sequences or amino acid sequences, the term "identity" is well known to skilled artisans (Carillo, H., and Lipton, D., SIAM J. Applied Math (1988) 48:1073). Methods commonly employed to determine identity or similarity between two sequences include, but are not limited to, those disclosed in Guide To Huge Computers, Martin J. Bishop, ed., Academic Press, San Diego, 1994, and Carillo, H., and Lipton, D., Siam J. Applied Math (1988) 48:1073. Methods to determine identity and similarity are codified in computer programs. Preferred computer program methods to determine identity and similarity between two sequences include, but are not limited to, GCG program package (Devereux, J., et al., Nucleic Acids Research (1984) 12(1):387), BLASTP, BLASTN, FASTA (Atschul, S. F. et al., J. Molec. Biol. (1990) 215:403).

As an illustration, by a polynucleotide having a nucleotide sequence having at least, for example, 95% "identity" to a reference nucleotide sequence encoding a polypeptide of a certain sequence, it is intended that the nucleotide sequence of the polynucleotide is identical to the reference sequence except that the polynucleotide sequence may include up to five point mutations per each 100 nucleotides of the reference amino acid sequence. In other words, to obtain a polynucleotide having a nucleotide sequence at least 95% identical to a reference nucleotide sequence, up to 5% of the nucleotides in the reference sequence may be deleted and/or substituted with another nucleotide, and/or a number of nucleotides up to 5% of the total nucleotides in the reference sequence may be inserted into the reference sequence. These mutations of the reference sequence may occur at the 5' or 3' terminal positions of the reference nucleotide sequence, or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO: X is intended that the amino acid sequence of the polypeptide is identical to the reference sequence except that the amino acid sequence may include up to five amino acid alterations per each 100 amino acids of the reference amino acid of SEQ ID NO: X. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As used herein, the term "in vitro" refers to experimentation or measurements conducted using components of an organism that have been isolated from their natural conditions.

As used herein, the term "ex vivo" refers to experimentation or measurements done in or on tissue from an organism in an external environment with minimal alteration of natural condition.

As used herein, the term "nucleic acid", "nucleic acid molecule" and "polynucleotide" is intended to include DNA molecules and RNA molecules. A nucleic acid (molecule) may be single-stranded or double-stranded, but preferably is double-stranded DNA.

As used herein, the terms "sequence" when referring to nucleotides, or "nucleic acid sequence", "nucleotide sequence" or "polynucleotide sequence" refer to the order of nucleotides of, or within, a nucleic acid and/or polynucleotide. Within the context of the current invention a first nucleic acid sequence may be comprised within or overlap with a further nucleic acid sequence.

As used herein, the term "subject" or "individual" or "animal" or "patient" or "mammal," used interchangeably, refer to any subject, particularly a mammalian subject, for whom diagnosis, prognosis, or therapy is desired. Mammalian subjects include humans, domestic animals, farm animals, and zoo-, sports-, or pet-animals such as dogs, cats, guinea pigs, rabbits, rats, mice, horses, cattle, cows, bears, and so on. As defined herein a subject may be alive or dead. Samples can be taken from a subject post-mortem, i.e. after death, and/or samples can be taken from a living subject.

As used herein, terms "treatment", "treating", "palliating", "alleviating" or "ameliorating", used interchangeably, refer to an approach for obtaining beneficial or desired results including, but not limited to, therapeutic benefit. By therapeutic benefit is meant eradication or amelioration or reduction (or delay) of progress of the underlying disease being treated. Also, a therapeutic benefit is achieved with the eradication or amelioration or reduction (or delay) of progress of one or more of the physiological symptoms associated with the underlying disease such that an improvement or slowing down or reduction of decline is observed in the patient, notwithstanding that the patient can still be afflicted with the underlying disease.

Herein, the term "outcome" relates to a specific result or effect that can be measured. Examples of an outcome of a subject having a glioma include an outcome of the glioma, a pathology outcome, an outcome of a therapy for treating the gliioma, such as a surgery outcome, a radiation therapy outcome, a chemotherapy outcome, and an immunotherapy outcome, as well as other outcomes, such as a biomarker related outcome (e.g., CEA (*Carcinoembryonic Antigen*)), a genomic profile related outcome, an imaging related outcome (e.g., a change in morphology or texture of the tumor), a biology related outcome (e.g., inflammation or immune response), a surrogate marker related outcome, a tumor size outcome, a treatment side effect outcome, a treatment toxicity outcome, a disease pain outcome, a quality of life outcome, a cancer specific survival, and an overall survival.

The term "clinical recurrence" refers to the presence of clinical signs indicating the presence of tumour cells as measured, for example using in vivo imaging.

When used herein, the term "immune defense response genes" is interchangeably used with "IDR genes" or "immune defense genes" and refers to one or more of the genes selected from AIM2, APOBEC3A, CIAO1, DDX58, DHX9, IFI16, IFIH1, IFIT1, IFIT3, LRRFIP1, MYD88, OAS1, TLR8, and ZBP1.

The term "metastases" refers to the presence of metastatic disease in organs other than a bladder tissue.

When used herein, the term "PDE4D7 correlated genes" is interchangeably used with "PDE4D7 genes" and refers to one or more of the genes selected from: ABCC5, CUX2, KIAA1549, PDE4D, RAP1GAP2, SLC39A11, TDRD1, and VWA2.

When used herein, the term "T-cell receptor signalling genes" is interchangeably used with "TCR signalling genes" or "TCR genes" and refers to one or more of the genes selected from: CD2, CD247, CD28, CD3E, CD3G, CD4, CSK, EZR, FYN, LAT, LCK, PAG1, PDE4D, PRKACA, PRKACB, PTPRC, and ZAP70.

DETAILED DESCRIPTION OF EMBODIMENTS

The section headings as used herein are for organizational purposes only and are not to be construed as limiting the subject matter described.

A portion of this invention contains material that is subject to copyright protection (such as, but not limited to, diagrams, device photographs, or any other aspects of this submission for which copyright protection is or may be available in any jurisdiction). The copyright owner has no objection to the facsimile reproduction by anyone of the patent document or patent invention, as it appears in the Patent Office patent file or records, but otherwise reserves all copyright rights whatsoever.

Various terms relating to the methods, compositions, uses and other aspects of the present invention are used throughout the specification and claims. Such terms are to be given their ordinary meaning in the art to which the invention relates, unless otherwise indicated. Other specifically defined terms are to be construed in a manner consistent with the definition as provided herein. The preferred materials and methods are described herein, although any methods and materials similar or equivalent to those described herein can be used in the practice for testing of the present invention.

Unless otherwise defined, all technical and scientific terms used herein have the same meaning as commonly understood by a person of ordinary skill in the art.

**Immune response defense genes**

The integrity and stability of genomics DNA is permanently under stress induced by various cell internal and external factors like exposure to radiation, viral or bacterial infections, but also oxidation and replication stress (see Gasser S. et al., "Sensing of dangerous DNA", Mechanisms of Aging and Development, Vol. 165, pages 33-46, 2017). In order to maintain DNA structure and stability, a cell must be able to recognize all types of DNA damages like single or double strand breaks etc. induced by various factors. This process involves the participation of a multitude of specific proteins depending on the kind of damage as part of DNA recognition pathways.

Recent evidence suggests that mis-localized DNA (e.g., DNA unnaturally appearing in the cytosolic fraction of the cell in contrast to the nucleus) and damaged DNA (e.g., through mutations occurring in cancer development) is used by the immune system to identify infected or otherwise diseased cells while genomic and mitochondrial DNA present in healthy cells is ignored by DNA recognition pathways. In diseased cells, cytosolic DNA sensor proteins have been demonstrated to be involved in the detection of DNA occurring unnaturally in the cytosol of the cell. Detection of such DNA by different nucleic acid sensors translates into similar responses leading to nuclear factor kappa-B (NF-kB) and interferon type I (IFN type I) signalling followed by the activation of innate immune system components. While the recognition of viral DNA is known to induce an IFN type I response, evidence that sensing of DNA damage can initiate immune responses has only recently been accumulating.

TLR9 (Toll-like receptor 9) located in the endosomes was one of the first DNA sensors molecules identified to be involved in the immune recognition of DNA by signalling downstream via the adaptor protein myeloid differentiation primary-response protein88 (MYD88). This interaction in turn activates mitogen-activated protein kinases (MAPKs) and NF-kB. TLR9 also induces the generation of type I interferons through the activation of IRF7 via IkB Kinase alpha (IKKalpha) in plasmacytoid dendritic cells (pDCs). Various other DNA immune receptors including IFI16 (IFN-gamma-inducible protein 16), cGAS (cyclic DMP-AMP synthase, DDX41 (DEAD-box helicase 41), as well as ZBP1 (Z-DNA-binding protein 1) interact with STING (stimulator of IFN genes), which activates the IKK complex and IRF3 through TBK1 (TANK binding kinase 1). ZBP1 also activates NF-kB via recruitment of RIP 1 and RIP3 (receptor-interacting protein 1 and 3, respectively). While the helicase DHX36 (DEAH-box helicase 36) interacts in a complex with TRID to induce NF-kB and IRF-3/7 the DHX9 helicase stimulates MYD88-dependent signalling in plasmacytoid dendritic cells. The DNA sensor LRRFIP1 (leucine-rich repeat flightless-interacting protein) complexes with beta-catenin to activate the transcription of IRF3 whereas AIM2 (absent in melanoma 2) recruits the adaptor protein ASC (apoptosis speck-like protein) to induce a caspase-1-activating inflammasome complex leading to the secretion of interleukin-1beta (IL-1beta) and IL-18 (see Fig. 1 of Gasser S. et al., 2017, ibid, which provides a schematic overview of DNA damage and DNA sensor pathways leading to the production of inflammatory cytokines and the expression of ligands for activating innate immune receptors. Members of the non-homologous end joining pathway (orange), homologous recombination (red), inflammasome (dark green), NF-kB and interferon responses (light green) are shown).

The factors and mechanisms responsible for activating the DNA sensor pathways in cancer are currently not well elucidated. It will be important to identify the intratumoral DNA species, sensors and pathways implicated in the expression of IFNs in different cancer types at all stages of the disease. In addition to therapeutic targets in cancer, such factors may also have prognostic and predictive value. Novel DNA sensor pathway agonists and antagonists are currently being developed and tested in preclinical trials. Such compounds will be useful in characterizing the role of DNA sensor pathways in the pathogenesis of cancer, autoimmunity and potentially other diseases.

## T-Cell receptor signaling genes

An immune response against pathogens can be elicited at different levels: there are physical barriers, such as the skin, to keep invaders out. If breached, innate immunity comes into play; a first and fast non-specific response. If this is not sufficient, the adaptive immune response is elicited. This is much more specific and needs time to develop when encountering a pathogen for the first time. Lymphocytes are activated by interacting with activated antigen presenting cells from the innate immune system, and are also responsible for maintenance of memory for faster responses upon next encounters with the same pathogen.

As lymphocytes are highly specific and effective when activated, they are subject to negative selection for their ability to recognize self, a process known as central tolerance. As not all self-antigens are expressed at selection sites, peripheral tolerance mechanisms evolved as well, such as ligation of the TCR in absence of co-stimulation, expression of inhibitory co-receptors, and suppression by Tregs. A disturbed balance between activation and suppression may lead to autoimmune disorders, or immune deficiencies and cancer, respectively.

T-cell activation can have different functional consequences, depending on the location the type of T-cell involved. CD8+ T-cells differentiate into cytotoxic effector cells, whereas CD4+ T-cells can differentiate into Th1 (IFNγ secretion and promotion of cell mediated immunity) or Th2 (IL4/5/13 secretion and promotion of B cell and humoral immunity). Differentiation towards other, more recently identified T-cell subsets is also possible, for example the Tregs, which have a suppressive effect on immune activation (see Mosenden R. and Tasken K., "Cyclic AMP-mediated immune regulation - Overview of mechanisms of action in T-cells", Cell Signal, Vol. 23, No. 6, pages 1009-1016, 2011, in particular, Fig. 4, which T-cell activation and its modulation by PKA, and Tasken K. and Ruppelt A., "Negative regulation of T-cell receptor activation by the cAMP-PKA-Csk signaling pathway in T-cell lipid rafts", Front Biosci, Vol. 11, pages 2929-2939, 2006).

Both PKA and PDE4 regulated signaling intersect with TCR induced T-cell activation to fine-tune its regulation, with opposing effects (see Abrahamsen H. et al., "TCR- and CD28-mediated recruitment of phosphodiesterase 4 to lipid rafts potentiates TCR signaling", J Immunol, Vol. 173, pages 4847-4848, 2004, in particular, Fig. 6, which shows opposing effects of PKA and PDE4 on TCR activation). The molecule that connects these effectors is cyclic AMP (cAMP), an intracellular second messenger of extracellular ligand action. In T-cells, it mediates effects of prostaglandins, adenosine, histamine, beta-adrenergic agonists, neuropeptide hormones and beta-endorphin. Binding of these extracellular molecules to GPCRs leads to their conformational change, release of stimulatory subunits and subsequent activation of adenylate cyclases (AC), which hydrolyze ATP to cAMP (see Fig. 6 of Abrahamsen H. et al., 2004, ibid). Although not the only one, PKA is the principal effector of cAMP signaling (see Mosenden R. and Tasken K., 2011, ibid, and Tasken K. and Ruppelt A., 2006, ibid). At a functional level, increased levels of cAMP lead to reduced IFNγ and IL-2 production in T-cells (see Abrahamsen H. et al., 2004, ibid). Aside from interfering with TCR activation, PKA has many more effector (see Fig. 15 of Torheim E.A., "Immunity Leashed - Mechanisms of Regulation in the Human Immune System", Thesis for the degree of Philosophiae Doctor (PhD), The Biotechnology Centre of Ola, University of Oslo, Norway, 2009).

In naive T-cells, hyperphosphorylated PAG targets Csk to lipid rafts. Via the Ezrin-EBP50-PAG scaffold complex PKA is targeted to Csk. Through specific phosphorylation by PKA, Csk can negatively regulate Lck and Fyn to dampen their activity and downregulate T-cell activation (see Fig. 6 of Abrahamsen H. et al., 2004, ibid). Upon TCR activation, PAG is dephosphorylated and Csk is released from the rafts. Dissociation of Csk is needed for T-cell activation to proceed. Within the same time course, a Csk-G3BP complex is formed and seems to sequester Csk outside lipid rafts (see Mosenden R. and Tasken K., 2011, ibid, and Tasken K. and Ruppelt A., 2006, ibid).

In contrast, combined TCR and CD28 stimulation mediates recruitment of the cyclic nucleotide phosphodiesterase PDE4 to lipid rafts, which enhances cAMP degradation (see Fig. 6 of Abrahamsen H. et al., 2004, ibid). As such, TCR induced production of cAMP is countered, and the T-cell immune response potentiated. Upon TCR stimulation alone, PDE4 recruitment may be too low to fully reduce the cAMP levels and therefore maximal T-cell activation cannot occur (see Abrahamsen H. et al., 2004, ibid).

Thus, by active suppression of proximal TCR signaling, signaling via cAMP-PKA-Csk is thought to set the threshold for T-cell activation. Recruitment of PDEs can counter this suppression. Tissue or cell-type specific regulation is accomplished through expression of multiple isoforms of AC, PKA, and PDEs. As mentioned above, the balance between activation and suppression needs to be tightly regulated to prevent development of autoimmune disorders, immune deficiencies and cancer.

**PDE4D7 correlated genes**

Phosphodiesterases (PDEs) provide the sole means for the degradation of the second messenger 3'-5'-cyclic AMP. As such they are poised to provide a key regulatory role. Thus, aberrant changes in their expression, activity and intracellular location may all contribute to the underlying molecular pathology of particular disease states. Indeed, it has recently been shown that mutations in PDE genes are enriched in prostate cancer patients leading to elevated cAMP signalling and a potential predisposition to prostate cancer. However, varied expression profiles in different cell types coupled with complex arrays of isoform variants within each PDE family makes understanding the links between aberrant changes in PDE expression and functionality during disease progression challenging. Several studies have endeavored to describe the complement of PDEs in prostate, all of which identified significant levels of PDE4 expression alongside other PDEs, leading to the development of a PDE4D7 biomarker (see Alves de Inda M. et al., "Validation of Cyclic Adenosine Monophosphate Phosphodiesterase-4D7 for its Independent Contribution to Risk Stratification in a Prostate Cancer Patient Cohort with Longitudinal Biological Outcomes", Eur Urol Focus, Vol. 4, No. 3, pages 376-384, 2018). Since the PDE4D7 biomarker has been proven to be a good predictor, we assumed that the ability to identify markers that are highly correlated with the PDE47 biomarker might also be helpful in prognosticating the outcome of certain cancer subjects.

**Selection of the genes**

The lists of genes have originally been selected by us for prognostication in prostate cancer subjects. In this document, it is shown that they are also of prognostic value with respect to an outcome of a subject having a glioma.

The identified immune defense response genes ZBP1, and AIM2, APOBEC3A, CIAO1, DDX58, DHX9, IFI16, IFIH1, IFIT1, IFIT3, LRRFIP1, MYD88, OAS1, TLR8, and ZBP1, respectively, were identified as follows: A group of 538 prostate cancer patients were treated with RP and the prostate cancer tissue was stored together with clinical (e.g., pathological Gleason grade group (pGGG), pathology state (pT stage)) as well as relevant outcome parameters (e.g., biochemical recurrence (BCR), metastatic recurrence, prostate cancer specific death (PCa death), salvage radiation treatment (SRT), salvage androgen deprivation treatment (SADT), chemotherapy (CTX)). For each of these patients, a PDE4D7 score was calculated and categorized into four PDE4D7 score classes (see Alves de Inda M. et al., 2018, ibid). PDE4D7 score class 1 represents patient samples with lowest expression levels of PDE4D7, whereas PDE4D7 score class 4 represents patient samples with highest levels of PDE4D7 expression. RNASeq expression data (TPM - Transcripts Per Million) of the 538 prostate cancer subjects was then investigated for differential gene expression between the PDE4D7 score classes 1 and 4. In particular, it was determined for around 20,000 protein coding transcripts whether the mean expression level of the PDE4D7 score class 1 patients was more than twice as high as the mean expression level of the PDE4D7 score class 4 patients. This analysis resulted in 637 genes with a ratio PDE4D7 score class 1 / PDE4D7 score class 4 of > 2 with a minimum mean expression of 1 TPM in each of the four PDE4D7 score classes. These 637 genes were then further subjected to molecular pathway analysis (www.david.ncifcrf.gov), which resulted in a range of enriched annotation clusters. The annotation cluster #2 demonstrated enrichment (enrichment score: 10.8) in 30 genes with a function in defense response to viruses, negative regulation of viral genome replication as well as type I interferon signaling. A further heat map analysis confirmed that these immune defense response genes were generally higher expressed in samples from patients in PDE4D7 score class 1 than from patients in PDE4D7 score class 4. The class of genes with a function in defense response to viruses, negative regulation of viral genome replication as well as type I interferon signaling was further enriched to 61 genes by literature search to identify additional genes with the same molecular function. A further selection from the 61 genes was made based on the combinatorial power to separate patients who died from prostate cancer vs. those who did not, resulting of a preferred set of 14 genes. It was found that the number of events (metastases, prostate cancer specific death) was enriched in sub-cohorts with a low expression of these genes compared to the total patient cohort (#538) and a sub-cohort of 151 patients undergoing salvage RT (SRT) after post-surgical disease recurrence.

The identified T-Cell receptor signaling genes CD2, CD247, CD28, CD3E, CD3G, CD4, CSK, EZR, FYN, LAT, LCK, PAG1, PDE4D, PRKACA, PRKACB, PTPRC, and ZAP70 were identified as follows: A group of 538 prostate cancer patients were treated with RP and the prostate cancer tissue was stored together with clinical (e.g., pathological Gleason grade group (pGGG), pathology state (pT stage)) as well as relevant outcome parameters (e.g., biochemical recurrence (BCR), metastatic recurrence, prostate cancer specific death (PCa death), salvage radiation treatment (SRT), salvage androgen deprivation treatment (SADT), chemotherapy (CTX)). For each of these patients, a PDE4D7 score was calculated and categorized into four PDE4D7 score classes (see Alves de Inda M. et al., 2018, ibid). PDE4D7 score class 1 represents patient samples with lowest expression levels of PDE4D7, whereas PDE4D7 score class 4 represents patient samples with highest levels of PDE4D7 expression. RNASeq expression data (TPM - Transcripts Per Million) of the 538 prostate cancer subjects was then investigated for differential gene expression between the PDE4D7 score classes 1 and 4. In particular, it was determined for around 20,000 protein coding transcripts whether the mean expression level of the PDE4D7 score class 1 patients was more than twice as high as the mean expression level of the PDE4D7 score class 4 patients. This analysis resulted in 637 genes with a ratio PDE4D7 score class 1 / PDE4D7 score class 4 of > 2 with a minimum mean expression of 1 TPM in each of the four PDE4D7 score classes. These 637 genes were then further subjected to molecular pathway analysis (www.david.ncifcrf.gov), which resulted in a range of enriched annotation clusters. The annotation cluster #6 demonstrated enrichment (enrichment score: 5.9) in 17 genes with a function in primary immune deficiency and activation of T-Cell receptor signaling. A further heat map analysis confirmed that these T-Cell receptor signaling genes were generally higher expressed in samples from patients in PDE4D7 score class 1 than from patients in PDE4D7 score class 4.

The identified PDE4D7 correlated genes ABCC5, CUX2, KIAA1549, PDE4D, RAP1GAP2, SLC39A11, TDRD1, and VWA2 were identified as follows: We have identified in RNAseq data generated on 571 prostate cancer patients on close to 60,000 transcripts a range of genes that are correlated to the expression of the known biomarker PDE4D7 in this data. The correlation between the expression of any of these genes and PDE4D7 across the 571 samples was done by Pearson correlation and is expressed as a value between 0 to 1 in case of positive correlation or a value between -1 to 0 in case of negative correlation. As input data for the calculation of the correlation coefficient we used the PDE4D7 score (see Alves de Inda M. et al., 2018, ibid) and the RNAseq determined TPM gene expression value per gene of interest (see below).

The maximum negative correlation coefficient identified between the expression of any of the approximately 60,000 transcripts and the expression of PDE4D7 was -0.38 while the maximum positive correlation coefficient identified between the expression of any of the approximately 60,000 transcripts and the expression of PDE4D7 was +0.56. We selected genes in the range of correlation -0.31 to -0.38 as well as +0.41 to +0.56. We identified in total 77 transcripts matching these characteristics. From those 77 transcripts we selected the eight PDE4D7 correlated genes ABCC5, CUX2, KIAA1549, PDE4D, RAP1GAP2, SLC39A11, TDRD1, and VWA2 by testing Cox regression combination models iteratively in a sub-cohort of 186 patients who were undergoing salvage radiation treatment (SRT) due to post-surgical biochemical relapse. The clinical endpoint tested was prostate cancer specific death after start of SRT. The boundary condition for the selection of the eight genes was given by the restriction that the p-values in the multivariate Cox-regression were <0.1 for all genes retained in the model.

## Use of the gene signatures in gliomas

In this document, it is shown that these genes are also of prognostic value with respect to an outcome of a subject suffering from a glioma. Herein the inventors describe data obtained from different glioma patients that the gene signatures "Immune defense response genes", "T-cell receptor signaling genes", and "PDE4D7 correlated genes" each individually or their combination (GLCAI score) could stratify patients based on the end point overall death (survival time in months).

The data described below in the examples describes the testing and validation of the IDR, TCR and PDE4D7 correlated gene signatures individually or combined (GLCAI score) on three different patient cohorts, TCGA, GSE16011 and GSE108476. The TCGA cohort comprises gene expression data from patients having astrocytoma, oligoastrocytoma, oligodendroglioma, treated glioblastoma multiforme, untreated (primary) glioblastoma multiforme. The GSE16011 cohort comprises gene expression data from patients having astrocytic tumors (incl pilocytic astrocytomas (PA), astrocytomas, and glioblastomas (GBM)), and oligodendroglial (OD) tumors (incl pure OD tumors and mixed oligoastrocytic (MOA) tumors). The GSE108476 cohort comprises gene expression data from patients having Astrocytoma, GBM, mixed, non-tumor (control samples), oligodendroglioma, or an unknown glioma type. In each cohort a training set was used to train the models (IDR, TCR, PDE4D7 correlated and GLCAI), the trained models were then tested on a test data set. The end point use to train the model was survival status based on survival time in months. The gene signatures allowed to distinguish between poor and improved survival time.

Therefore in a first aspect the invention relates to method of predicting an outcome of a subject having a glioma, the method comprising:

- determining or receiving the result of a determination of six or more gene expression levels selected from a first gene expression profile, a second gene expression profile and/or a third gene expression profile, wherein
- the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1;
- the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70;
- the third gene expression profile consists of the PDE4D7 correlated genes selected from the group consisting of: ABCC5, CUX2, KIAA1549, PDE4D, RAP1GAP2, SLC39A11, TDRD1, and VWA2;
- said gene expression levels being determined in a biological sample obtained from the subject; and
- determining the prediction of the outcome based on six or more gene expression levels.

In an embodiment the invention provides for a computer implemented method of predicting an outcome of a subject having a glioma, the method comprising:

- receiving the result of a determination of six or more gene expression levels selected from a first gene expression profile, a second gene expression profile and/or a third gene expression profile, wherein
- the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1;
- the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70;
- the third gene expression profile consists of the PDE4D7 correlated genes selected from the group consisting of: ABCC5, CUX2, KIAA1549, PDE4D, RAP1GAP2, SLC39A11, TDRD1, and VWA2;
- said gene expression levels being determined in a biological sample obtained from the subject; and
- determining the prediction of the outcome based on six or more gene expression levels.

The inventors describe herein that the gene signatures or selection of their individual genes may be used to predict survival of a patient diagnosed with a glioma. Therefore in an embodiment the invention provides for a method of predicting an outcome of a subject having a glioma, comprising: determining or receiving the result of a determination of a gene expression profile comprising six or more, such as 6, 7, 8, 9, 10, 11, 12, 13, 14, 16, 17, 18, 19, 20, 21, 22, 23, 24, 25, 26, 27, 28, 29, 30, 31, 32, 33, 34, 35, 36, 37, 38 or even 39, gene expression levels, wherein the six or more gene expression levels are selected from: immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or PDE4D7 correlated genes selected from the group consisting of: ABCC5, CUX2, KIAA1549, PDE4D, RAP1GAP2, SLC39A11, TDRD1, and VWA2, said gene expression profile being determined in a biological sample obtained from the subject, determining the prediction of the outcome based on the gene expression profile, wherein said prediction is survival. The survival may refer to overall survival, or cancer specific death.

Patients diagnosed with a glioma generally have a poor prognosis, however distinction can be made between a favorable and a unfavorable outcome. In this context a favorable outcome is understood as a longer predicted survival time and an unfavorable outcome is understood as a shorter predicted survival time, for example compared to the average or mean survival time for a patient diagnosed with a glioma. The present method has shown to be able to stratify patients for having a favorable or unfavorable outcome. This holds relevance for example for treatment recommendation, which may be adapted based on the predicted outcome (favorable or unfavorable). For example, for a patient having a predicted favorable outcome, one of the following treatment options may be considered: *Surgery*: Surgical removal of the tumor is often the first line of treatment when feasible. However, in cases of gliomas with a poor prognosis, complete removal may not be possible due to the tumor's location or invasive nature.

*Radiation therapy*: Radiation therapy uses high-energy beams to target and kill cancer cells. It is commonly used in the treatment of gliomas, including those with a poor prognosis. Different techniques, such as stereotactic radiosurgery or fractionated radiation, may be employed depending on the tumor characteristics.

*Chemotherapy*: Chemotherapy involves the use of drugs to destroy cancer cells. Temozolomide is a commonly used chemotherapy drug for gliomas and may be administered in conjunction with radiation therapy, however other chemotherapies are known and may be used instead.

*Targeted therapies*: Some gliomas may have specific genetic mutations that can be targeted with specific drugs. For example, certain gliomas with mutations in the IDH1 or IDH2 genes may respond to targeted therapies like ivosidenib or enasidenib. Other targeted therapies are known and may be used instead.

For example, for a patient having a predicted unfavorable outcome, one of the following treatment options may be considered:

*Combination therapy*: a combination of two or more therapies selected from surgery, chemotherapy radiotherapy and targeted therapy.

*Immunotherapy*: Immunotherapy aims to enhance the body's immune response to fight cancer. While it has shown promising results in other types of cancers, its effectiveness in gliomas is still being studied, and its use in cases with poor prognosis is limited.

*Clinical trials*: Clinical trials may provide access to novel treatments or experimental therapies that are not yet widely available. Participation in clinical trials should be considered after carefully evaluating the potential risks and benefits.

The inventors further describe that a treatment response of a subject having a glioma may be predicted using the gene signatures described herein or individual genes selected therefrom (e.g. six or more genes). Therefore in a an embodiment the invention provides for a method of predicting an outcome of subject having a glioma, comprising: determining or receiving the result of a determination of a gene expression profile comprising three or more, such as 3, 4, 5, 6, 7, 8, 9, 10, 11, 12, 13, 14, 16, 17, 18, 19, 20, 21, 22, 23, 24, 25, 26, 27, 28, 29, 30, 31, 32, 33, 34, 35, 36, 37, 38 or even 39, gene expression levels, wherein the three or more gene expression levels are selected from: immune defence response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or PDE4D7 correlated genes selected from the group consisting of: ABCC5, CUX2, KIAA1549, PDE4D, RAP1GAP2, SLC39A11, TDRD1, and VWA2, said gene expression profile being determined in a biological sample obtained from the subject, determining the prediction of the outcome based on the gene expression profile, wherein said prediction is a treatment response. The treatment may for example be radiotherapy, surgery, chemotherapy, targeted therapy, immunotherapy or a combination thereof.

The present invention describes the use of a gene signature to predict the outcome of a subject with glioma. The gene signature comprises a gene expression profile comprising six or more, such as 6, 7, 8, 9, 10, 11, 12, 13, 14, 16, 17, 18, 19, 20, 21, 22, 23, 24, 25, 26, 27, 28, 29, 30, 31, 32, 33, 34, 35, 36, 37, 38 or even 39, gene expression levels, wherein the three or more gene expression levels are selected from: immune defense response genes and/or T-cell receptor signaling genes and/or PDE4D7 correlated genes. Therefore in an embodiment the six or more genes may be selected from immune defense response genes. In an embodiment the six or more genes may be selected from the T-cell receptor signaling genes. In an embodiment the six or more genes may be selected from the PDE4D7 correlated genes. In an embodiment the six or more genes comprise one or more immune defense response genes and one or more T-cell receptor signaling genes. In an embodiment the six or more genes comprise one or more immune defense response genes and one or more PDE4D7 correlated genes. In an embodiment the six or more genes comprise one or more PDE4D7 correlated genes and one or more T-cell receptor signaling genes. In an embodiment the six or more genes comprise one or more immune defense response genes and one or more T-cell receptor signaling genes and one or more PDE4D7 correlated genes.

With respect to the biological processes described above, three immune system related gene signatures were selected, including the genes listed in tables 1-3. Before, the relevance of these signatures to prediction of prostate cancer survival was shown.

**Table 1:** Immune Defence Response (IDR) Signature.

| Gene Symbol | Ensembl_ID |
|---|---|
| AIM2 | ENSG00000163568 |
| APOBEC3A | ENSG00000128383 |
| CIAO1 | ENSG00000144021 |
| DDX58 | ENSG00000107201 |
| DHX9 | ENSG00000135829 |
| IFI16 | ENSG00000163565 |
| IFIH1 | ENSG00000115267 |
| IFIT1 | ENSG00000185745 |
| IFIT3 | ENSG00000119917 |
| LRRFIP1 | ENSG00000124831 |
| MYD88 | ENSG00000172936 |

(continued)

| Gene Symbol | Ensembl_ID |
|---|---|
| OAS1 | ENSG00000089127 |
| TLR8 | ENSG00000101916 |
| ZBP1 | ENSG00000124256 |

**Table 2:** T-Cell Receptor (TCR) Signature.

| Gene Symbol | Ensembl_ID |
|---|---|
| CD2 | ENSG00000116824 |
| CD247 | ENSG00000198821 |
| CD28 | ENSG00000178562 |
| CD3E | ENSG00000198851 |
| CD3G | ENSG00000160654 |
| CD4 | ENSG00000010610 |
| CSK | ENSG00000103653 |
| EZR | ENSG00000092820 |
| FYN | ENSG00000010810 |
| LAT | ENSG00000213658 |
| LCK | ENSG00000182866 |
| PAG1 | ENSG00000076641 |
| PDE4D | ENSG00000113448 |
| PRKACA | ENSG00000072062 |
| PRKACB | ENSG00000142875 |
| PTPRC | ENSG00000081237 |
| ZAP70 | ENSG00000115085 |

**Table 3:** PDE4D7 Correlated (PDE4D7_R2) signature.

| Gene Symbol | Ensembl_ID |
|---|---|
| ABCC5 | ENSG00000114770 |
| CUX2 | ENSG00000111249 |
| HIAA1549 | ENSG00000122778 |
| PDE4D | ENSG00000113448 |
| RAP1GAP2 | ENSG00000132359 |
| SLC39A11 | ENSG00000133195 |
| TDRD1 | ENSG00000095627 |
| VWA2 | ENSG00000165816 |

It was found that the genes AIM2, APOBEC3A, CIAO1, DDX58, DHX9, IFI16, IFIH1, IFIT1, IFIT3, LRRFIP1, MYD88, OAS1, TLR8, ZBP1, CD2, CD247, CD28, CD3E, CD3G, CD4, CSK, EZR, FYN, LAT, LCK, PAG1, PDE4D, PRKACA, PRKACB, PTPRC, ZAP70, ABCC5, CUX2, KIAA1549, PDE4D, RAP1GAP2, SLC39A11, TDRD1, and VWA2 each individually, one or more per gene panel, in a combination of one or more per gene panel or when all combined, are able to predict the outcome in a subject having a glioma.

The term "ABCC5" refers to the human ATP binding cassette subfamily C member 5 gene (Ensembl:

ENSG00000114770), for example, to the sequence as defined in NCBI Reference Sequence NM_001023587.2 or in NCBI Reference Sequence NM_005688.3, specifically, to the nucleotide sequence as set forth in SEQ ID NO:1 or in SEQ ID NO:2, which correspond to the sequences of the above indicated NCBI Reference Sequences of the ABCC5 transcript, and also relates to the corresponding amino acid sequence for example as set forth in SEQ ID NO:3 or in SEQ ID NO:4, which correspond to the protein sequences defined in NCBI Protein Accession Reference Sequence NP_001018881.1 and in NCBI Protein Accession Reference Sequence NP_005679 encoding the ABCC5 polypeptide.

The term "ABCC5" also comprises nucleotide sequences showing a high degree of homology to ABCC5, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 1 or in SEQ ID NO:2 or amino acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:1 or in SEQ ID NO:2.

The term "AIM2" refers to the Absent in Melanoma 2 gene (Ensembl: ENSG00000163568), for example, to the sequence as defined in NCBI Reference Sequence NM_004833, specifically, to the nucleotide sequence as set forth in SEQ ID NO:5, which corresponds to the sequence of the above indicated NCBI Reference Sequence of the AIM2 transcript, and also relates to the corresponding amino acid sequence for example as set forth in SEQ ID NO:6, which corresponds to the protein sequence defined in NCBI Protein Accession Reference Sequence NP_004824 encoding the AIM2 polypeptide.

The term "AIM2" also comprises nucleotide sequences showing a high degree of homology to AIM2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:5 or amino acid sequences being at least 75%, 80%, 85%, 90%, 91%, 92%, 93%, 94%, 95%, 96%, 97%, 98% or 99% identical to the sequence as set forth in SEQ ID NO:6 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:6 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:5.

The term "APOBEC3A" refers to the Apolipoprotein B mRNA Editing Enzyme Catalytic Subunit 3A gene (Ensembl: ENSG00000128383), for example, to the sequence as defined in NCBI Reference Sequence NM_145699, specifically, to the nucleotide sequence as set forth in SEQ ID NO:7, which corresponds to the sequence of the above indicated NCBI Reference Sequence of the APOBEC3A transcript, and also relates to the corresponding amino acid sequence for example as set forth in SEQ ID NO:8, which corresponds to the protein sequence defined in NCBI Protein Accession Reference Sequence NP_663745 encoding the APOBEC3A polypeptide.

The term "APOBEC3A" also comprises nucleotide sequences showing a high degree of homology to APOBEC3A, e.g., nucleic acid sequences being at least 75%, 80%, 85%, 90%, 91%, 92%, 93%, 94%, 95%, 96%, 97%, 98% or 99% identical to the sequence as set forth in SEQ ID NO:7 or amino acid sequences being at least 75%, 80%, 85%, 90%, 91%, 92%, 93%, 94%, 95%, 96%, 97%, 98% or 99% identical to the sequence as set forth in SEQ ID NO:8 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:8 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:7.

The term "CD2" refers to the Cluster Of Differentiation 2 gene (Ensembl: ENSG00000116824), for example, to the sequence as defined in NCBI Reference Sequence NM_001767, specifically, to the nucleotide sequence as set forth in SEQ ID NO:9, which corresponds to the sequence of the above indicated NCBI Reference Sequence of the CD2 transcript, and also relates to the corresponding amino acid sequence for example as set forth in SEQ ID NO: 10, which corresponds to the protein sequence defined in NCBI Protein Accession Reference Sequence NP_001758 encoding the CD2 polypeptide.

The term "CD2" also comprises nucleotide sequences showing a high degree of homology to CD2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:9 or amino acid sequences being at least 75%, 80%, 85%, 90%, 91%, 92%, 93%, 94%, 95%, 96%, 97%, 98% or 99% identical to the sequence as set forth in SEQ ID NO: 10 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 10 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:9.

The term "CD247" refers to the Cluster Of Differentiation 247 gene (Ensembl: ENSG00000198821), for example, to the sequence as defined in NCBI Reference Sequence NM_000734 or in NCBI Reference Sequence NM_198053, specifically, to the nucleotide sequence as set forth in SEQ ID NO: 11 or in SEQ ID NO: 12, which correspond to the sequences of the above indicated NCBI Reference Sequences of the CD247 transcript, and also relates to the corresponding amino acid sequence for example as set forth in SEQ ID NO: 13 or in SEQ ID NO: 14, which correspond to the protein sequences defined in NCBI Protein Accession Reference Sequence NP_000725 and in NCBI Protein Accession Reference Sequence NP_932170 encoding the CD247 polypeptide.

The term "CD247" also comprises nucleotide sequences showing a high degree of homology to CD247, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 11 or in SEQ ID NO: 12 or amino acid sequences being at least 75%, 80%, 85%, 90%, 91%, 92%, 93%, 94%, 95%, 96%, 97%, 98% or 99% identical to the sequence as set forth in SEQ ID NO: 13 or in SEQ ID NO: 14 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 13 or in SEQ ID NO: 14 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 11 or in SEQ ID NO: 12.

The term "CD28" refers to the Cluster Of Differentiation 28 gene (Ensembl: ENSG00000178562), for example, to the sequence as defined in NCBI Reference Sequence NM_006139 or in NCBI Reference Sequence NM_001243078, specifically, to the nucleotide sequence as set forth in SEQ ID NO: 15 or in SEQ ID NO: 16, which correspond to the sequences of the above indicated NCBI Reference Sequences of the CD28 transcript, and also relates to the corresponding amino acid sequence for example as set forth in SEQ ID NO: 17 or in SEQ ID NO: 18, which correspond to the protein sequences defined in NCBI Protein Accession Reference Sequence NP_006130 and in NCBI Protein Accession Reference Sequence NP_001230007 encoding the CD28 polypeptide.

The term "CD28" also comprises nucleotide sequences showing a high degree of homology to CD28, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 15 or in SEQ ID NO: 16 or amino acid sequences being at least 75%, 80%, 85%, 90%, 91%, 92%, 93%, 94%, 95%, 96%, 97%, 98% or 99% identical to the sequence as set forth in SEQ ID NO: 17 or in SEQ ID NO: 18 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 17 or in SEQ ID NO: 18 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 15 or in SEQ ID NO: 16.

The term "CD3E" refers to the Cluster Of Differentiation 3E gene (Ensembl: ENSG00000198851), for example, to the sequence as defined in NCBI Reference Sequence NM_000733, specifically, to the nucleotide sequence as set forth in SEQ ID NO: 19, which corresponds to the sequence of the above indicated NCBI Reference Sequence of the CD3E transcript, and also relates to the corresponding amino acid sequence for example as set forth in SEQ ID NO:20, which corresponds to the protein sequence defined in NCBI Protein Accession Reference Sequence NP_000724 encoding the CD3E polypeptide.

The term "CD3E" also comprises nucleotide sequences showing a high degree of homology to CD3E, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 19 or amino acid sequences being at least 75%, 80%, 85%, 90%, 91%, 92%, 93%, 94%, 95%, 96%, 97%, 98% or 99% identical to the sequence as set forth in SEQ ID NO:20 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:20 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 19.

The term "CD3G" refers to the Cluster Of Differentiation 3G gene (Ensembl: ENSG00000160654), for example, to the sequence as defined in NCBI Reference Sequence NM_000073, specifically, to the nucleotide sequence as set forth in SEQ ID NO:21, which corresponds to the sequence of the above indicated NCBI Reference Sequence of the CD3G transcript, and also relates to the corresponding amino acid sequence for example as set forth in SEQ ID NO:22, which corresponds to the protein sequence defined in NCBI Protein Accession Reference Sequence NP_000064 encoding the CD3G polypeptide.

The term "CD3G" also comprises nucleotide sequences showing a high degree of homology to CD3G, e.g., nucleic acid sequences being at least 75%, 80%, 85%, 90%, 91%, 92%, 93%, 94%, 95%, 96%, 97%, 98% or 99% identical to the sequence as set forth in SEQ ID NO:21 or amino acid sequences being at least 75%, 80%, 85%, 90%, 91%, 92%, 93%, 94%, 95%, 96%, 97%, 98% or 99% identical to the sequence as set forth in SEQ ID NO:22 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:22 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:21.

The term "CD4" refers to the Cluster Of Differentiation 4 gene (Ensembl: ENSG00000010610), for example, to the sequence as defined in NCBI Reference Sequence NM_000616, specifically, to the nucleotide sequence as set forth in SEQ ID NO:23, which corresponds to the sequence of the above indicated NCBI Reference Sequence of the CD4 transcript, and also relates to the corresponding amino acid sequence for example as set forth in SEQ ID NO:24, which corresponds to the protein sequence defined in NCBI Protein Accession Reference Sequence NP_000607 encoding the CD4 polypeptide.

The term "CD4" also comprises nucleotide sequences showing a high degree of homology to CD4, e.g., nucleic acid sequences being at least 75%, 80%, 85%, 90%, 91%, 92%, 93%, 94%, 95%, 96%, 97%, 98% or 99% identical to the sequence as set forth in SEQ ID NO:23 or amino acid sequences being at least 75%, 80%, 85%, 90%, 91%, 92%, 93%, 94%, 95%, 96%, 97%, 98% or 99% identical to the sequence as set forth in SEQ ID NO:24 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:24 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:23.

The term "CIAO1" refers to the Cytosolic Iron-Sulfur Assembly Component 1 gene (Ensembl: ENSG00000144021), for example, to the sequence as defined in NCBI Reference Sequence NM_004804, specifically, to the nucleotide sequence as set forth in SEQ ID NO:25, which corresponds to the sequence of the above indicated NCBI Reference Sequence of the CIAO1 transcript, and also relates to the corresponding amino acid sequence for example as set forth in SEQ ID NO:26, which corresponds to the protein sequence defined in NCBI Protein Accession Reference Sequence NP_663745 encoding the CIAO1 polypeptide.

The term "CIAO1" also comprises nucleotide sequences showing a high degree of homology to CIAO1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:25 or amino acid sequences being at least 75%, 80%, 85%, 90%, 91%, 92%, 93%, 94%, 95%, 96%, 97%, 98% or 99% identical to the sequence as set forth in SEQ ID NO:26 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:26 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:25.

The term "CSK" refers to the C-Terminal Src Kinase gene (Ensembl: ENSG00000103653), for example, to the sequence as defined in NCBI Reference Sequence NM_004383, specifically, to the nucleotide sequence as set forth in SEQ ID NO:27, which corresponds to the sequence of the above indicated NCBI Reference Sequence of the CSK transcript, and also relates to the corresponding amino acid sequence for example as set forth in SEQ ID NO:28, which corresponds to the protein sequence defined in NCBI Protein Accession Reference Sequence NP_004374 encoding the CSK polypeptide.

The term "CSK" also comprises nucleotide sequences showing a high degree of homology to CSK, e.g., nucleic acid sequences being at least 75%, 80%, 85%, 90%, 91%, 92%, 93%, 94%, 95%, 96%, 97%, 98% or 99% identical to the sequence as set forth in SEQ ID NO:27 or amino acid sequences being at least 75%, 80%, 85%, 90%, 91%, 92%, 93%, 94%, 95%, 96%, 97%, 98% or 99% identical to the sequence as set forth in SEQ ID NO:28 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:28 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:27.

The term "CUX2" refers to the human Cut Like Homeobox 2 gene (Ensembl: ENSG00000111249), for example, to the sequence as defined in NCBI Reference Sequence NM_015267.3, specifically, to the nucleotide sequence as set forth in SEQ ID NO:29, which corresponds to the sequence of the above indicated NCBI Reference Sequence of the CUX2 transcript, and also relates to the corresponding amino acid sequence for example as set forth in SEQ ID NO:30, which corresponds to the protein sequence defined in NCBI Protein Accession Reference Sequence NP_056082.2 encoding the CUX2 polypeptide.

The term "CUX2" also comprises nucleotide sequences showing a high degree of homology to CUX2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:29 or amino acid sequences being at least 75%, 80%, 85%, 90%, 91%, 92%, 93%, 94%, 95%, 96%, 97%, 98% or 99% identical to the sequence as set forth in SEQ ID NO:30 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:30 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:29.

The term "DDX58" refers to the DExD/H-box Helicase 58 gene (Ensembl: ENSG00000107201), for example, to the sequence as defined in NCBI Reference Sequence NM_014314, specifically, to the nucleotide sequence as set forth in SEQ ID NO:31, which corresponds to the sequence of the above indicated NCBI Reference Sequence of the DDX58 transcript, and also relates to the corresponding amino acid sequence for example as set forth in SEQ ID NO:32, which corresponds to the protein sequence defined in NCBI Protein Accession Reference Sequence NP_055129 encoding the DDX58 polypeptide.

The term "DDX58" also comprises nucleotide sequences showing a high degree of homology to DDX58, e.g., nucleic acid sequences being at least 75%, 80%, 85%, 90%, 91%, 92%, 93%, 94%, 95%, 96%, 97%, 98% or 99% identical to the sequence as set forth in SEQ ID NO:31 or amino acid sequences being at least 75%, 80%, 85%, 90%, 91%, 92%, 93%, 94%, 95%, 96%, 97%, 98% or 99% identical to the sequence as set forth in SEQ ID NO:32 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:32 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:31.

The term "DHX9" refers to the DExD/H-box Helicase 9 gene (Ensembl: ENSG00000135829), for example, to the sequence as defined in NCBI Reference Sequence NM_001357, specifically, to the nucleotide sequence as set forth in SEQ ID NO:33, which corresponds to the sequence of the above indicated NCBI Reference Sequence of the DHX9 transcript, and also relates to the corresponding amino acid sequence for example as set forth in SEQ ID NO:34, which corresponds to the protein sequence defined in NCBI Protein Accession Reference Sequence NP_001348 encoding the DHX9 polypeptide.

The term "DHX9" also comprises nucleotide sequences showing a high degree of homology to DHX9, e.g., nucleic acid sequences being at least 75%, 80%, 85%, 90%, 91%, 92%, 93%, 94%, 95%, 96%, 97%, 98% or 99% identical to the sequence as set forth in SEQ ID NO:33 or amino acid sequences being at least 75%, 80%, 85%, 90%, 91%, 92%, 93%, 94%, 95%, 96%, 97%, 98% or 99% identical to the sequence as set forth in SEQ ID NO:34 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:34 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:33.

The term "EZR" refers to the Ezrin gene (Ensembl: ENSG00000092820), for example, to the sequence as defined in NCBI Reference Sequence NM_003379, specifically, to the nucleotide sequence as set forth in SEQ ID NO:35, which corresponds to the sequence of the above indicated NCBI Reference Sequence of the EZR transcript, and also relates to the corresponding amino acid sequence for example as set forth in SEQ ID NO:36, which corresponds to the protein sequence defined in NCBI Protein Accession Reference Sequence NP_003370 encoding the EZR polypeptide.

The term "EZR" also comprises nucleotide sequences showing a high degree of homology to EZR, e.g., nucleic acid sequences being at least 75%, 80%, 85%, 90%, 91%, 92%, 93%, 94%, 95%, 96%, 97%, 98% or 99% identical to the sequence as set forth in SEQ ID NO:35 or amino acid sequences being at least 75%, 80%, 85%, 90%, 91%, 92%, 93%, 94%, 95%, 96%, 97%, 98% or 99% identical to the sequence as set forth in SEQ ID NO:36 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:36 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:35.

The term "FYN" refers to the FYN Proto-Oncogene gene (Ensembl: ENSG00000010810), for example, to the sequence as defined in NCBI Reference Sequence NM_002037 or in NCBI Reference Sequence NM_153047 or in NCBI Reference Sequence NM_153048, specifically, to the nucleotide sequence as set forth in SEQ ID NO:37 or in SEQ ID NO:38 or in SEQ ID NO:39, which correspond to the sequences of the above indicated NCBI Reference Sequences of the FYN transcript, and also relates to the corresponding amino acid sequence for example as set forth in SEQ ID NO:40 or in SEQ ID NO:41 or in SEQ ID NO:42, which correspond to the protein sequences defined in NCBI Protein Accession Reference Sequence NP_002028 and in NCBI Protein Accession Reference Sequence NP_694592 and in NCBI Protein Accession Reference Sequence XP_005266949 encoding the FYN polypeptide.

The term "FYN" also comprises nucleotide sequences showing a high degree of homology to FYN, e.g., nucleic acid sequences being at least 75%, 80%, 85%, 90%, 91%, 92%, 93%, 94%, 95%, 96%, 97%, 98% or 99% identical to the sequence as set forth in SEQ ID NO:37 or in SEQ ID NO:38 or in SEQ ID NO:39 or amino acid sequences being at least 75%, 80%, 85%, 90%, 91%, 92%, 93%, 94%, 95%, 96%, 97%, 98% or 99% identical to the sequence as set forth in SEQ ID NO:40 or in SEQ ID NO:41 or in SEQ ID NO:42 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:40 or in SEQ ID NO:41 or in SEQ ID NO:42 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:37 or in SEQ ID NO:38 or in SEQ ID NO:39.

The term "IFI16" refers to the Interferon Gamma Inducible Protein 16 gene (Ensembl: ENSG00000163565), for example, to the sequence as defined in NCBI Reference Sequence NM_005531, specifically, to the nucleotide sequence as set forth in SEQ ID NO:43, which corresponds to the sequence of the above indicated NCBI Reference Sequence of the IFI16 transcript, and also relates to the corresponding amino acid sequence for example as set forth in SEQ ID NO:44, which corresponds to the protein sequence defined in NCBI Protein Accession Reference Sequence NP_005522 encoding the IFI16 polypeptide.

The term "IFI16" also comprises nucleotide sequences showing a high degree of homology to IFI16, e.g., nucleic acid sequences being at least 75%, 80%, 85%, 90%, 91%, 92%, 93%, 94%, 95%, 96%, 97%, 98% or 99% identical to the sequence as set forth in SEQ ID NO:43 or amino acid sequences being at least 75%, 80%, 85%, 90%, 91%, 92%, 93%, 94%, 95%, 96%, 97%, 98% or 99% identical to the sequence as set forth in SEQ ID NO:44 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:44 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:43.

The term "IFIH1" refers to the Interferon Induced With Helicase C Domain 1 gene (Ensembl: ENSG00000115267), for example, to the sequence as defined in NCBI Reference Sequence NM_022168, specifically, to the nucleotide sequence as set forth in SEQ ID NO:45, which corresponds to the sequence of the above indicated NCBI Reference Sequence of the IFIH1 transcript, and also relates to the corresponding amino acid sequence for example as set forth in SEQ ID NO:46, which corresponds to the protein sequence defined in NCBI Protein Accession Reference Sequence NP_071451 encoding the IFIH1 polypeptide.

The term "IFIH1" also comprises nucleotide sequences showing a high degree of homology to IFIH1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:45 or amino acid sequences being at least 75%, 80%, 85%, 90%, 91%, 92%, 93%, 94%, 95%, 96%, 97%, 98% or 99% identical to the sequence as set forth in SEQ ID NO:46 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:46 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:45.

The term "IFIT1" refers to the Interferon Induced Protein With Tetratricopeptide Repeats 1 gene (Ensembl: ENSG00000185745), for example, to the sequence as defined in NCBI Reference Sequence NM_001270929 or in NCBI Reference Sequence NM_001548.5, specifically, to the nucleotide sequence as set forth in SEQ ID NO:47 or in SEQ ID NO:48, which correspond to the sequences of the above indicated NCBI Reference Sequences of the IFIT1 transcript, and also relates to the corresponding amino acid sequence for example as set forth in SEQ ID NO:49 or in SEQ ID NO:50, which correspond to the protein sequences defined in NCBI Protein Accession Reference Sequence NP_001257858 and in NCBI Protein Accession Reference Sequence NP_001539 encoding the IFIT1 polypeptide.

The term "IFIT1" also comprises nucleotide sequences showing a high degree of homology to IFIT1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:47 or in SEQ ID NO:48 or amino acid sequences being at least 75%, 80%, 85%, 90%, 91%, 92%, 93%, 94%, 95%, 96%, 97%, 98% or 99% identical to the sequence as set forth in SEQ ID NO:49 or in SEQ ID NO:50 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:49 or SEQ ID NO:50 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:47 or in SEQ ID NO:48.

The term "IFIT3" refers to the Interferon Induced Protein With Tetratricopeptide Repeats 3 gene (Ensembl: ENSG00000119917), for example, to the sequence as defined in NCBI Reference Sequence NM_001031683, specifically, to the nucleotide sequence as set forth in SEQ ID NO:51, which corresponds to the sequence of the above indicated NCBI Reference Sequence of the IFIT3 transcript, and also relates to the corresponding amino acid sequence for example as set forth in SEQ ID NO:52, which corresponds to the protein sequence defined in NCBI Protein Accession Reference Sequence NP_001026853 encoding the IFIT3 polypeptide.

The term "IFIT3" also comprises nucleotide sequences showing a high degree of homology to IFIT3, e.g., nucleic acid sequences being at least 75%, 80%, 85%, 90%, 91%, 92%, 93%, 94%, 95%, 96%, 97%, 98% or 99% identical to the sequence as set forth in SEQ ID NO:51 or amino acid sequences being at least 75%, 80%, 85%, 90%, 91%, 92%, 93%, 94%, 95%, 96%, 97%, 98% or 99% identical to the sequence as set forth in SEQ ID NO:52 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:52 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:51.

The term "KIAA1549" refers to the human KIAA1549 gene (Ensembl: ENSG00000122778), for example, to the sequence as defined in NCBI Reference Sequence NM_020910 or in NCBI Reference Sequence NM_001164665, specifically, to the nucleotide sequence as set forth in SEQ ID NO:53 or in SEQ ID NO:54, which correspond to the sequences of the above indicated NCBI Reference Sequence of the KIAA1549 transcript, and also relates to the corresponding amino acid sequence for example as set forth in SEQ ID NO:55 or in SEQ ID NO:56, which correspond to the protein sequences defined in NCBI Protein Accession Reference Sequence NP_065961 and in NCBI Protein Accession Reference Sequence NP_001158137 encoding the KIAA1549 polypeptide.

The term "KIAA1549" also comprises nucleotide sequences showing a high degree of homology to KIAA1549, e.g., nucleic acid sequences being at least 75%, 80%, 85%, 90%, 91%, 92%, 93%, 94%, 95%, 96%, 97%, 98% or 99% identical to the sequence as set forth in SEQ ID NO:53 or in SEQ ID NO:54 or amino acid sequences being at least 75%, 80%, 85%, 90%, 91%, 92%, 93%, 94%, 95%, 96%, 97%, 98% or 99% identical to the sequence as set forth in SEQ ID NO:55 or in SEQ ID NO:56 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:55 or in SEQ ID NO:56 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:53 or in SEQ ID NO:54.

The term "LAT" refers to the Linker For Activation Of T-Cells gene (Ensembl: ENSG00000213658), for example, to the sequence as defined in NCBI Reference Sequence NM_001014987 or in NCBI Reference Sequence NM_014387, specifically, to the nucleotide sequence as set forth in SEQ ID NO:57 or in SEQ ID NO:58, which correspond to the sequences of the above indicated NCBI Reference Sequences of the LAT transcript, and also relates to the corresponding amino acid sequence for example as set forth in SEQ ID NO:59 or in SEQ ID NO:60, which corresponds to the protein sequences defined in NCBI Protein Accession Reference Sequence NP_001014987 and in NCBI Protein Accession Reference Sequence NP_055202 encoding the LAT polypeptide.

The term "LAT" also comprises nucleotide sequences showing a high degree of homology to LAT, e.g., nucleic acid sequences being at least 75%, 80%, 85%, 90%, 91%, 92%, 93%, 94%, 95%, 96%, 97%, 98% or 99% identical to the sequence as set forth in SEQ ID NO:57 or in SEQ ID NO:58 or amino acid sequences being at least 75%, 80%, 85%, 90%, 91%, 92%, 93%, 94%, 95%, 96%, 97%, 98% or 99% identical to the sequence as set forth in SEQ ID NO:59 or in SEQ ID NO:60 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:59 or in SEQ ID NO:60 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:57 or in SEQ ID NO:58.

The term "LCK" refers to the LCK Proto-Oncogene gene (Ensembl: ENSG00000182866), for example, to the sequence as defined in NCBI Reference Sequence NM_005356, specifically, to the nucleotide sequence as set forth in SEQ ID NO:61, which corresponds to the sequence of the above indicated NCBI Reference Sequence of the LCK transcript, and also relates to the corresponding amino acid sequence for example as set forth in SEQ ID NO:62, which corresponds to the protein sequence defined in NCBI Protein Accession Reference Sequence NP_005347 encoding the LCK polypeptide.

The term "LCK" also comprises nucleotide sequences showing a high degree of homology to LCK, e.g., nucleic acid sequences being at least 75%, 80%, 85%, 90%, 91%, 92%, 93%, 94%, 95%, 96%, 97%, 98% or 99% identical to the sequence as set forth in SEQ ID NO:61 or amino acid sequences being at least 75%, 80%, 85%, 90%, 91%, 92%, 93%, 94%, 95%, 96%, 97%, 98% or 99% identical to the sequence as set forth in SEQ ID NO:62 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:62 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:61.

The term "LRRFIP1" refers to the LRR Binding FLII Interacting Protein 1 gene (Ensembl: ENSG00000124831), for example, to the sequence as defined in NCBI Reference Sequence NM_004735 or in NCBI Reference Sequence NM_001137550 or in NCBI Reference Sequence NM_001137553 or in NCBI Reference Sequence NM_001137552, specifically, to the nucleotide sequence as set forth in SEQ ID NO:63 or in SEQ ID NO:64 or in SEQ ID NO:65 or in SEQ ID NO:66, which correspond to the sequences of the above indicated NCBI Reference Sequences of the LRRFIP1 transcript, and also relates to the corresponding amino acid sequence for example as set forth in SEQ ID NO:67 or in SEQ ID NO:68 or in SEQ ID NO:69 or in SEQ ID NO:70, which correspond to the protein sequences defined in NCBI Protein Accession Reference Sequence NP_004726 and in NCBI Protein Accession Reference Sequence NP_001131022 and in NCBI Protein Accession Reference Sequence NP_001131025 and in NCBI Protein Accession Reference Sequence NP_001131024 encoding the LRRFIP1 polypeptide.

The term "LRRFIP1" also comprises nucleotide sequences showing a high degree of homology to LRRFIP1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:63 or in SEQ ID NO:64 or in SEQ ID NO:65 or in SEQ ID NO:66 or amino acid sequences being at least 75%, 80%, 85%, 90%, 91%, 92%, 93%, 94%, 95%, 96%, 97%, 98% or 99% identical to the sequence as set forth in SEQ ID NO:67 or in SEQ ID NO:68 or in SEQ ID NO:69 or in SEQ ID NO: 70 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:67 or in SEQ ID NO:68 or in SEQ ID NO:69 or in SEQ ID NO:70 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:63 or in SEQ ID NO:64 or in SEQ ID NO:65 or in SEQ ID NO:66.

The term "MYD88" refers to the MYD88 Innate Immune Signal Transduction Adaptor gene (Ensembl: ENSG00000172936), for example, to the sequence as defined in NCBI Reference Sequence NM_001172567 or in NCBI Reference Sequence NM_001172568 or in NCBI Reference Sequence NM_001172569 or in NCBI Reference Sequence NM_001172566 or in NCBI Reference Sequence NM_002468, specifically, to the nucleotide sequences as set forth in SEQ ID NO:71 or in SEQ ID NO:72 or in SEQ ID NO:73 or in SEQ ID NO:74 or in SEQ ID NO:75, which correspond to the sequences of the above indicated NCBI Reference Sequences of the MYD88 transcript, and also relates to the corresponding amino acid sequence for example as set forth in SEQ ID NO:76 or in SEQ ID NO:77 or in SEQ ID NO:78 or in SEQ ID NO:79 or in SEQ ID NO:80, which correspond to the protein sequences defined in NCBI Protein Accession Reference Sequence NP_001166038 and in NCBI Protein Accession Reference Sequence NP_001166039 and in NCBI Protein Accession Reference Sequence NP_001166040 and in NCBI Protein Accession Reference Sequence NP_001166037 and in NCBI Protein Accession Reference Sequence NP_002459 encoding the MYD88 polypeptide.

The term "MYD88" also comprises nucleotide sequences showing a high degree of homology to MYD88, e.g., nucleic acid sequences being at least 75%, 80%, 85%, 90%, 91%, 92%, 93%, 94%, 95%, 96%, 97%, 98% or 99% identical to the sequence as set forth in SEQ ID NO:71 or in SEQ ID NO:72 or in SEQ ID NO:73 or in SEQ ID NO:74 or in SEQ ID NO:75 or amino acid sequences being at least 75%, 80%, 85%, 90%, 91%, 92%, 93%, 94%, 95%, 96%, 97%, 98% or 99% identical to the sequence as set forth in SEQ ID NO:76 or in SEQ ID NO:77 or in SEQ ID NO:78 or in SEQ ID NO:79 or in SEQ ID NO:80 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:76 or in SEQ ID NO:77 or in SEQ ID NO:78 or in SEQ ID NO:79 or in SEQ ID NO:80 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:71 or in SEQ ID NO:72 or in SEQ ID NO:73 or in SEQ ID NO:74 or in SEQ ID NO:75.

The term "OAS 1" refers to the 2'-5'-Oligoadenylate Synthetase 1 gene (Ensembl: ENSG00000089127), for example, to the sequence as defined in NCBI Reference Sequence NM_001320151 or in NCBI Reference Sequence NM_002534 or in NCBI Reference Sequence NM_001032409 or in NCBI Reference Sequence NM_016816, specifically, to the nucleotide sequences as set forth in SEQ ID NO:81 or in SEQ ID NO:82 or in SEQ ID NO:83 or in SEQ ID NO:84, which correspond to the sequences of the above indicated NCBI Reference Sequences of the OAS1 transcript, and also relates to the corresponding amino acid sequences for example as set forth in SEQ ID NO:85 or in SEQ ID NO:86 or in SEQ ID NO:87 or in SEQ ID NO:88, which correspond to the protein sequences defined in NCBI Protein Accession Reference Sequence NP_001307080 and in NCBI Protein Accession Reference Sequence NP_002525 and in NCBI Protein Accession Reference Sequence NP_001027581 and in NCBI Protein Accession Reference Sequence NP_058132 encoding the OAS1 polypeptide.

The term "OAS1" also comprises nucleotide sequences showing a high degree of homology to OAS1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:81 or in SEQ ID NO:82 or in SEQ ID NO:83 or in SEQ ID NO:84 or amino acid sequences being at least 75%, 80%, 85%, 90%, 91%, 92%, 93%, 94%, 95%, 96%, 97%, 98% or 99% identical to the sequence as set forth in SEQ ID NO:85 or in SEQ ID NO:86 or in SEQ ID NO:87 or in SEQ ID NO:88 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:85 or in SEQ ID NO:86 or in SEQ ID NO:87 or in SEQ ID NO:88 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:81 or in SEQ ID NO:82 or in SEQ ID NO:83 or in SEQ ID NO:84.

The term "PAG1" refers to the Phosphoprotein Membrane Anchor With Glycosphingolipid Microdomains 1 gene (Ensembl: ENSG00000076641), for example, to the sequence as defined in NCBI Reference Sequence NM_018440, specifically, to the nucleotide sequence as set forth in SEQ ID NO:89, which corresponds to the sequence of the above indicated NCBI Reference Sequence of the PAG1 transcript, and also relates to the corresponding amino acid sequence for example as set forth in SEQ ID NO:90, which corresponds to the protein sequence defined in NCBI Protein Accession Reference Sequence NP_060910 encoding the PAG1 polypeptide.

The term "PAG1" also comprises nucleotide sequences showing a high degree of homology to PAG1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:89 or amino acid sequences being at least 75%, 80%, 85%, 90%, 91%, 92%, 93%, 94%, 95%, 96%, 97%, 98% or 99% identical to the sequence as set forth in SEQ ID NO:90 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:90 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:89.

The term "PDE4D" refers to the human Phosphodiesterase 4D gene (Ensembl: ENSG00000113448), for example, to the sequence as defined in NCBI Reference Sequence NM_001104631 or in NCBI Reference Sequence NM_001349242 or in NCBI Reference Sequence NM_001197218 or in NCBI Reference Sequence NM_006203 or in NCBI Reference Sequence NM_001197221 or in NCBI Reference Sequence NM_001197220 or in NCBI Reference Sequence NM_001197223 or in NCBI Reference Sequence NM_001165899 or in NCBI Reference Sequence NM_001165899, specifically, to the nucleotide sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99, which correspond to the sequences of the above indicated NCBI Reference Sequence of the PDE4D transcript, and also relates to the corresponding amino acid sequence for example as set forth in SEQ ID NO:100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO:107 or in SEQ ID NO:108, which correspond to the protein sequences defined in NCBI Protein Accession Reference Sequence NP_001098101 and in NCBI Protein Accession Reference Sequence NP_001336171 and in NCBI Protein Accession Reference Sequence NP_001184147 and in NCBI Protein Accession Reference Sequence NP_006194 and in NCBI Protein Accession Reference Sequence NP_001184150 and in NCBI Protein Accession Reference Sequence NP_001184149 and in NCBI Protein Accession Reference Sequence NP_001184152 and in NCBI Protein Accession Reference Sequence NP_001159371 and in NCBI Protein Accession Reference Sequence NP_001184148 encoding the PDE4D polypeptide.

The term "PDE4D" also comprises nucleotide sequences showing a high degree of homology to PDE4D, e.g., nucleic acid sequences being at least 75%, 80%, 85%, 90%, 91%, 92%, 93%, 94%, 95%, 96%, 97%, 98% or 99% identical to the sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99 or amino acid sequences being at least 75%, 80%, 85%, 90%, 91%, 92%, 93%, 94%, 95%, 96%, 97%, 98% or 99% identical to the sequence as set forth in SEQ ID NO:100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO:107 or in SEQ ID NO:108 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO:107 or in SEQ ID NO:108 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99.

The term "PRKACA" refers to the Protein Kinase cAMP-Activated Catalytic Subunit Alpha gene (Ensembl: ENSG00000072062), for example, to the sequence as defined in NCBI Reference Sequence NM_002730 or in NCBI Reference Sequence NM_207518, specifically, to the nucleotide sequences as set forth in SEQ ID NO:109 or in SEQ ID NO:110, which correspond to the sequences of the above indicated NCBI Reference Sequences of the PRKACA transcript, and also relates to the corresponding amino acid sequence for example as set forth in SEQ ID NO:111 or in SEQ ID NO:112, which correspond to the protein sequences defined in NCBI Protein Accession Reference Sequence NP_002721 and in NCBI Protein Accession Reference Sequence NP_997401 encoding the PRKACA polypeptide.

The term "PRKACA" also comprises nucleotide sequences showing a high degree of homology to PRKACA, e.g., nucleic acid sequences being at least 75%, 80%, 85%, 90%, 91%, 92%, 93%, 94%, 95%, 96%, 97%, 98% or 99% identical to the sequence as set forth in SEQ ID NO:109 or in SEQ ID NO:110 or amino acid sequences being at least 75%, 80%, 85%, 90%, 91%, 92%, 93%, 94%, 95%, 96%, 97%, 98% or 99% identical to the sequence as set forth in SEQ ID NO:111 or in SEQ ID NO:112 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:111 or in SEQ ID NO:112 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:109 or in SEQ ID NO:110.

The term "PRKACB" refers to the Protein Kinase cAMP-Activated Catalytic Subunit Beta gene (Ensembl: ENSG00000142875), for example, to the sequence as defined in NCBI Reference Sequence NM_002731 or in NCBI Reference Sequence NM_182948 or in NCBI Reference Sequence NM_001242860 or in NCBI Reference Sequence NM_001242859 or in NCBI Reference Sequence NM_001242858 or in NCBI Reference Sequence NM_001242862 or in NCBI Reference Sequence NM_001242861 or in NCBI Reference Sequence NM_001300915 or in NCBI Reference Sequence NM_207578 or in NCBI Reference Sequence NM_001242857 or in NCBI Reference Sequence NM_001300917, specifically, to the nucleotide sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID NO:123, which correspond to the sequences of the above indicated NCBI Reference Sequences of the PRKACB transcript, and also relates to the corresponding amino acid sequence for example as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134, which correspond to the protein sequences defined in NCBI Protein Accession Reference Sequence NP_002722 and in NCBI Protein Accession Reference Sequence NP_891993 and in NCBI Protein Accession Reference Sequence NP_001229789 and in NCBI Protein Accession Reference Sequence NP_001229788 and in NCBI Protein Accession Reference Sequence NP_001229787 and in NCBI Protein Accession Reference Sequence NP_001229791 and in NCBI Protein Accession Reference Sequence NP_001229790 and in NCBI Protein Accession Reference Sequence NP_001287844 and in NCBI Protein Accession Reference Sequence NP_997461 and in NCBI Protein Accession Reference Sequence NP_001229786 and in NCBI Protein Accession Reference Sequence NP_001287846 encoding the PRKACB polypeptide.

The term "PRKACB" also comprises nucleotide sequences showing a high degree of homology to PRKACB, e.g., nucleic acid sequences being at least 75%, 80%, 85%, 90%, 91%, 92%, 93%, 94%, 95%, 96%, 97%, 98% or 99% identical to the sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID NO:123 or amino acid sequences being at least 75%, 80%, 85%, 90%, 91%, 92%, 93%, 94%, 95%, 96%, 97%, 98% or 99% identical to the sequence as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID NO:123.

The term "PTPRC" refers to the Protein Tyrosine Phosphatase Receptor Type C gene (Ensembl: ENSG00000081237), for example, to the sequence as defined in NCBI Reference Sequence NM_002838 or in NCBI Reference Sequence NM_080921, specifically, to the nucleotide sequence as set forth in SEQ ID NO:135 or in SEQ ID NO:136, which correspond to the sequences of the above indicated NCBI Reference Sequences of the PTPRC transcript, and also relates to the corresponding amino acid sequence for example as set forth in SEQ ID NO:137 or in SEQ ID NO:138, which correspond to the protein sequences defined in NCBI Protein Accession Reference Sequence NP_002829 encoding the PTPRC polypeptide and in NCBI Protein Accession Reference Sequence NP_563578.

The term "PTPRC" also comprises nucleotide sequences showing a high degree of homology to PTPRC, e.g., nucleic acid sequences being at least 75%, 80%, 85%, 90%, 91%, 92%, 93%, 94%, 95%, 96%, 97%, 98% or 99% identical to the sequence as set forth in SEQ ID NO:135 or in SEQ ID NO:136 or amino acid sequences being at least 75%, 80%, 85%, 90%, 91%, 92%, 93%, 94%, 95%, 96%, 97%, 98% or 99% identical to the sequence as set forth in SEQ ID NO:137 or in SEQ ID NO:138 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:137 or in SEQ ID NO:138 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:135 or in SEQ ID NO:136.

The term "RAP1GAP2" refers to the human RAP1 GTPase Activating Protein 2 gene (ENSG00000132359), for example, to the sequence as defined in NCBI Reference Sequence NM_015085 or in NCBI Reference Sequence NM_001100398 or in NCBI Reference Sequence NM_001330058, specifically, to the nucleotide sequence as set forth in SEQ ID NO:139 or in SEQ ID NO:140 or in SEQ ID NO:141, which correspond to the sequences of the above indicated NCBI Reference Sequences of the RAP1GAP2 transcript, and also relates to the corresponding amino acid sequence for example as set forth in SEQ ID NO: 142 or in SEQ ID NO:143 or in SEQ ID NO:144, which correspond to the protein sequences defined in NCBI Protein Accession Reference Sequence NP_055900 and in NCBI Protein Accession Reference Sequence NP _001093868 and in NCBI Protein Accession Reference Sequence NP_001316987 encoding the RAP1GAP2 polypeptide.

The term "RAP1GAP2" also comprises nucleotide sequences showing a high degree of homology to RAP1-GAP2, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 139 or in SEQ ID NO:140 or in SEQ ID NO:141 or amino acid sequences being at least 75%, 80%, 85%, 90%, 91%, 92%, 93%, 94%, 95%, 96%, 97%, 98% or 99% identical to the sequence as set forth in SEQ ID NO: 142 or in SEQ ID NO:143 or in SEQ ID NO:144 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 142 or in SEQ ID NO: 143 or in SEQ ID NO: 144 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 139 or in SEQ ID NO:140 or in SEQ ID NO:141.

The term "SLC39A11" refers to the human Solute Carrier Family 39 Member 11 gene (Ensembl: ENSG00000133195), for example, to the sequence as defined in NCBI Reference Sequence NM_139177 or in NCBI Reference Sequence NM_001352692, specifically, to the nucleotide sequence as set forth in SEQ ID NO:145 or in SEQ ID NO:146, which correspond to the sequences of the above indicated NCBI Reference Sequences of the SLC39A11 transcript, and also relates to the corresponding amino acid sequence for example as set forth in SEQ ID NO:147 or in SEQ ID NO:148, which correspond to the protein sequences defined in NCBI Protein Accession Reference Sequence NP_631916 and in NCBI Protein Accession Reference Sequence NP_001339621 encoding the SLC39A11 polypeptide.

The term "SLC39A11" also comprises nucleotide sequences showing a high degree of homology to SLC39A11, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 145 or in SEQ ID NO:146 or amino acid sequences being at least 75%, 80%, 85%, 90%, 91%, 92%, 93%, 94%, 95%, 96%, 97%, 98% or 99% identical to the sequence as set forth in SEQ ID NO:147 or in SEQ ID NO: 148 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:147 or in SEQ ID NO:148 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:145 or in SEQ ID NO: 146.

The term "TDRD1" refers to the human Tudor Domain Containing 1 gene (Ensembl: ENSG00000095627), for example, to the sequence as defined in NCBI Reference Sequence NM_198795, specifically, to the nucleotide sequence as set forth in SEQ ID NO: 149, which corresponds to the sequence of the above indicated NCBI Reference Sequence of the TDRD1 transcript, and also relates to the corresponding amino acid sequence for example as set forth in SEQ ID NO: 150, which corresponds to the protein sequence defined in NCBI Protein Accession Reference Sequence NP_942090 encoding the TDRD1 polypeptide.

The term "TDRD1" also comprises nucleotide sequences showing a high degree of homology to TDRD1, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 149 or amino acid sequences being at least 75%, 80%, 85%, 90%, 91%, 92%, 93%, 94%, 95%, 96%, 97%, 98% or 99% identical to the sequence as set forth in SEQ ID NO: 150 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 150 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 149.

The term "TLR8" refers to the Toll Like Receptor 8 gene (Ensembl: ENSG00000101916), for example, to the sequence as defined in NCBI Reference Sequence NM_138636 or in NCBI Reference Sequence NM_016610, specifically, to the nucleotide sequences as set forth in SEQ ID NO:151 or in SEQ ID NO:152, which correspond to the sequences of the above indicated NCBI Reference Sequences of the TLR8 transcript, and also relates to the corresponding amino acid sequences for example as set forth in SEQ ID NO: 153 or in SEQ ID NO:154, which corresponds to the protein sequences defined in NCBI Protein Accession Reference Sequence NP_619542 and in NCBI Protein Accession Reference Sequence NP_057694 encoding the TLR8 polypeptide.

The term "TLR8" also comprises nucleotide sequences showing a high degree of homology to TLR8, e.g., nucleic acid sequences being at least 75%, 80%, 85%, 90%, 91%, 92%, 93%, 94%, 95%, 96%, 97%, 98% or 99% identical to the sequence as set forth in SEQ ID NO:151 or in SEQ ID NO:152 or amino acid sequences being at least 75%, 80%, 85%, 90%, 91%, 92%, 93%, 94%, 95%, 96%, 97%, 98% or 99% identical to the sequence as set forth in SEQ ID NO:153 or in SEQ ID NO:154 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 153 or in SEQ ID NO: 154 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 151 or in SEQ ID NO: 152.

The term "VWA2" refers to the human Von Willebrand Factor A Domain Containing 2 gene (Ensembl: ENSG00000165816), for example, to the sequence as defined in NCBI Reference Sequence NM_001320804, specifically, to the nucleotide sequence as set forth in SEQ ID NO:155, which corresponds to the sequence of the above indicated NCBI Reference Sequence of the VWA2 transcript, and also relates to the corresponding amino acid sequence for example as set forth in SEQ ID NO:156, which corresponds to the protein sequence defined in NCBI Protein Accession Reference Sequence NP_001307733 encoding the VWA2 polypeptide.

The term "VWA2" also comprises nucleotide sequences showing a high degree of homology to VWA2, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 155 or amino acid sequences being at least 75%, 80%, 85%, 90%, 91%, 92%, 93%, 94%, 95%, 96%, 97%, 98% or 99% identical to the sequence as set forth in SEQ ID NO:156 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 156 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:155.

The term "ZAP70" refers to the Zeta Chain Of T-Cell Receptor Associated Protein Kinase 70 gene (Ensembl: ENSG00000115085), for example, to the sequence as defined in NCBI Reference Sequence NM_001079 or in NCBI Reference Sequence NM_207519, specifically, to the nucleotide sequences as set forth in SEQ ID NO:157 or in SEQ ID NO:158, which correspond to the sequences of the above indicated NCBI Reference Sequences of the ZAP70 transcript, and also relates to the corresponding amino acid sequence for example as set forth in SEQ ID NO:159 or in SEQ ID NO:160, which corresponds to the protein sequences defined in NCBI Protein Accession Reference Sequence NP_001070 and in NCBI Protein Accession Reference Sequence NP_997402 encoding the ZAP70 polypeptide.

The term "ZAP70" also comprises nucleotide sequences showing a high degree of homology to ZAP70, e.g., nucleic acid sequences being at least 75%, 80%, 85%, 90%, 91%, 92%, 93%, 94%, 95%, 96%, 97%, 98% or 99% identical to the sequence as set forth in SEQ ID NO:157 or in SEQ ID NO:158or amino acid sequences being at least 75%, 80%, 85%, 90%, 91%, 92%, 93%, 94%, 95%, 96%, 97%, 98% or 99% identical to the sequence as set forth in SEQ ID NO:159 or in SEQ ID NO:160 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:159 or in SEQ ID NO:160 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:157 or in SEQ ID NO:158.

The term "ZBP1" refers to the Z-DNA Binding Protein 1 gene (Ensembl: ENSG00000124256), for example, to the sequence as defined in NCBI Reference Sequence NM_030776 or in NCBI Reference Sequence NM_001160418 or in NCBI Reference Sequence NM_001160419, specifically, to the nucleotide sequence as set forth in SEQ ID NO:161 or in SEQ ID NO:162 or in SEQ ID NO:163, which corresponds to the sequence of the above indicated NCBI Reference Sequence of the ZBP1 transcript, and also relates to the corresponding amino acid sequence for example as set forth in SEQ ID NO:164 or in SEQ ID NO:165 or in SEQ ID NO:166, which corresponds to the protein sequences defined in NCBI Protein Accession Reference Sequence NP_110403 and in NCBI Protein Accession Reference Sequence NP_001153890 and in NCBI Protein Accession Reference Sequence NP_001153891 encoding the ZBP1 polypeptide.

The term "ZBP1" also comprises nucleotide sequences showing a high degree of homology to ZBP1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:161 or in SEQ ID NO:162 or in SEQ ID NO:163 or amino acid sequences being at least 75%, 80%, 85%, 90%, 91%, 92%, 93%, 94%, 95%, 96%, 97%, 98% or 99% identical to the sequence as set forth in SEQ ID NO:164 or in SEQ ID NO:165 or in SEQ ID NO:166 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:164 or in SEQ ID NO:165 or in SEQ ID NO:166 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:161 or in SEQ ID NO:162 or in SEQ ID NO:163.

The term "biological sample" or "sample obtained from a subject" refers to any biological material obtained via suitable methods known to the person skilled in the art from a subject, e.g., a patient having a glioma. The biological sample used may be collected in a clinically acceptable manner, e.g., in a way that nucleic acids (in particular RNA) or proteins are preserved.

The biological sample(s) may include body tissue and/or a fluid, such as, but not limited to, blood (or blood derived such as serum, plasma, or PBMC's (peripheral blood mononuclear cells)), sweat, saliva, urine, and a needle biopsy or resection biopsy. Furthermore, the biological sample may contain a cell extract derived from or a cell population including a cancerous cell or a cell derived from tissue suspected to be cancerous, such as a glioma cell. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumour cells, extracellular vesicles, a sample containing prostate secreted exosomes, or cell lines or cancer cell line.

In one particular realization, biopsy or resections samples may be obtained and/or used. Such samples may include cells or cell lysates.

It is also conceivable that the content of a biological sample is submitted to an enrichment step. For instance, a sample may be contacted with ligands specific for the cell membrane or organelles of certain cell types, e.g., glioma cells, functionalized for example with magnetic particles. The material concentrated by the magnetic particles may subsequently be used for detection and analysis steps as described herein above or below.

Furthermore, cells, e.g., tumour cells, may be enriched via filtration processes of fluid or liquid samples, e.g., blood, urine, etc. Such filtration processes may also be combined with enrichment steps based on ligand specific interactions as described herein above.

In an embodiment, the six or more gene expression levels comprise

- one or more immune defense response genes, preferably two or more, more preferably three or more, most preferably all of the immune defense genes, and
- one or more T-Cell receptor signaling genes, preferably two or more, more preferably three or more, most preferably all of the T-Cell receptor signaling genes, and

- one or more PDE4D7 correlated genes, preferably two or more, more preferably three or more, most preferably all of the PDE4D7 correlated genes.

In an embodiment:

- the one or more immune defense response genes comprise three or more, preferably, six or more, more preferably, nine or more, most preferably, all of the immune defense genes, and/or
- the one or more T-Cell receptor signaling genes comprise three or more, preferably, six or more, more preferably, nine or more, most preferably, all of the T-Cell receptor signaling genes, and/or
- the one or more PDE4D7 correlated genes comprise three or more, preferably, six or more, most preferably, all of the PDE4D7 correlated genes.

It is preferred that the determining of the outcome comprises:

- combining the first gene expression profiles for two or more, for example, 2, 3, 4, 5, 6, 7, 8, 9, 10, 11, 12, 13 or all, of the immune defense response genes with a regression function that had been derived from a population of subjects having a glioma, and/or
- combining the second gene expression profiles for two or more, for example, 2, 3, 4, 5, 6, 7, 8, 9, 10, 11, 12, 13, 14, 15, 16 or all, of the T-Cell receptor signaling genes with a regression function that had been derived from a population of subjects having a glioma, and/or
- combining the third gene expression profiles for two or more, for example, 2, 3, 4, 5, 6, 7 or all, of the PDE4D7 correlated genes with a regression function that had been derived from a population of subjects having a glioma, and/or
- combining the six or more gene expression levels, for example 6, 7, 8, 9, 10, 11, 12, 13, 14, 15, 16, or more, or all gene expression levels with a regression function that had been derived from a population of subjects having a glioma.

Cox proportional-hazards regression allows analyzing the effect of several risk factors on time to a tested event like survival. Thereby, the risk factors maybe dichotomous or discrete variables like a risk score or a clinical stage but may also be a continuous variable like a biomarker measurement or gene expression values. The probability of the endpoint (e.g., death or disease recurrence) is called the hazard. Next to the information on whether or not the tested endpoint was reached by e.g. subject in a patient cohort (e.g., patient did die or not) also the time to the endpoint is considered in the regression analysis. The hazard is modeled as: $H(t) = H_0(t) \cdot \exp(w_1 \cdot V_1 + w_2 \cdot V_2 + w_3 \cdot V_3 + ...)$, where $V_1$, $V_2$, $V_3$ ... are predictor variables and $H_0(t)$ is the baseline hazard while $H(t)$ is the hazard at any time t. The hazard ratio (or the risk to reach the event) is represented by $\mathrm{Ln}[H(t)/ H_0(t)] = w_1 \cdot V_1 + w_2 \cdot V_2 + w_3 \cdot V_3 + ...$, where the coefficients or weights $w_1$, $w_2$, $w_3$ ... are estimated by the Cox regression analysis and can be interpreted in a similar manner as for logistic regression analysis.

In one particular realization, the combination of the first gene expression profiles for the six or more, for example 6, 7, 8, 9, 10, 11, 12, 13 or all, of the immune defense response genes with a regression function is determined as follows:
IDR model:

$$
(w_1 \cdot \mathrm{AIM2}) + (w_2 \cdot \mathrm{APOBEC3A}) + (w_3 \cdot \mathrm{CIAO1}) + (w_4 \cdot \mathrm{DDX58}) +
$$
$$
(w_5 \cdot \mathrm{DHX9}) + (w_6 \cdot \mathrm{IFI16}) + (w_7 \cdot \mathrm{IFIH1}) + (w_8 \cdot \mathrm{IFIT1}) + (w_9 \cdot \mathrm{IFIT3}) \quad (1)
$$
$$
+ (w_{10} \cdot \mathrm{LRRFIP1}) + (w_{11} \cdot \mathrm{MYD88}) + (w_{12} \cdot \mathrm{OAS1}) + (w_{13} \cdot \mathrm{TLR8}) +
$$
$$
(w_{14} \cdot \mathrm{ZBP1})
$$

where $w_1$ to $w_{14}$ are weights and AIM2, APOBEC3A, CIAO1, DDX58, DHX9, IFI16, IFIH1, IFIT1, IFIT3, LRRFIP1, MYD88, OAS1, TLR8, and ZBP1 are the expression levels of the immune defense response genes.

In one particular realization, the combination of the second gene expression profiles for the six or more, for example, 6, 7, 8, 9, 10, 11, 12, 13, 14, 15, 16 or all, of the T-Cell receptor signaling genes with a regression function is determined as follows:
TCR SIGNALING model:

$$(w_{15} \cdot C2) + (w_{16} \cdot CD247) + (w_{17} \cdot CD28) + (w_{18} \cdot CD3E) + (w_{19} \cdot$$
$$CD3G) + (w_{20} \cdot CD4) + (w_{21} \cdot CSK) + (w_{22} \cdot EZR) + (w_{23} \cdot FYN) + \qquad (2)$$
$$(w_{24} \cdot LAT) + (w_{25} \cdot LCK) + (w_{26} \cdot PAG1) + (w_{27} \cdot PDE4D) + (w_{28} \cdot$$
$$PRKACA) + (w_{29} \cdot PRKACB) + (w_{30} \cdot PTPRC) + (w_{31} \cdot ZAP70)$$

where $w_{15}$ to $w_{31}$ are weights and CD2, CD247, CD28, CD3E, CD3G, CD4, CSK, EZR, FYN, LAT, LCK, PAG1, PDE4D, PRKACA, PRKACB, PTPRC, and ZAP70 are the expression levels of the T-Cell receptor signaling genes.

In one particular realization, the combination of the third gene expression profiles for the six or more, for example, 6, 7 or all, of the PDE4D7 correlated genes with a regression function is determined as follows: PDE4D7_CORR_model:

$$(w_{32} \cdot ABCC5) + (w_{33} \cdot CUX2) + (w_{34} \cdot KIAA1549) + (w_{35} \cdot PDE4D) +$$
$$(w_{36} \cdot RAP1GAP2) + (w_{37} \cdot SLC39A11) + (w_{38} \cdot TDRD1) + (w_{39} \cdot \qquad (3)$$
$$VWA2)$$

where $w_{32}$ to $w_{39}$ are weights and ABCC5, CUX2, KIAA1549, PDE4D, RAP1GAP2, SLC39A11, TDRD1, and VWA2 are the expression levels of the PDE4D7 correlated genes.

It is further preferred that the determining of the prediction of the outcome further comprises combining the combination of the first gene expression profiles, the combination of the second gene expression profiles, and the combination of the third gene expression profiles with a regression function that had been derived from a population of glioma subjects.

In one particular realization, the prediction of the outcome is determined as follows: GLCAI_model:

$$(w_{40} \cdot IDR\_model) + (w_{41} \cdot TCR\_SIGNALING\_model) + (w_{42} \cdot \qquad (4)$$
$$PDE4D7\_CORR\_model)$$

where $w_{40}$ to $w_{42}$ are weights, IDR_model is the above-described regression model based on the expression profiles for the two or more, for example, 2, 3, 4, 6, 7, 8, 9, 10, 11, 12, 13 or all, of the immune defense response genes, TCR_SIGNALING_model is the above-described regression model based on the expression profiles for the two or more, for example, 2, 3, 4, 6, 7, 8, 9, 10, 11, 12, 13, 14, 15, 16 or all, of the T-Cell receptor signaling genes, and PDE4D7_CORR_model is the above-described regression model based on the expression profiles for the two or more, for example, 2, 3, 4, 6, 7 or all, of the PDE4D7 correlated genes.

In one particular realization, the combination of the expression levels of the six or more genes are calculated as follows: GLCAI_6 model:

$$(w_a \cdot [geneA]) + (w_b \cdot [geneB]) + (w_c \cdot [geneC]) + (w_d \cdot [geneD]) + (w_e \cdot [geneE]) + (w_f \cdot$$
$$[geneF])$$

where $w_x$ represents the weight for the respective gene geneX. It is within the ability of the skilled artisan to generate combinations of six or more genes as defined herein and calculated the respective weights for such model.

The prediction of the outcome may also be classified or categorized into one of at least two risk groups, based on the value of the prediction of the outcome. For example, there may be two risk groups, or three risk groups, or four risk groups, or more than four predefined risk groups. Each risk group covers a respective range of (non-overlapping) values of the prediction of the outcome. For example, a risk group may indicate a probability of occurrence of a specific clinical event from 0 to <0.1 or from 0.1 to <0.25 or from 0.25 to <0.5 or from 0.5 to 1.0 or the like.

It is further preferred that the determining of the prediction of the outcome is further based on one or more clinical parameters obtained from the subject.

As mentioned above, various measures based on clinical parameters have been investigated. By further basing the prediction of the outcome on such clinical parameter(s), it can be possible to further improve the prediction.

It is preferred that the clinical parameters comprise one or more of: (i) Karnofsky Performance Score; (ii) Tumor Grade; (iii) presence and amount of lesions. Additionally or alternatively, the clinical parameters comprise one or more other clinical parameters that is/are relevant for the diagnosis and/or prognosis of glioma.

The Karnofsky Performance Score (KPS) ranking runs from 100 to 0, where 100 is "perfect" health and 0 is death. Scores are typically assigned in between standard intervals of 10. The primary purpose of its development was to allow physicians to evaluate a patient's ability to survive chemotherapy for cancer.

100 - Normal; no complaints; no evidence of disease.
90 - Able to carry on normal activity; minor signs or symptoms of disease.
80 - Normal activity with effort; some signs or symptoms of disease.
70 - Cares for self; unable to carry on normal activity or to do active work.
60 - Requires occasional assistance, but is able to care for most of their personal needs.
50 - Requires considerable assistance and frequent medical care.
40 - Disabled; requires special care and assistance.
30 - Severely disabled; hospital admission is indicated although death not imminent.
20 - Very sick; hospital admission necessary; active supportive treatment necessary.
10 - Moribund; fatal processes progressing rapidly.
0 - Dead

For example, the Karnofsky Performance score may group patients as follows: 0-20; 20-40; 40-60; 60-80; and 80-100.

It is further preferred that the determining of the prediction of the outcome comprises combining one or more of: (i) the first gene expression profile(s) for the one or more immune defense response genes; (ii) the second gene expression profile(s) for the one or more T-Cell receptor signaling genes; (iii) the third gene expression profile(s) for the one or more PDE4D7 correlated genes, and; (iv) the combination of the first gene expression profiles, the combination of the second gene expression profiles, and the combination of the third gene expression profiles, and the one or more clinical parameters obtained from the subject with a regression function that had been derived from a population of subjects having a glioma.

It is preferred that the biological sample is obtained from the subject before the start of the therapy. The gene expression profile(s) may be determined in the form of mRNA or protein in tissue of glioma. Alternatively, if the genes are present in a soluble form, the gene expression profile(s) may be determined in blood, for example a blood sample comprising a circulating tumor cell.

It is further preferred that the therapy is surgery, radiotherapy, cytotoxic chemotherapy (CTX), short- or long-course chemo-radiation therapy (CRT), immunotherapy, targeted therapy or any combination thereof.

It is preferred that the prediction of the therapy response is unlikely or likely for the effectiveness of the therapy, wherein a therapy is recommended based on the prediction and, if the prediction is negative, the recommended therapy comprises one or more of: (i) therapy provided earlier than is the standard; (ii) radiotherapy with an increased effective dose; (iii) an adjuvant therapy, such as chemotherapy; (iv) long-course CRT (chemo-radiation therapy), and; (iv) an alternative therapy, such as immunotherapy.

In a further aspect of the present invention, an apparatus for predicting an outcome of a subject having a glioma, comprising:

- an input adapted to receive data indicative of six or more gene expression levels, for example 6, 7, 8, 9, 10, 11, 12, 13, 14, 16, 17, 18, 19, 20, 21, 22, 23, 24, 25, 26, 27, 28, 29, 30, 31, 32, 33, 34, 35, 36, 37, 38 or even 39, gene expression levels selected from a first gene expression profile, a second gene expression profile and/or a third gene expression profile, wherein
- the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1;
- the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70;
- the third gene expression profile consists of the PDE4D7 correlated genes selected from the group consisting of: ABCC5, CUX2, KIAA1549, PDE4D, RAP1GAP2, SLC39A11, TDRD1, and VWA2;
said gene expression levels being determined in a biological sample obtained from the subject; and
- a processor adapted to determine the prediction of outcome based on the six or more gene expression levels. In an optional embodiment the apparatus further comprises a providing unit adapted to provide the prediction to a medical caregiver or the subject.

In a further aspect of the present invention, a computer program product comprising instructions which, when the program is executed by a computer, cause the computer to carry out a method for predicting an outcome of a subject having a glioma comprising:

- receiving data indicative of six or more, for example 6, 7, 8, 9, 10, 11, 12, 13, 14, 16, 17, 18, 19, 20, 21, 22, 23, 24, 25, 26, 27, 28, 29, 30, 31, 32, 33, 34, 35, 36, 37, 38 or even 39, gene expression levels selected from a first gene expression profile, a second gene expression profile and/or a third gene expression profile, wherein
- the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1;
- the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70;
- the third gene expression profile consists of the PDE4D7 correlated genes selected from the group consisting of: ABCC5, CUX2, KIAA1549, PDE4D, RAP1GAP2, SLC39A11, TDRD1, and VWA2;
  said gene expression levels being determined in a biological sample obtained from the subject; and
- determining a prediction of an outcome of the subject based on the six or more gene expression levels. In an optional embodiment the computer program further comprises instructions for providing the prediction to a medical caregiver or the subject.

In a further aspect of the present invention, a diagnostic kit comprising:

- means for determining in a biological sample obtained from a subject, six or more gene expression levels selected from a first gene expression profile, a second gene expression profile and a third gene expression profile, wherein said six or more gene expression levels comprise at least one or more gene expression level selected from the third gene expression profile and at least one or more gene expression level selected from the first and/or the second gene expression profile, wherein
- the first gene expression profile consists of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1;
- the second gene expression profile consists of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70; and
- the third gene expression profile consisting of the PDE4D7 correlated genes selected from the group consisting of: ABCC5, CUX2, KIAA1549, PDE4D, RAP1GAP2, SLC39A11, TDRD1, and VWA2. Optionally the kit further comprises an apparatus as defined herein, and/or a computer program product as defined herein.

Alternatively a diagnostic kit is disclosed comprising:

- means for determining a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in a biological sample obtained from a subject, and/or
- means for determining a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, in a biological sample obtained from a subject, and/or
- means for determining a gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, in a biological sample obtained from a subject, and
- optionally, an apparatus as defined herein and/or a computer program product as defined herein.

The means for determining the expression levels of the target genes may be primers and/or probes suitable for any one of PCR, quantitative PCR, digital PCR RNA sequencing, or targeted RNA sequencing. Thus for example the means may be PCR primers, quantitative PCR primers and probes, digital PCR primers and probes, or capture probes for targeted mRNA sequencing. Preferably the means are PCR amplification primers and optionally probes or mRNA targeted sequencing capture probes.

In a further aspect the present invention relates to the use of a kit, the use comprising:

- determining six or more gene expression levels in a sample obtained from a subject having a glioma; and
- providing an outcome for the subject based on the six or more gene expression levels;

wherein the kit comprises means for determining six or more gene expression levels selected from a first gene expression profile, a second gene expression profile and/or a third gene expression profile, wherein

- the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1;
- the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70;
- the third gene expression profile consists of the PDE4D7 correlated genes selected from the group consisting of: ABCC5, CUX2, KIAA1549, PDE4D, RAP1GAP2, SLC39A11, TDRD1, and VWA2. In an embodiment the use comprises performing the method of predicting an outcome of a subject having a glioma as broadly described herein.

In a further aspect of the present invention, a use of six or more gene expression levels selected from a first gene expression profile, a second gene expression profile and a third gene expression profile, wherein said six or more gene expression levels comprise at least one or more gene expression level selected from the third gene expression profile and at least one or more gene expression level selected from the first and/or the second gene expression profile, wherein the first gene expression profile consists of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, the second gene expression profile consists of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70the third gene expression profile consists of thePDE4D7 correlated genes selected from the group consisting of: ABCC5, CUX2, KIAA1549, PDE4D, RAP1GAP2, SLC39A11, TDRD1, and VWA2, in a method of predicting an outcome of a subject having a glioma, comprising:

- determining the prediction of the outcome based on the six or more gene expression levels, and
- optionally, providing the prediction to a medical caregiver or the subject.

Alternatively the method relates to the use of a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or of a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or of a third gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, in a method of predicting an outcome of a subject having a glioma is presented, comprising:

- determining the prediction of the outcome based on the first gene expression profile(s), or on the second gene expression profile(s), or on the third gene expression profile(s), or on the first, second, and third gene expression profile(s), and
- optionally, providing the prediction or the personalization or a therapy recommendation based on the prediction or the personalization to a medical caregiver or the subject.

In a further aspect the invention relates to a product for use in the treatment or amelioration of a subject having a glioma, the product comprising a chemotherapeutic compound, a targeted cancer therapeutic compound or an immunotherapeutic compound;
wherein the use comprises determining or receiving the result of a determination of six or more gene expression levels selected from a first gene expression profile, a second gene expression profile and/or a third gene expression profile, wherein

- the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1;
- the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70;
- the third gene expression profile consists of the PDE4D7 correlated genes selected from the group consisting of: ABCC5, CUX2, KIAA1549, PDE4D, RAP1GAP2, SLC39A11, TDRD1, and VWA2;
  said gene expression levels being determined in a biological sample obtained from the subject; and
- determining the prediction of the outcome based on six or more gene expression levels; and administering to the subject the product.

The product thus may comprise comprising a chemotherapeutic compound, a targeted cancer therapeutic compound or an immunotherapeutic compound or a combination thereof. Non limiting examples of chemotherapeutic compounds are Abraxane, Actinomycin, Alitretinoin, All-trans retinoic acid, Altretamine, Azacitidine, Azathioprine, Belotecan, Bendamustine, Bexarotene, Bleomycin, Bortezomib, Busulfan, Cabazitaxel, Camptothecin, Carboplatin, Carboquone, Carmustine, Capecitabine, Cisplatin, Chlorambucil, Chlormethine, Chlorozotocin, Cyclophosphamide, Cytarabine, Dacarbazine, Daunorubicin, Docetaxel, Doxifluridine, Doxorubicin, Epirubicin, Epothilone, Erlotinib, Etoposide, Exatecan, Fluorouracil, Fotemustine, Gefitinib, Gemcitabine, Gimatecan, Hydroxyurea, Idarubicin, Ifosfamide, Imatinib, Irinotecan, Ixabepilone, Larotaxel, Lomustine, Melphalan, Melphalan, flufenamide, Mercaptopurine, Methotrexate, Mitobronitol, Mitomycin C, Mitoxantrone, Nimustine, Nitrosoureas, Oxaliplatin, Paclitaxel, Pemetrexed, Pipobroman, Ranimustine, Romidepsin, Semustine, Streptozotocin, Tafluposide, Taxotere, Temozolomide, Tesetaxel, Teniposide, Thiotepa, Tioguanine, Topotecan, Treosulfan, Tretinoin, Triaziquone, Triethylenemelamine, Valrubicin, Vemurafenib, Vinblastine, Vincristine, Vindesine, Vinorelbine, Vismodegib, and Vorinostat. Therefore in an embodiment the product comprises a chemotherapeutic compound as listed above.

Non limiting examples of immunotherapeutic compounds are Ipilimumab (Yervoy), tremelimumab, Nivolumab (Opdivo), Pembrolizumab (Keytruda), Atezolizumab (Tecentriq), Avelumab (Bavencio), Durvalumab (Imfinzi), Cemiplimab (Libtayo), Dostarlimab (Jemperli), JTX-4014, Spartalizumab (PDR001), Camrelizumab (SHR1210), Sintilimab (IBI308), Tislelizumab (BGB-A317), Toripalimab (JS 001), Relatlimab (BMS-986016), INCMGA00012 (MGA012), AMP-224, AMP-514 (MEDI0680), KN035, CK-301, ALTNP12, CA-170, BCD-100, and BMS-986189, even more preferably an antibody treatment selected from Ipilimumab (Yervoy), tremelimumab, Nivolumab (Opdivo), Pembrolizumab (Keytruda), Atezolizumab (Tecentriq), Avelumab (Bavencio), Durvalumab (Imfinzi), Cemiplimab (Libtayo), Dostarlimab (Jemperli), JTX-4014, Spartalizumab (PDR001), Camrelizumab (SHR1210), Sintilimab (IBI308), Tislelizumab (BGB-A317), Toripalimab (JS 001), INCMGA00012 (MGA012), AMP-224, AMP-514 (MEDI0680), KN035, CK-301, ALTNP12, CA-170, LY3300054, INCAGN02385, Sym023, Cobolimab (TSR-022), RO7121661, AZD7789, LY3321367, LB1410, INCAGN02390 and BMS-986189. Therefore in an embodiment the product comprises a immunotherapeutic compound as listed above.

Non-limiting examples of target therapeutic compounds are belzutifan (Welireg), bevacizumab (Avastin), dabrafenib (Tafinlar), everolimus (Afinitor), and trametinib (Mekinist). Therefore in an embodiment the product comprises a target therapeutic compound as listed above.

In an embodiment the product comprises a combination of two or more chemotherapeutic compounds, immunotherapeutic compounds and/or target therapeutic compound as listed above.

It is envisioned that when a favorable outcome is predicted, the following treatment options are ideally recommended: surgery; radiation therapy; chemotherapy; or targeted therapy. When an unfavorable outcome is predicted the following treatment options are ideally recommended: a combination of two or more selected from surgery, radiation therapy, chemotherapy, and targeted therapy; or immunotherapy; or an experimental drug or treatment (clinical trial). Thus, in an embodiment the product comprises a chemotherapeutic compound or a targeted therapeutic compound when a favorable outcome is predicted for the subject. In an embodiment the product comprises an immunotherapeutic compound or a combination of a chemotherapeutic or a comprises a chemotherapeutic compound or a targeted therapeutic compound when an unfavorable outcome is predicted for the subject.

In an alternative aspect the invention relates to a method of treating a subject having a glioma, the method comprising: determining or receiving the result of a determination of six or more gene expression levels selected from a first gene expression profile, a second gene expression profile and/or a third gene expression profile, wherein

- the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1;
- the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70;
- the third gene expression profile consists of the PDE4D7 correlated genes selected from the group consisting of: ABCC5, CUX2, KIAA1549, PDE4D, RAP1GAP2, SLC39A11, TDRD1, and VWA2; said gene expression levels being determined in a biological sample obtained from the subject; and
- determining the prediction of the outcome based on six or more gene expression levels; and administering to the subject a treatment based on the determined outcome.

In an embodiment favorable outcome is predicted and a treatment is administered or performed selected from surgery, radiation therapy, chemotherapy, or targeted therapy. In an embodiment an unfavorable outcome is predicted and a treatment is administered or performed selected from a combination of two or more selected from surgery, radiation therapy, chemotherapy, and targeted therapy, or immunotherapy, or an experimental drug or treatment (clinical trial).

It shall be understood that the method of predicting an outcome of a subject having a glioma as broadly described herein, the apparatus as broadly described herein, the computer program product as broadly described herein, the diagnostic kit as broadly described herein, the use of the diagnostic kit as broadly described herein, the use of first, second, and/or third gene expression profile(s) as broadly described herein and the product for use as broadly described herein have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

Fig. 1 shows schematically and exemplarily a flowchart of an embodiment of a method of predicting an outcome of a subject having a glioma.

A biological sample is obtained from each of a first set of patients (subjects) diagnosed with a glioma. Preferably, monitoring glioma has been performed for these patients over a period of time, such as at least one year, or at least two years, or about five years, after obtaining the biological sample.

In a next step, a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or a third gene expression profile for each of two or more, for example, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, is obtained for each of the biological samples obtained from the first set of patients, e.g., by performing RT-qPCR (real-time quantitative PCR) on RNA extracted from each biological sample. The exemplary gene expression profiles include an expression level (e.g., value) for each of the two or more genes which can be normalized using value(s) for each of a set of reference genes, such as B2M, HPRT1, POLR2A, and/or PUM1. In one realization, the gene expression level for each of the two or more genes of the first gene expression profiles, and/or the second gene expression profiles, and/or the third gene expression profiles is normalized with respect to one or more reference genes selected from the group consisting of ACTB, ALAS1, B2M, HPRT1, POLR2A, PUM1, RPLP0, TBP, TUBA1B, and/or YWHAZ, e.g., at least one, or at least two, or at least three, or, preferably, all of these reference genes.

In a next step, a regression function for assigning a prediction of the outcome is determined based on the first gene expression profiles for the two or more immune defense response genes, AIM2, APOBEC3A, CIAO1, DDX58, DHX9, IFI16, IFIH1, IFIT1, IFIT3, LRRFIP1, MYD88, OAS1, TLR8, and/or ZBP1, and/or the second gene expression profiles for the two or more T-Cell receptor signaling genes, CD2, CD247, CD28, CD3E, CD3G, CD4, CSK, EZR, FYN, LAT, LCK, PAG1, PDE4D, PRKACA, PRKACB, PTPRC, and/or ZAP70, and/or the third gene expression profiles for the two or more PDE4D7 correlated genes, ABCC5, CUX2, KIAA1549, PDE4D, RAP1GAP2, SLC39A11, TDRD1, and/or VWA2, obtained for at least some of the biological samples obtained for the first set of patients and respective results obtained from the monitoring. In one particular realization, the regression function is determined as specified above.

In a next step, a biological sample is obtained from a patient (subject or individual). The patient can be a new patient or one of the first set.

In a next step, a first gene expression profile for each of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or a second gene expression profile for each of one or more, for example, 1, 2, 3, 4, 5, 6, 7, 8, 9, 10, 11, 12, 13, 14, 15, 16 or all, T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or a third gene expression profile is obtained for each of the two or more, for example, 2, 3, 4, 5, 6, 7 or all, PDE4D7 correlated genes, e.g., by performing PCR on the biological sample. In one realization, the gene expression level for each of the two or more genes of the first gene expression profiles, and/or the second gene expression profiles, and/or the third gene expression profiles is normalized with respect to one or more reference genes selected from the group consisting of ACTB, ALAS1, B2M, HPRT1, POLR2A, PUM1, RPLP0, TBP, TUBA1B, and/or YWHAZ, e.g., at least one, or at least two, or at least three, or, preferably, all of these reference genes.

In a next step, a prediction of the outcome based on the first, second, and third gene expression profiles is determined for the patient using the regression function. This will be described in more detail later in the description.

In a next step, a therapy recommendation may be provided, e.g., to the patient or his or her guardian, to a doctor, or to another healthcare worker, based on the prediction or the personalization. To this end, the prediction or personalization may be categorized into one of a predefined set of risk groups, based on the value of the prediction or personalization. In one particular realization, the therapy may be radiotherapy and the prediction of the therapy response may be unlikely or likely for the effectiveness of the therapy. If the prediction is unlikely, the recommended therapy may comprise one or more of: (i) therapy provided earlier than is the standard; (ii) radiotherapy with an increased effective dose; (iii) an adjuvant therapy, such as chemotherapy; (iv) long-course CRT (chemo-radiation therapy), and; (iv) an alternative therapy, such as immunotherapy.

In one embodiment, the gene expression profiles are determined by detecting mRNA expression using two or more primers and/or probes and/or two or more sets thereof.

In one embodiment, the steps further comprise obtaining clinical parameters from the first set of patients and the patient, respectively. The clinical parameters may comprise one or more of: (i) Karnofsky Performance Score; (ii) Tumor Grade; (iii) presence and amount of lesions. Additionally or alternatively, the clinical parameters comprise one or more other clinical parameters that is/are relevant for the diagnosis and/or prognosis of glioma. The regression function for assigning the prediction of the outcome that is determined above is then further based on the one or more clinical parameters obtained from at least some of the first set of patients. In a step, the prediction of the outcome is then further based on the one or more clinical parameters, e.g., (i) Karnofsky Performance Score; (ii) Tumor Grade; (iii) presence and amount of lesions, obtained from the patient and is determined for the patient using the regression function. In one particular realization, the regression function is determined as specified above.

Based on the significant correlation with survival outcome after therapy, we expect that the identified molecules will provide predictive value with regard to the effectiveness of the treatment of gliomas. Therefore in an embodiment the method is based on for example three or more gene expression levels, such as preferably four, five, six, seven eight nine or ten expression levels selected from the immune defense response genes AIM2, APOBEC3A, CIAO1, DDX58, DHX9, IFI16, IFIH1, IFIT1, IFIT3, LRRFIP1, MYD88, OAS1, TLR8, and ZBP1, and/or T-Cell receptor signaling genes selected from CD2, CD247, CD28, CD3E, CD3G, CD4, CSK, EZR, FYN, LAT, LCK, PAG1, PDE4D, PRKACA, PRKACB, PTPRC, and ZAP70, and/or PDE4D7 correlated genes selected from ABCC5, CUX2, KIAA1549, PDE4D, RAP1GAP2, SLC39A11, TDRD1.

In a fifth aspect the invention relates to a product for use in the treatment or amelioration of a subject having a glioma, the product comprising a chemotherapeutic compound, a targeted cancer therapeutic compound or an immunotherapeutic compound, wherein the use comprises determining or receiving the result of a determination of six or more gene expression levels selected from a first gene expression profile, a second gene expression profile and/or a third gene expression profile, wherein the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1; the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70; the third gene expression profile consists of the PDE4D7 correlated genes selected from the group consisting of: ABCC5, CUX2, KIAA1549, PDE4D, RAP1GAP2, SLC39A11, TDRD1, and VWA2; said gene expression levels being determined in a biological sample obtained from the subject; and determining the prediction of the outcome based on six or more gene expression levels; and administering to the subject the product.

EXAMPLES

**Example 1**

*Datasets*

The following datasets have been used:

**TGCA Cohort;** This cohort comprises data from patients having Astrocytoma (29%), glioblastoma multiforme (GBM, 0.1%), oligoastrocytoma (19%), oligodendroglioma (29%), treated primary GBM (0.1%), untreated primary (denovo) GBM (22%), or N.A. (0.3%). The median follow-up of the patients is 20 months, max follow-up is approximately 17.5 yrs. Of the patients in the cohort 31% received radiotherapy and 62% did not receive radiotherapy, for 6.4% of the patients it is unknown if radiotherapy has been received. The cohort comprises 669 patients for which combined RNA sequencing data and clinical metadata is available, of these for 625 samples survival data is available. The cohort was divided in a training cohort of 413 patients and a test cohort of 212 patients randomly. The data obtained with this cohort is depicted in Figs. 3-12 and Example 2 below.

**GSE16011 Cohort;** This cohort comprises data from patients having astrocytic tumors (including pilocytic astrocytomas (PA; 2.8%), astrocytomas, and glioblastomas (GBM; 56%)), and oligodendroglial (OD; 18%) tumors (incl pure OD tumors and mixed oligoastrocytic (MOA) tumors)). The median follow-up of the patients is 14.5 months, max follow-up is over 20 years. Of the patients in the cohort 70% received radiotherapy and 26% did not receive radiotherapy, for 4% of the patients it is unknown if radiotherapy has been received. The cohort comprises 284 patients for which combined RNA sequencing data and clinical metadata is available, of these for 272 samples survival data is available. The cohort was divided in a training cohort of 180 patients and a test cohort of 92 patients randomly. The data obtained with this cohort is depicted in Figs. 13-22 and Example 2 below.

**GSE108476 Cohort;** This cohort comprises data from patients having Astrocytoma (29%), glioblastoma multiforme (GBM, 39%), mixed (2%), non-tumor (4%), oligodendroglioma (12%), unknown (14%). The median follow-up of the patients is 22 months, max follow-up is almost 21 yrs. The cohort comprises 493 patients for which combined RNA sequencing data and clinical metadata is available, of these for 428 samples survival data is available. The cohort was divided in a training cohort of 281 patients and a test cohort of 147 patients randomly. The data obtained with this cohort is depicted in Figs. 23-34 and Example 2 below.

*Generation and validation of gene signature based models*

For each gene, the log2 expression value as provided in the download from the TCGA database (TCGA Glioma) was obtained.

The log2 expression values for each gene were transformed into z-scores by calculating:

$$\text{log2\_gene transformed z-score} = ((\text{log2\_gene}) - (\text{mean\_samples}))/(\text{stdev\_samples}) \quad (6)$$

where log2_gene is the log2 gene expression value per gene, mean_samples is the mathematical mean of the log2_gene values across all samples, and stdev_samples is the standard deviation of the log2_gene values across all samples.

This process distributes the transformed log2_gene values around the mean 0 with a standard deviation of 1.

For the multivariate analysis of the genes of interest we used the log2_gene transformed z-score value of each gene as input.

**Cox Regression Analysis**

We then set out to test whether the combination of the 14 immune defense response genes, the combination of the 17 T-Cell receptor signaling genes, the combination of the eight PDE4D7 correlated genes, and a combination thereof will exhibit a prognostic value for glioma. With Cox regression we modelled the expression levels of the 14 immune defense response genes, of the 17 T-Cell receptor signaling genes, and of the eight PDE4D7 correlated genes, respectively, to overall survival in a TCGA cohort of 377 colorectal cancer patients.

The Cox regression functions were derived as follows:

IDR_model:

$$(w_1 \cdot \text{AIM2}) + (w_2 \cdot \text{APOBEC3A}) + (w_3 \cdot \text{CIAO1}) + (w_4 \cdot \text{DDX58}) +$$
$$(w_5 \cdot \text{DHX9}) + (w_6 \cdot \text{IFI16}) + (w_7 \cdot \text{IFIH1}) + (w_8 \cdot \text{IFIT1}) + (w_9 \cdot \text{IFIT3})$$
$$+ (w_{10} \cdot \text{LRRFIP1}) + (w_{11} \cdot \text{MYD88}) + (w_{12} \cdot \text{OAS1}) + (w_{13} \cdot \text{TLR8}) +$$
$$(w_{14} \cdot \text{ZBP1})$$

TCR_SIGNALING_model:

$$(w_{15} \cdot \text{C2}) + (w_{16} \cdot \text{CD247}) + (w_{17} \cdot \text{CD28}) + (w_{18} \cdot \text{CD3E}) + (w_{19} \cdot \text{CD3G}) + (w_{20} \cdot \text{CD4}) + (w_{21} \cdot \text{CSK}) + (w_{22} \cdot \text{EZR}) + (w_{23} \cdot \text{FYN}) + (w_{24} \cdot \text{LAT}) + (w_{25} \cdot \text{LCK}) + (w_{26} \cdot \text{PAG1}) + (w_{27} \cdot \text{PDE4D}) + (w_{28} \cdot \text{PRKACA}) + (w_{29} \cdot \text{PRKACB}) + (w_{30} \cdot \text{PTPRC}) + (w_{31} \cdot \text{ZAP70})$$

PDE4D7_CORR_model:

$$(w_{32} \cdot \text{ABCC5}) + (w_{33} \cdot \text{CUX2}) + (w_{34} \cdot \text{KIAA1549}) + (w_{35} \cdot \text{PDE4D}) + (w_{36} \cdot \text{RAP1GAP2}) + (w_{37} \cdot \text{SLC39A11}) + (w_{38} \cdot \text{TDRD1}) + (w_{39} \cdot \text{VWA2})$$

The details for the weights $w_1$ to $w_{39}$ are shown in the following TABLE 4.

TABLE 4: Variables and weights for the three individual Cox regression models as trained on the TGCA Glioma dataset, i.e., the immune defense response model (IDR_model), the T-Cell receptor signaling model (TCR SIGNALING model), and the PDE4D7 correlation model (PDE4D7_CORR_model) for glioma; NA - not available.

| Variable | | Weights | | |
| --- | --- | --- | --- | --- |
| Model | | IDR_model | TCR_ SIGNALING_ model | PDE4D7_ CORR_model |
| AIM2 | $w_1$ | 0.051 | NA | NA |
| APOBEC3A | $w_2$ | 0.042 | NA | NA |
| CIAO1 | $w_3$ | -0.13 | NA | NA |
| DDX58 | $w_4$ | -0.096 | NA | NA |
| DHX9 | $w_5$ | -0.095 | NA | NA |
| IFI16 | $w_6$ | 0.31 | NA | NA |
| IFIH1 | $w_7$ | 0.43 | NA | NA |
| IFIT1 | $w_8$ | -0.30 | NA | NA |
| IFIT3 | $w_9$ | -0.60 | NA | NA |
| LRRFIP1 | $w_{10}$ | -0.098 | NA | NA |
| MYD88 | $w_{11}$ | 0.53 | NA | NA |
| OAS1 | $w_{12}$ | 0.80 | NA | NA |
| TLR8 | $w_{13}$ | -0.041 | NA | NA |
| ZBP1 | $w_{14}$ | -0.19 | NA | NA |
| CD2 | $w_{15}$ | NA | 0.15 | NA |
| CD247 | $w_{16}$ | NA | 0.11 | NA |
| CD28 | $w_{17}$ | NA | 0.62 | NA |
| CD3E | $w_{18}$ | NA | -0.50 | NA |
| CD3G | $w_{19}$ | NA | -0.11 | NA |
| CD4 | $w_{20}$ | NA | 0.18 | NA |
| CSK | $w_{21}$ | NA | -0.30 | NA |
| EZR | $w_{22}$ | NA | -0.12 | NA |
| FYN | $w_{23}$ | NA | 0.059 | NA |
| LAT | $w_{24}$ | NA | -0.14 | NA |
| LCK | $w_{25}$ | NA | -0.31 | NA |
| PAG1 | $w_{26}$ | NA | -0.24 | NA |
| PDE4D | $w_{27}$ | NA | 0.31 | NA |
| PRKACA | $w_{28}$ | NA | 0.23 | NA |
| PRKACB | $w_{29}$ | NA | -0.27 | NA |
| PTPRC | $w_{30}$ | NA | 0.42 | NA |
| ZAP70 | $w_{31}$ | NA | 0.53 | NA |
| ABCC5 | $w_{32}$ | NA | NA | 0.011 |
| CUX2 | $w_{33}$ | NA | NA | -0.84 |
| KIAA1549 | $w_{34}$ | NA | NA | 0.32 |
| PDE4D | $w_{35}$ | NA | NA | -0.30 |

(continued)

| Variable | | Weights | | |
|---|---|---|---|---|
| Model | | IDR_model | TCR_ SIGNALING_ model | PDE4D7_ CORR_model |
| RAP1GAP2 | $w_{36}$ | NA | NA | 0.14 |
| SLC39A11 | $w_{37}$ | NA | NA | -0.27 |
| TDRD1 | $w_{38}$ | NA | NA | -0.053 |
| VWA2 | $w_{39}$ | NA | NA | -0.042 |

Based on the three individual Cox regression models (IDR_model, TCR_SIGNALING_model, PDE4D7_CORR_model) we then again used Cox regression to model the combination thereof to overall survival with (GLCAI&Clinical_model) or without (GLCAI_model) the presence of the clinical variables (N stage attributes) in the respective cohorts of glioma patients. We tested the two models in Kaplan-Meier survival analysis.

The Cox regression functions were derived as follows:
GLCAI_model:

$$(w_{40} \cdot \text{IDR\_model}) + (w_{41} \cdot \text{TCR\_SIGNALING\_model}) + (w_{42} \cdot \text{PDE4D7\_CORR\_model})$$

The details for the weights $w_{40}$ to $w_{44}$ are shown in the following TABLE 5.

TABLE 5: Variables and weights for two combination Cox regression models, i.e., glioma AI model (GLCAI_model) and the glioma & clinical model (GLCAI&Clinical_model); NA - not available.

| Variable | | Weights | |
|---|---|---|---|
| Model | | | GLCAI_model |
| IDR_model | $w_{40}$ | | 0.39 |
| TCR_SIGNALING_model | $w_{41}$ | | 0.54 |
| PDE47_CORR_model | $w_{42}$ | | 0.24 |

TABLE 6: Variables and weights for the three individual Cox regression models as trained on the GSE16011 dataset, i.e., the immune defense response model (IDR_model), the T-Cell receptor signaling model (TCR SIGNALING model), and the PDE4D7 correlation model (PDE4D7_CORR_model) for glioma; NA - not available.

| Variable | | Weights | | |
|---|---|---|---|---|
| Model | | IDR_model | TCR_ SIGNALING_ model | PDE4D7_ CORR_model |
| AIM2 | $w_1$ | -0.27 | NA | NA |
| APOBEC3A | $w_2$ | 0.00 | NA | NA |
| CIAO1 | $w_3$ | -0.11 | NA | NA |
| DDX58 | $w_4$ | -0.33 | NA | NA |
| DHX9 | $w_5$ | -0.032 | NA | NA |
| IFI16 | $w_6$ | -0.13 | NA | NA |
| IFIH1 | $w_7$ | 0.66 | NA | NA |
| IFIT1 | $w_8$ | -0.34 | NA | NA |
| IFIT3 | $w_9$ | -0.017 | NA | NA |
| LRRFIP1 | $w_{10}$ | 0.25 | NA | NA |
| MYD88 | $w_{11}$ | 0.26 | NA | NA |

(continued)

| Variable | | IDR_model | TCR_ SIGNALING_ model | PDE4D7_ CORR_model |
|---|---|---|---|---|
| **Model** | | | | |
| **OAS1** | $w_{12}$ | 0.35 | NA | NA |
| **TLR8** | $w_{13}$ | -0.25 | NA | NA |
| **ZBP1** | $w_{14}$ | -0.12 | NA | NA |
| **CD2** | $w_{15}$ | NA | -0.043 | NA |
| **CD247** | $w_{16}$ | NA | -0.12 | NA |
| **CD28** | $w_{17}$ | NA | 0.21 | NA |
| **CD3E** | $w_{18}$ | NA | -0.040 | NA |
| **CD3G** | $w_{19}$ | NA | -0.23 | NA |
| **CD4** | $w_{20}$ | NA | -0.061 | NA |
| **CSK** | $w_{21}$ | NA | 0.19 | NA |
| **EZR** | $w_{22}$ | NA | 0.13 | NA |
| **FYN** | $w_{23}$ | NA | -0.35 | NA |
| **LAT** | $w_{24}$ | NA | -0.13 | NA |
| **LCK** | $w_{25}$ | NA | 0.17 | NA |
| **PAG1** | $w_{26}$ | NA | -0.051 | NA |
| **PDE4D** | $w_{27}$ | NA | -0.056 | NA |
| **PRKACA** | $w_{28}$ | NA | 0.11 | NA |
| **PRKACB** | $w_{29}$ | NA | 0.10 | NA |
| **PTPRC** | $w_{30}$ | NA | 0.12 | NA |
| **ZAP70** | $w_{31}$ | NA | 0.25 | NA |
| **ABCC5** | $w_{32}$ | NA | NA | -0.37 |
| **CUX2** | $w_{33}$ | NA | NA | -0.29 |
| **KIAA1549** | $w_{34}$ | NA | NA | -0.11 |
| **PDE4D** | $w_{35}$ | NA | NA | -0.070 |
| **RAP1GAP2** | $w_{36}$ | NA | NA | 0.17 |
| **SLC39A11** | $w_{37}$ | NA | NA | -0.13 |
| **TDRD1** | $w_{38}$ | NA | NA | -0.0072 |
| **VWA2** | $w_{39}$ | NA | NA | 0.00 |

Based on the three individual Cox regression models (IDR_model, TCR_SIGNALING_model, PDE4D7_CORR_model) we then again used Cox regression to model the combination thereof to overall survival with (GLCAI&Clinical _model) or without (GLCAI_model) the presence of the clinical variables (N stage attributes) in the respective cohorts of glioma patients. We tested the two models in Kaplan-Meier survival analysis.

The Cox regression functions were derived as follows:

GLCAI_model:

$$(w_{40} \cdot \text{IDR\_model}) + (w_{41} \cdot \text{TCR\_SIGNALING\_model}) + (w_{42} \cdot \text{PDE4D7\_CORR\_model})$$

The details for the weights $w_{40}$ to $w_{44}$ are shown in the following TABLE 7.

TABLE 7: Variables and weights for two combination Cox regression models, i.e., glioma AI model (GLCAI_model) and the glioma & clinical model (GLCAI&Clinical_model); NA - not available.

| Variable | Weights | |
|---|---|---|
| Model | | GLCAI_model |
| IDR_model | $w_{40}$ | 0.15 |
| TCR_SIGNALING_model | $w_{41}$ | 0.85 |
| PDE47_CORR_model | $w_{42}$ | 0.54 |

TABLE 8: Variables and weights for the three individual Cox regression models as trained on the GSE108476 dataset, i.e., the immune defense response model (IDR_model), the T-Cell receptor signaling model (TCR SIGNALING model), and the PDE4D7 correlation model (PDE4D7_CORR_model) for glioma; NA - not available.

| Variable | Weights | IDR_model | TCR_ SIGNALING_ model | PDE4D7_ CORR_model |
|---|---|---|---|---|
| Model | | | | |
| AIM2 | $w_1$ | 0.048 | NA | NA |
| APOBEC3A | $w_2$ | -0.093 | NA | NA |
| CIAO1 | $w_3$ | 0.019 | NA | NA |
| DDX58 | $w_4$ | -0.15 | NA | NA |
| DHX9 | $w_5$ | -0.18 | NA | NA |
| IFI16 | $w_6$ | -0.12 | NA | NA |
| IFIH1 | $w_7$ | 0.27 | NA | NA |
| IFIT1 | $w_8$ | -0.013 | NA | NA |
| IFIT3 | $w_9$ | 0.12 | NA | NA |
| LRRFIP1 | $w_{10}$ | 0.044 | NA | NA |
| MYD88 | $w_{11}$ | -0.045 | NA | NA |
| OAS1 | $w_{12}$ | -0.079 | NA | NA |
| TLR8 | $w_{13}$ | -0.080 | NA | NA |
| ZBP1 | $w_{14}$ | -0.026 | NA | NA |
| CD2 | $w_{15}$ | NA | 0.10 | NA |
| CD247 | $w_{16}$ | NA | -0.10 | NA |
| CD28 | $w_{17}$ | NA | -0.11 | NA |
| CD3E | $w_{18}$ | NA | -0.34 | NA |
| CD3G | $w_{19}$ | NA | -0.057 | NA |
| CD4 | $w_{20}$ | NA | 0.055 | NA |
| CSK | $w_{21}$ | NA | -0.12 | NA |
| EZR | $w_{22}$ | NA | -0.15 | NA |
| FYN | $w_{23}$ | NA | -0.018 | NA |
| LAT | $w_{24}$ | NA | 0.062 | NA |
| LCK | $w_{25}$ | NA | 0.020 | NA |
| PAG1 | $w_{26}$ | NA | -0.024 | NA |
| PDE4D | $w_{27}$ | NA | -0.064 | NA |
| PRKACA | $w_{28}$ | NA | 0.082 | NA |
| PRKACB | $w_{29}$ | NA | -0.087 | NA |

(continued)

| Variable | Weights | | | |
|---|---|---|---|---|
| Model | | IDR_model | TCR_ SIGNALING_ model | PDE4D7_ CORR_model |
| PTPRC | $w_{30}$ | NA | 0.062 | NA |
| ZAP70 | $w_{31}$ | NA | 0.054 | NA |
| ABCC5 | $w_{32}$ | NA | NA | -0.27 |
| CUX2 | $w_{33}$ | NA | NA | -0.15 |
| KIAA1549 | $w_{34}$ | NA | NA | 0.014 |
| PDE4D | $w_{35}$ | NA | NA | -0.13 |
| RAP1GAP2 | $w_{36}$ | NA | NA | -0.053 |
| SLC39A11 | $w_{37}$ | NA | NA | 0.013 |
| TDRD1 | $w_{38}$ | NA | NA | 0.019 |
| VWA2 | $w_{39}$ | NA | NA | -0.042 |

Based on the three individual Cox regression models (IDR_model, TCR_SIGNALING_model, PDE4D7_CORR_model) we then again used Cox regression to model the combination thereof to overall survival with (GLCAI&Clinical_model) or without (GLCAI_model) the presence of the clinical variables (N stage attributes) in the respective cohorts of glioma patients. We tested the two models in Kaplan-Meier survival analysis.

The Cox regression functions were derived as follows:
GLCAI_model:

$$(w_{40} \cdot \text{IDR\_model}) + (w_{41} \cdot \text{TCR\_SIGNALING\_model}) + (w_{42} \cdot$$
$$\text{PDE4D7\_CORR\_model})$$

The details for the weights $w_{40}$ to $w_{44}$ are shown in the following TABLE 9.

TABLE 9: Variables and weights for two combination Cox regression models, i.e., glioma AI model (GLCAI_model) and the glioma & clinical model (GLCAI&Clinical_model); NA - not available.

| Variable | Weights | |
|---|---|---|
| Model | | GLCAI_model |
| IDR_model | $w_{40}$ | 0.59 |
| TCR_SIGNALING_model | $w_{41}$ | 0.81 |
| PDE47_CORR_model | $w_{42}$ | 0.19 |

## Example 2 - Kaplan-Meier Survival Analysis

For Kaplan-Meier survival curve analysis, the Cox functions of the risk models (IDR_model, TCR_SIGNALING_model, PDE4D7_CORR_model, and GLCAI_model) were categorized into two sub-cohorts based on a cut-off. The threshold for group separation into low and high risk was based on the risk to experience the clinical endpoint (outcome) as predicted by the respective Cox regression model

The Kaplan-Meier survival curve analysis as shown in Figs. 4 to 34 demonstrates the presence of different patient risk groups. The risk group of a patient is determined by the probability to suffer from the respective clinical endpoint (overall death) as calculated by the respective risk model as shown in the figures. Depending on the predicted risk of a patient (i.e., depending on in which risk group the patient may belong) to die from glioma different types of interventions might be indicated. In the low risk group (probability <0.5) standard of care (SOC) delivers acceptable long-term oncological control. This is definitely not the case for the patient group with a risk >0.5 to experience any of the relevant outcomes. In this patient group escalation of intervention or application of alternative treatment needs to happen. Alternative options for treatment escalation are adjuvant therapies with radiation or cytotoxic drugs or alternative therapies like immunotherapies (e.g., atezolizumab; pembrolizumab; nivolumab; avelumab; durvalumab) or other experimental therapies.

Example 3

Next it was hypothesized that a smaller subset of genes would still have predictive power. It is expected that a model of six genes selected from the Immune defense response signature, the T-cell signaling signature and the PDE4D7 correlated signature as described herein, suffices to make a prediction.

**Discussion**

The effectiveness of therapies for gliomas is limited, resulting in disease progression and ultimately death of patients, especially for those at high risk of recurrence of disease after primary intervention. The prediction of the therapy outcome is very challenging as many factors play a role in therapy effectiveness and disease recurrence. It is likely that important factors have not yet been identified, while the effect of others cannot be determined precisely. Multiple clinico-pathological measures are currently investigated and applied in a clinical setting to improve response prediction and therapy selection, providing some degree of improvement. Nevertheless, a strong need remains for better prediction of the treatment response in order to increase the success rate of glioma therapies.

We have identified molecules of which expression shows a significant relation to mortality after primery therapy of gliomas and therefore are expected to improve the prediction of the effectiveness of secondary treatments. This can be achieved by guiding patients with lower or high risk of progressive disease and subsequent death from cancer to the most appropriate, potentially more effective form of treatment as compared to currently applied standard of care. This would reduce suffering for those patients who would be spared ineffective therapy, improve chances for patients requiring more aggressive treatment, and would reduce cost spent on ineffective therapies.

Other variations to the disclosed realizations can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

One or more steps of the method illustrated in Fig. 1 may be implemented in a computer program product that may be executed on a computer. The computer program product may comprise a non-transitory computer-readable recording medium on which a control program is recorded (stored), such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other non-transitory medium from which a computer can read and use.

Alternatively, the one or more steps of the method may be implemented in transitory media, such as a transmittable carrier wave in which the control program is embodied as a data signal using transmission media, such as acoustic or light waves, such as those generated during radio wave and infrared data communications, and the like.

The exemplary method may be implemented on one or more general purpose computers, special purpose computer(s), a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLA, FPGA, Graphical card CPU (GPU), or PAL, or the like. In general, any device, capable of implementing a finite state machine that is in turn capable of implementing the flowchart shown in Fig. 1, can be used to implement one or more steps of the method of risk stratification for therapy selection in a patient with prostate cancer is illustrated. As will be appreciated, while the steps of the method may all be computer implemented, in some embodiments one or more of the steps may be at least partially performed manually.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Any reference signs in the claims should not be construed as limiting the scope.

The attached Sequence Listing, entitled 2023PF00423_SEQ_LST is incorporated herein by reference, in its entirety.**

**Claims**

**1.** A method of predicting an outcome of a subject having a glioma, the method comprising:

- determining or receiving the result of a determination of six or more gene expression levels selected from a first gene expression profile, a second gene expression profile and/or a third gene expression profile, wherein
- the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1;
- the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70;
- the third gene expression profile consists of the PDE4D7 correlated genes selected from the group consisting of: ABCC5, CUX2, KIAA1549, PDE4D, RAP1GAP2, SLC39A11, TDRD1, and VWA2;
said gene expression levels being determined in a biological sample obtained from the subject; and
- determining the prediction of the outcome based on six or more gene expression levels.

2. The method according to claim 1 further comprising the step of providing the prediction to a medical caregiver or the subject.

3. The method as according to claim 1 or 2, wherein the six or more gene expression levels comprise six or more gene expression levels selected from the first expression profile, six or more gene expression levels selected from the second expression profile, or six or more gene expression levels selected from the third expression profile.

4. The method according to any one of the preceding claims, wherein the determining of the prediction of the outcome further comprises combining the six or more gene expression levels with a regression function that had been derived from a population of subjects having a glioma.

5. The method according to any one of the preceding claims, wherein the determining of the outcome is further based on one or more clinical parameters obtained from the subject.

6. The method according to claim 5, wherein the clinical parameters comprise one or more of: (i) Karnofsky Performance Score; (ii) Tumor Grade; (iii) presence and amount of lesions.

7. The method according to any one of the preceding claims, wherein the biological sample(s) is/are obtained from the subject before the start of the therapy.

8. The method according to any one of the preceding claims, wherein the biological sample is a biopsy obtained from the subject, preferably a biopsy from the glioma or a metastasis.

9. The method according to any one of the preceding claims, wherein the prediction is mean survival time.

10. The method according to any one of the preceding claims, wherein the glioma is an astrocytoma, a pilocytic astrocytoma, a mixed oligoastrocytic tumor, glioblastoma (GBM), treated GBM, untreated (primary) GBM, oligoastrocytoma, oligodendroglioma, or an oligodendroglial tumor.

11. An apparatus for predicting an outcome of a subject having a glioma, comprising:

- an input adapted to receive data indicative of six or more gene expression levels selected from a first gene expression profile, a second gene expression profile and/or a third gene expression profile, wherein
- the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1;
- the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70;
- the third gene expression profile consists of the PDE4D7 correlated genes selected from the group consisting of: ABCC5, CUX2, KIAA1549, PDE4D, RAP1GAP2, SLC39A11, TDRD1, and VWA2;
said gene expression levels being determined in a biological sample obtained from the subject; and
- a processor adapted to determine the prediction of outcome based on the six or more gene expression levels, and
- optionally, a providing unit adapted to provide the prediction to a medical caregiver or the subject.

12. A computer program product comprising instructions which, when the program is executed by a computer, cause the computer to carry out a method for predicting an outcome of a subject having a glioma comprising:

- receiving data indicative of six or more gene expression levels selected from a first gene expression profile, a second gene expression profile and/or a third gene expression profile, wherein
- the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1;
- the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70;
- the third gene expression profile consists of the PDE4D7 correlated genes selected from the group consisting of: ABCC5, CUX2, KIAA1549, PDE4D, RAP1GAP2, SLC39A11, TDRD1, and VWA2;
said gene expression levels being determined in a biological sample obtained from the subject; and
- determining a prediction of an outcome of the subject based on the six or more gene expression levels, and
- optionally, providing the prediction to a medical caregiver or the subject.

13. Use of a kit, the use comprising:

- determining six or more gene expression levels in a sample obtained from a subject having a glioma; and
- providing an outcome for the subject based on the six or more gene expression levels;
wherein the kit comprises means for determining six or more gene expression levels selected from a first gene expression profile, a second gene expression profile and/or a third gene expression profile, wherein
- the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1;
- the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70;
- the third gene expression profile consists of the PDE4D7 correlated genes selected from the group consisting of: ABCC5, CUX2, KIAA1549, PDE4D, RAP1GAP2, SLC39A11, TDRD1, and VWA2.

14. Use according to claim 13, wherein the use comprises performing the method according to any one of claims 1 to 10.

15. Product for use in the treatment or amelioration of a subject having a glioma, the product comprising a chemotherapeutic compound, a targeted cancer therapeutic compound or an immunotherapeutic compound;
wherein the use comprises determining or receiving the result of a determination of six or more gene expression levels selected from a first gene expression profile, a second gene expression profile and/or a third gene expression profile, wherein

- the first gene expression profile consist of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1;
- the second gene expression profile consist of the T-Cell receptor signaling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70;
- the third gene expression profile consists of the PDE4D7 correlated genes selected from the group consisting of: ABCC5, CUX2, KIAA1549, PDE4D, RAP1GAP2, SLC39A11, TDRD1, and VWA2;
said gene expression levels being determined in a biological sample obtained from the subject; and
- determining the prediction of the outcome based on six or more gene expression levels;
and administering to the subject the product.

Fig. 1

Fig. 2

Fig. 3

Fig. 4

Fig. 5

Fig. 6

survival_status

Fig. 7

survival_status

Fig. 8

Fig. 9

Fig. 10

survival_status

Fig. 11

survival_status

Fig. 12

Fig. 13

Fig. 14

survival_status

p<0,0001
HR=2,5 [95%CI 1,8 to 3,5]

TCR_17
—— <=threshold (0)
—— >threshold (0)

Survival Time [months]
training set (#180 patients)

Number at risk
Group: <=threshold (0)
| 93 | 54 | 35 | 28 | 21 | 13 | 10 | 4 | 4 | 4 | 3 | 2 |
Group: >threshold (0)
| 87 | 19 | 10 | 9 | 5 | 3 | 2 | 2 | 1 | 1 | 0 | 0 |

Fig. 15

survival_status

p=0,3
HR=1,3 [95%CI 0,8 to 2,0]

TCR_17
—— <=threshold (0)
—— >threshold (0)

Survival Time [months]
testing set (#92 patients)

Number at risk
Group: <=threshold (0)
| 45 | 22 | 14 | 8 | 3 | 2 | 0 | 0 | 0 |
Group: >threshold (0)
| 47 | 18 | 7 | 5 | 4 | 3 | 3 | 2 | 0 |

Fig. 16

survival_status

p<0,0001
HR=3,1 [95%CI 2,2 to 4,5]

PDE4D7_R2
— <=threshold (0)
— >threshold (0)

Survival Time [months]
training set (#180 patients)

Number at risk
Group: <=threshold (0)

| 87 | 57 | 38 | 30 | 23 | 13 | 10 | 6 | 5 | 5 | 3 | 2 |

Group: >threshold (0)

| 93 | 16 | 7 | 7 | 3 | 3 | 2 | 0 | 0 | 0 | 0 | 0 |

**Fig. 17**

survival_status

p<0,0001
HR=2,8 [95%CI 1,7 to 4,4]

PDE4D7_R2
— <=threshold (0)
— >threshold (0)

Survival Time [months]
testing set (#92 patients)

Number at risk
Group: <=threshold (0)

| 41 | 29 | 18 | 10 | 5 | 4 | 2 | 1 | 0 |

Group: >threshold (0)

| 51 | 11 | 3 | 3 | 2 | 1 | 1 | 1 | 0 |

**Fig. 18**

Fig. 19

Fig. 20

Fig. 21

Fig. 22

Fig. 23

Fig. 24

survival_status

logrank p<0,0001
HR=2,5 [95% CI 1,9-3,3]

TCR_17
<=thresold (0)
>thresold (0)

Survival Time [months]
training set (#281 patients)

Number at risk
Group: <=thresold (0)
137  95  49  33  20  13  10  4  2  2  2  1  0  0
Group: >thresold (0)
144  46  20  9  5  1  1  1  1  1  1  1  1  0

Fig. 25

survival_status

logrank p<0,0001
HR=2,4 [95% CI 1,6-3,8]

TCR_17
<=thresold (0)
>thresold (0)

Survival Time [months]
testing set (#147 patients)

Number at risk
Group: <=thresold (0)
76  49  33  21  13  10  6  2  2  2  0
Group: >thresold (0)
71  31  10  3  0  0  0  0  0  0  0

Fig. 26

Fig. 27

Fig. 28

Fig. 29

Fig. 30

Fig. 31

Fig. 32

survival_status

logrank p=0.03
KS 0-20%; median survival 15,3 months
KS 20-40%; median survival 21,0 months
KS 40-60%; median survival 37,5 months
KS 60-80%; median survival 34,2 months
KS 80-100%; median survival 45,3 months

Karnofsky Performance Score
0-20%
20-40%
40-60%
60-80%
80-100%

Survival time [months]
GLCAI<0;all disease types

Number at risk
Group: 0-20%
2 1 0 0 0 0 0 0 0 0 0 0 0 0
Group: 20-40%
2 2 1 1 1 1 1 1 1 1 1 1 0 0
Group: 40-60%
13 9 5 4 2 1 0 0 0 0 0 0 0 0
Group: 60-80%
27 17 5 4 3 3 3 3 2 2 0 0 0 0
Group: 80-100%
84 67 33 20 11 8 4 2 2 2 2 1 1 0

Fig. 33

survival_status

logrank p=0.1
KS 0-20%; median survival 13,3 months
KS 20-40%; median survival 7,9 months
KS 40-60%; median survival 20, months
KS 60-80%; median survival 18,3 months
KS 80-100%; median survival 27,4 months

Karnofsky Performance Score
0-20%
20-40%
40-60%
60-80%
80-100%

Survival time [months]
GLCAI>=0;all disease types

Number at risk
Group: 0-20%
7 2 2 0 0 0
Group: 20-40%
6 1 1 1 0 0
Group: 40-60%
34 10 6 1 0 0
Group: 60-80%
43 14 1 0 0 0
Group: 80-100%
50 24 11 4 1 0

Fig. 34

**Europäisches Patentamt**
**European Patent Office**
**Office européen des brevets**

## PARTIAL EUROPEAN SEARCH REPORT

under Rule 62a and/or 63 of the European Patent Convention.
This report shall be considered, for the purposes of
subsequent proceedings, as the European search report

**Application Number**

EP 23 18 6098

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2015/038357 A1 (JOUBERT DOMINIQUE [FR] ET AL) 5 February 2015 (2015-02-05) * abstract; claim 16 * | 1-15 | INV. C12Q1/6886 |
| Y | FREIJE W A ET AL: "Gene expression profiling of gliomas strongly predicts survival", CANCER RESEARCH, AMERICAN ASSOCIATION FOR CANCER RESEARCH, US, vol. 64, no. 18, 15 September 2004 (2004-09-15), pages 6503-6510, XP003010811, ISSN: 0008-5472, DOI: 10.1158/0008-5472.CAN-04-0452 * abstract; table 2 * | 1-15 | |
| Y | WO 2023/083581 A1 (KONINKLIJKE PHILIPS NV [NL]) 19 May 2023 (2023-05-19) * abstract * | 1-15 | |
| Y | WO 2022/152597 A1 (KONINKLIJKE PHILIPS NV [NL]) 21 July 2022 (2022-07-21) * abstract; claim 1; table 1 * | 1-15 | **TECHNICAL FIELDS SEARCHED (IPC)** C12Q |

-/--

## INCOMPLETE SEARCH

The Search Division considers that the present application, or one or more of its claims, does/do
not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| The Hague | 30 January 2024 | Aguilera, Miguel |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
................................................................
& : member of the same patent family, corresponding
    document

EPO FORM 1503 03.82 (P04E07)

Europäisches
Patentamt

European
Patent Office

Office européen
des brevets

# PARTIAL EUROPEAN SEARCH REPORT

Application Number

EP 23 18 6098

## DOCUMENTS CONSIDERED TO BE RELEVANT

| Category | Citation of document with indication, where appropriate, of relevant passages | Relevant to claim |
|---|---|---|
| Y | WO 2022/069201 A1 (KONINKLIJKE PHILIPS NV [NL]) 7 April 2022 (2022-04-07) * abstract; claim 1; table 1 * ----- | 1-15 |
| Y | WO 2021/175746 A1 (KONINKLIJKE PHILIPS NV [NL]) 10 September 2021 (2021-09-10) * abstract * ----- | 1-15 |

**CLASSIFICATION OF THE APPLICATION (IPC)**

**TECHNICAL FIELDS SEARCHED (IPC)**

EPO FORM 1503 03.82 (P04C10)

Europäisches
Patentamt

European
Patent Office

Office européen
des brevets

**INCOMPLETE SEARCH
SHEET C**

```
Claim(s) searched incompletely:
      1-15

Reason for the limitation of the search:

The reasons for which a complete search is not possible are specified in
the annexed provisional opinion accompanying the partial search results
(EPO Form 1707).
```

Europäisches
Patentamt

European
Patent Office

Office européen
des brevets

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**1-15(partially)**

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

**EP 23 18 6098**

The Search Division considers that the present European patent application does not comply with the requirements of unity of invention and relates to several inventions or groups of inventions, namely:

1. claims: 1-15(partially)

    A method of predicting an outcome of a subject having a glioma, the method comprising determining or receiving the result of a determination of gene expression levels from a gene expression profile consisting of the immune defense response genes: AIM2, APOBEC3A, CIAO 1, DDX58, DHX9, IFI16, IFIH1, IFIT1, IFIT3, LRRFIP1, MYD88, OAS1, TLR8 and ZBP1. An apparatus for predicting an outcome of a subject having a glioma, comprising an input adapted to receive data indicative of said expression profile and a processor adapted to determine the prediction of outcome. A computer program product comprising instructions to carry out said method. Use of a kit for carrying out said method, wherein the kit comprises means for determining the gene expression levels of said genes. Product for use in the treatment or amelioration of a subject having a glioma wherein the use comprises determining or receiving the result of a determination of said expression profile in a sample from the subject and determining the prediction of outcome.
    ---

2. claims: 1-15(partially)

    A method of predicting an outcome of a subject having a glioma, the method comprising determining or receiving the result of a determination of gene expression levels from a gene expression profile consisting of the T-Cell receptor signaling genes: CD2, CD247, CD28, CD3E, CD3G, CD4, CSK, EZR, FYN, LAT, LCK, PAG1, PDE4D, PRKACA, PRKACB, PTPRC and ZAP70. An apparatus for predicting an outcome of a subject having a glioma, comprising an input adapted to receive data indicative of said expression profile and a processor adapted to determine the prediction of outcome. A computer program product comprising instructions to carry out said method. Use of a kit for carrying out said method, wherein the kit comprises means for determining the gene expression levels of said genes. Product for use in the treatment or amelioration of a subject having a glioma wherein the use comprises determining or receiving the result of a determination of said expression profile in a sample from the subject and determining the prediction of outcome.
    ---

3. claims: 1-15(partially)

    A method of predicting an outcome of a subject having a glioma, the method comprising determining or receiving the result of a determination of gene expression levels from a gene expression profile consisting of the PDE4D7 correlated genes: ABCC5, CUX2, KIAA1549, PDE4D, RAP1GAP2, SLC39A11,

page 1 of 2

Europäisches
Patentamt

European
Patent Office

Office européen
des brevets

**LACK OF UNITY OF INVENTION**
**SHEET B**

The Search Division considers that the present European patent application does not comply with the requirements of unity of invention and relates to several inventions or groups of inventions, namely:

> **TDRD1 and VWA2. An apparatus for predicting an outcome of a subject having a glioma, comprising an input adapted to receive data indicative of said expression profile and a processor adapted to determine the prediction of outcome. A computer program product comprising instructions to carry out said method. Use of a kit for carrying out said method, wherein the kit comprises means for determining the gene expression levels of said genes. Product for use in the treatment or amelioration of a subject having a glioma wherein the use comprises determining or receiving the result of a determination of said expression profile in a sample from the subject and determining the prediction of outcome.**
> ---

page 2 of 2

## EP 4 495 265 A1

## ANNEX TO THE EUROPEAN SEARCH REPORT
## ON EUROPEAN PATENT APPLICATION NO.

EP 23 18 6098

This annex lists the patent family members relating to the patent documents cited in the above-mentioned European search report.
The members are as contained in the European Patent Office EDP file on
The European Patent Office is in no way liable for these particulars which are merely given for the purpose of information.

30-01-2024

| Patent document cited in search report | | Publication date | Patent family member(s) | | Publication date |
|---|---|---|---|---|---|
| US 2015038357 A1 | | 05-02-2015 | CA | 2850646 A1 | 11-04-2013 |
| | | | EP | 2751287 A1 | 09-07-2014 |
| | | | JP | 2015501138 A | 15-01-2015 |
| | | | US | 2015038357 A1 | 05-02-2015 |
| | | | WO | 2013050331 A1 | 11-04-2013 |
| WO 2023083581 A1 | | 19-05-2023 | EP | 4177357 A1 | 10-05-2023 |
| | | | WO | 2023083581 A1 | 19-05-2023 |
| WO 2022152597 A1 | | 21-07-2022 | CN | 117203352 A | 08-12-2023 |
| | | | EP | 4026918 A1 | 13-07-2022 |
| | | | EP | 4278013 A1 | 22-11-2023 |
| | | | JP | 2024502489 A | 19-01-2024 |
| | | | US | 2024076746 A1 | 07-03-2024 |
| | | | WO | 2022152597 A1 | 21-07-2022 |
| WO 2022069201 A1 | | 07-04-2022 | CN | 116249788 A | 09-06-2023 |
| | | | EP | 3978629 A1 | 06-04-2022 |
| | | | EP | 4222286 A1 | 09-08-2023 |
| | | | JP | 2023543838 A | 18-10-2023 |
| | | | US | 2023357858 A1 | 09-11-2023 |
| | | | WO | 2022069201 A1 | 07-04-2022 |
| WO 2021175746 A1 | | 10-09-2021 | CN | 115398009 A | 25-11-2022 |
| | | | EP | 3875608 A1 | 08-09-2021 |
| | | | EP | 4114980 A1 | 11-01-2023 |
| | | | JP | 2023521280 A | 24-05-2023 |
| | | | US | 2023160017 A1 | 25-05-2023 |
| | | | WO | 2021175746 A1 | 10-09-2021 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **THORSEN et al.** *NMR Biomed.*, October 2008, vol. 21 (8), 830-8
- **KAN et al.** *BMJ Neurol Open.*, 24 August 2020, vol. 2 (2), e000069
- **GRAVENDEEL et al.** *Cancer Res.*, 01 December 2009, vol. 69 (23), 9065-72
- **SAMBROOK et al.** Molecular Cloning. A Laboratory Manual. Cold Spring Harbor Laboratory Press, 1989
- **AUSUBEL et al.** Current Protocols in Molecular Biology. John Wiley & Sons, 1987
- Methods in Enzymology. Academic Press
- Computational Molecular Biology. Oxford University Press, 1988
- Biocomputing: Informatics And Genome Projects. Academic Press, 1993
- Computer Analysis Of Sequence Data, Part I. Humana Press, 1994
- **VON HEINJE, G.** Sequence Analysis In Molecular Biology. Academic Press, 1987
- Sequence Analysis Primer. M Stockton Press, 1991
- **CARILLO, H.** ; **LIPTON, D.** *SIAM J. Applied Math*, 1988, vol. 48, 1073
- Guide To Huge Computers. Academic Press, 1994
- **CARILLO, H.** ; **LIPTON, D.** *Siam J. Applied Math*, 1988, vol. 48, 1073
- **DEVEREUX, J. et al.** *Nucleic Acids Research*, 1984, vol. 12 (1), 387
- **ATSCHUL, S. F. et al.** *J. Molec. Biol.*, 1990, vol. 215, 403
- **GASSER S. et al.** Sensing of dangerous DNA. *Mechanisms of Aging and Development*, 2017, vol. 165, 33-46
- **MOSENDEN R.** ; **TASKEN K.** Cyclic AMP-mediated immune regulation - Overview of mechanisms of action in T-cells. *Cell Signal*, 2011, vol. 23 (6), 1009-1016
- **TASKEN K.** ; **RUPPELT A.** Negative regulation of T-cell receptor activation by the cAMP-PKA-Csk signaling pathway in T-cell lipid rafts. *Front Biosci*, 2006, vol. 11, 2929-2939
- **ABRAHAMSEN H. et al.** TCR- and CD28-mediated recruitment of phosphodiesterase 4 to lipid rafts potentiates TCR signaling. *J Immunol*, 2004, vol. 173, 4847-4848
- Immunity Leashed - Mechanisms of Regulation in the Human Immune System. **TORHEIM E.A.** Thesis for the degree of Philosophiae Doctor (PhD). The Biotechnology Centre of Ola, University of Oslo, 2009
- **ALVES DE INDA M. et al.** Validation of Cyclic Adenosine Monophosphate Phosphodiesterase-4D7 for its Independent Contribution to Risk Stratification in a Prostate Cancer Patient Cohort with Longitudinal Biological Outcomes. *Eur Urol Focus*, 2018, vol. 4 (3), 376-384